# EUROPEAN PATENT APPLICATION

(11) **EP 2 216 356 A1**
(43) Date of publication of application: **11.08.2010**
(21) Application number: 08853335.1
(22) Date of filing: 20.11.2008
(51) Int. Cl.: C08G 61/10, C07C 205/12, C07C 209/10, C07C 209/18, C07C 211/52, C07C 211/56, C09K 11/06, H01L 51/50

(54) **AMINE-TYPE POLYMERIC COMPOUND, AND LIGHT-EMITTING ELEMENT COMPRISING THE SAME**

(30) Priority: 30.11.2007 JP 2007310073
(71) Applicant: Sumitomo Chemical Company, Limited, Tokyo 104-8260 (JP); Sumation Co., Ltd., Chuo-ku Tokyo 104-8260 (JP)
(72) Inventor: OHUCHI, Kazuei, Tsukuba-shi Ibaraki 305-0005 (JP); ANRYU, Makoto, Tsukuba-shi Ibaraki 305-0821 (JP)
(74) Representative: Duckworth, Timothy John
(86) International application number: PCT/JP2008/071068
(87) International publication number: WO 2009/069523

(57) **Abstract**

Disclased is a polymeric compound having a constitutional unit represented by formula (1a), wherein ring A and ring B independently represent an aromatic hydrocarbon ring which may have a substituent; R1 represents a group represented by formula (2); and R2 represents an aryl group or a monovalent aromatic heterocyclic group which may be substituted by an alkyl group or the like. In formula (2), Ar1 to Ar3 independently represent an arylene group or a divalent group to which two or more arylene groups are attached via a single bond; Ar4 to Ar7 independently represent an aryl group or a monovalent aromatic heterocyclic group; R6 represents a hydrogen atom, an alkyl group, an aryl group or a monovalent aromatic heterocyclic group; Ar1 to Ar7 and R6 may be substituted by an alkyl group or the like; and k and kk independently represent an integer of 0 to 3, provided that at least one of k and kk represents an integer of 1 to 3.

## Description

### TECHNICAL FIELD

The present invention relates to an amine polymer compound and a light-emitting device comprising the same.

### BACKGROUND ART

A polymer compound having a fluorene structure such as 9,9-dialkylfluorene, in which two alkyl groups are introduced into the position 9 of the fluorene structure, has been known to be useful for light-emitting devices (polymer LED, etc.) and the like (Non-Patent Document 1). In addition, as a polymer compound having excellent luminous efficiency, there has been proposed a polymer compound in which two amine skeletons such as triphenylamine are introduced into the position 9 of the above-mentioned fluorene structure (Patent Documents 1 and 2).

Patent Document 1: JP 2004-500463 A
Patent Document 2: JP 2007-031704 A
Non-Patent Document 1: Advanced Materials 2000, 12(23), 1737-1750

### DISCLOSURE OF THE INVENTION

### PROBLEMS TO BE SOLVED BY THE INVENTION

However, when the aforementioned polymer compound has been used in the production of a light-emitting device, it has not had a sufficient external quantum yield used as an indicator of luminous efficiency when the chromaticity of the light-emitting device is considered.

Thus, it is an object of the present invention to provide a polymer compound, which brings on an excellent external quantum yield of a light-emitting device, when it is used to produce a light-emitting device or the like.

### MEANS FOR SOLVING THE PROBLEMS

A first aspect of the present invention provides a polymer compound comprising a constitutional unit represented by the following formula (1a): 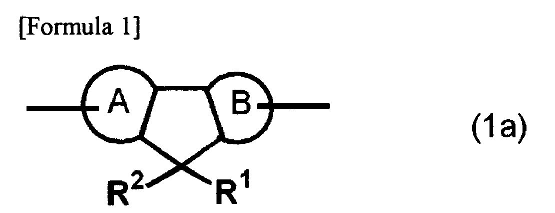 wherein each of ring A and ring B independently represents an aromatic hydrocarbon ring that may have a substituent; R¹ represents a group represented by formula (2) provided below; and R² represents an aryl group or a monovalent aromatic heterocyclic group, and these groups may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group; 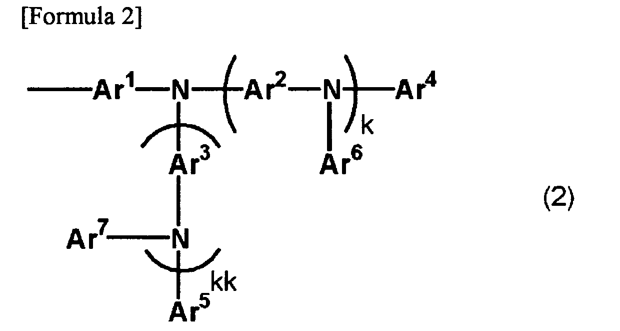 wherein each of Ar¹, Ar² and Ar³ independently represents an arylene group or a divalent group in which two or more identical or different arylene groups are bound to one another by a single bond; each of Ar⁴, Ar⁵, Ar⁶ and Ar⁷ independently represents an aryl group or a monovalent aromatic heterocyclic group; a group selected from among the groups represented by Ar³, Ar⁶ and Ar⁷ may form a 5- to 7-membered ring by being bound by a single bond or by -O-, -S-, - C(=O)-, -C(=O)-O-, -N(R⁶)-, -C(=O)-N(R⁶)- or -C(R⁶)(R⁶)- to a group that is selected from among the groups represented by Ar¹, Ar², Ar³, Ar⁴, Ar⁵, Ar⁶ and Ar⁷ and that is attached to a nitrogen atom which is the same as that to which the group selected from the groups represented by Ar³, Ar⁶ and Ar⁷ is attached. R⁶ represents a hydrogen atom, an alkyl group, an aryl group or a monovalent aromatic heterocyclic group; each of the groups represented by Ar¹, Ar², Ar³, Ar⁴, Ar⁵, Ar⁶, Ar⁷ and R⁶ may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group; each of k and kk independently represents an integer of 0 to 3, but at least one of k and kk is an integer of 1 to 3; and if a plurality of Ar², Ar³, Ar⁶, Ar⁷ or R⁶ are present, they may be identical to or different from one another.

A second aspect of the present invention provides a compound represented by the following formula (A): 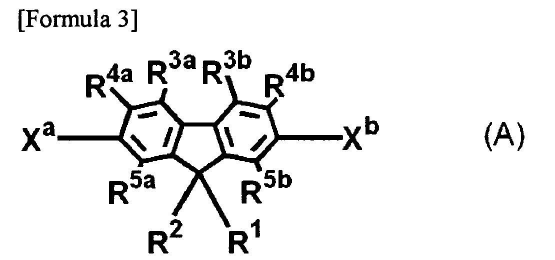 wherein R¹, R², R^{3a}, R^{4a}, R^{5a}, R^{3b}, R^{4b} and R^{5b} are the same as above in meaning; each of X^{a} and X^{b} independently represents a bromine atom; an iodine atom, a chlorine atom, -O-SO₂R²⁰, - B(OR²¹)₂, -BF₄Q¹, -Sn(R²²)₃, -MgY¹ or -ZnY¹; R²⁰ represents an alkyl group, or an aryl group; the aryl group represented by R²⁰ may be substituted with an alkyl group, an alkoxy group, a nitro group, a fluorine atom or a cyano group; each of R²¹ and R²² independently represents a hydrogen atom or an alkyl group; Q¹ represents a monovalent cation of lithium, sodium, potassium, rubidium or cesium; Y¹ represents a bromine atom, an iodine atom or a chlorine atom; two R²¹s may be identical to or different from each other or may together form a ring; and three R²²s may be identical to or different from one another or may together form a ring.

A third aspect of the present invention provides a compound represented by the following formula (B): 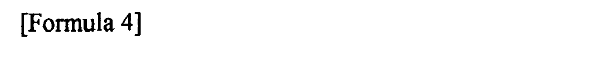 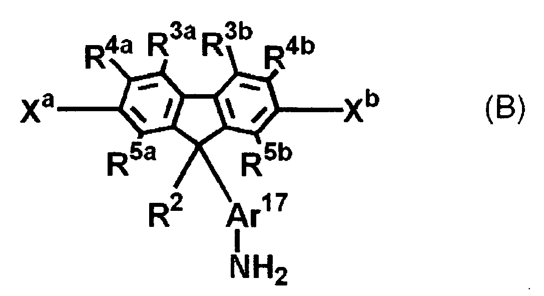 wherein R², R^{3a}, R^{4a}, R^{5a}, R^{3b}, R^{4b}, R^{5b}, X^{a} and X^{b} are the same as above in meaning, and Ar¹⁷ represents an arylene group, wherein the arylene group represented by Ar¹⁷ may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group.

A fourth aspect of the present invention provides a compound represented by the following formula (C): 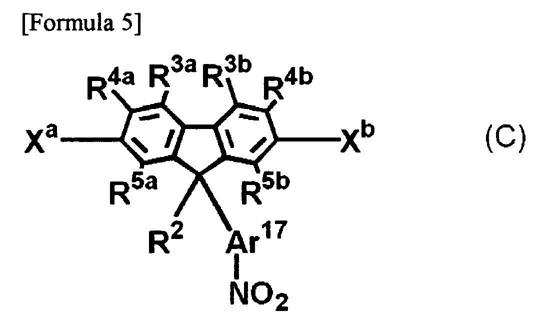 wherein R², R^{3a}, R^{4a}, R^{5a}, R^{3b}, R^{4b}, R^{5b}, X^{a}, X^{b} and Ar¹⁷ are the same as above in meaning.

A fifth aspect of the present invention provides a method for producing the compound represented by formula (A), which comprises making the compound represented by formula (B) react with a compound represented by the following formula (E): 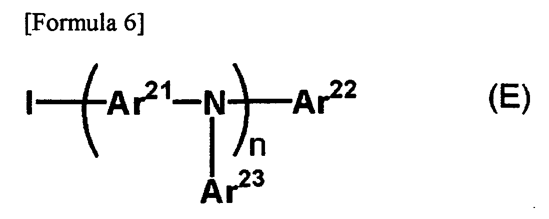 wherein Ar²¹ represents an arylene group, or a divalent group in which two or more identical or different arylene groups are bound to one another by a single bond; the arylene group and the divalent group represented by Ar²¹ may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group; each of Ar²² and Ar²³ independently represents an aryl group or a monovalent aromatic heterocyclic group; the aryl group and the monovalent aromatic heterocyclic group represented by Ar²² and Ar²³ may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group; two groups which are selected from among the groups represented by Ar²¹, Ar²² and Ar²³ and which are attached to the same nitrogen atom may be bound to each other to form a 5- to 7-membered ring by a single bond or by -O-, -S-, - C(=O)-, -C(=O)-O-, -N(R⁶)-, -C(=O)-N(R⁶)- or -C(R⁶)(R⁶)-; n represents an integer of 0 to 2; if a plurality of Ar²¹ or Ar²³ are present, they may be identical to or different from one another; and R⁶ is the same as above in meaning.

A sixth aspect of the present invention provides a method for producing the compound represented by formula (A), which comprises making a compound represented by the following formula (D) react with a compound represented by the following formula (F): 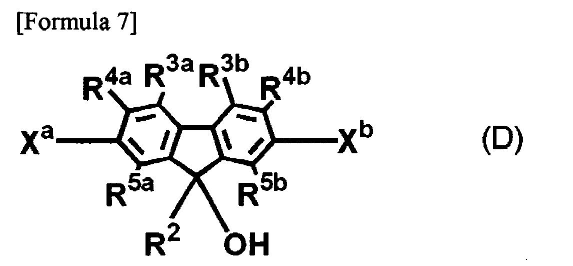 wherein R², R^{3a}, R^{4a}, R^{5a}, R^{3b}, R^{4b}, R^{5b}, X^{a} and X^{b} are the same as above in meaning; 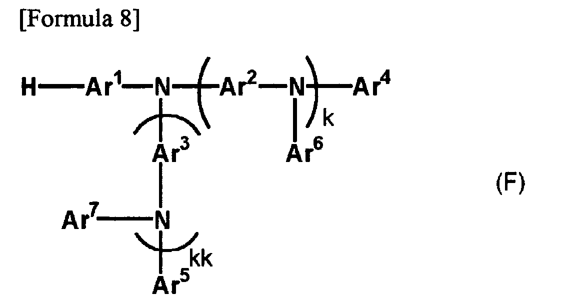 wherein Ar¹, Ar², Ar³, Ar⁴, Ar⁵, Ar⁶, Ar⁷, k and kk are the same as above in meaning.

A seventh aspect of the present invention provides a composition comprising the polymer compound; a solution comprising the polymer compound and a solvent; and a thin film comprising the polymer compound.

An eighth aspect of the present invention provides a light-emitting device which comprises electrodes consisting of an anode and a cathode, and an organic layer containing the polymer compound provided between the electrodes; and a planar light source and a display device which comprise the light-emitting device.

### EFFECT OF THE INVENTION

When the polymer compound of the present invention is used to produce light-emitting devices such as polymer LED, it brings on an excellent external quantum yield of the obtained light-emitting device. Moreover, in general, the polymer compound of the present invention is excellent in terms of heat resistance, and it is useful as a material for light-emitting devices. Furthermore, in a preferred embodiment of the present invention, since the polymer compound of the present invention emits pure blue light, it is useful as a material for the light-emitting layer of a light-emitting device.
A light-emitting device, in which the polymer compound of the present invention is used, is useful as a backlight for liquid crystal display, a curved or planar light source for lighting, a segment-type display device, and a display device such as a flat panel display of dot matrix.

### BEST MODE FOR CARRYING OUT THE INVENTION

The present invention will be described in detail below.
The term "constitutional unit" is used in the present specification to mean one or more units existing in a polymer compound. The "constitutional unit" in the present specification is preferably comprised in a polymer compound as a "repeating unit" (that is, two or more units existing in a polymer compound). The term "n-valent aromatic heterocyclic group" (wherein n is 1 or 2) means an atomic group formed by removing an n number of hydrogen atoms from a heterocyclic compound having aromaticity, and this group also includes those having a condensed ring. The term "heterocyclic compound" means an organic compound having a cyclic structure, in which atoms constituting the ring include not only a carbon atom, but also heteroatoms such as an oxygen atom, a sulfur atom, a nitrogen atom, a phosphorus atom, a boron atom or a silicon atom. The term "aromatic heterocyclic compound" means: heterocyclic compounds containing the above-mentioned heteroatoms in which the heterocyclic ring itself exhibits aromaticity, such as oxadiazole, thiadiazole, thiazole, oxazole, thiophene, pyrrole, phosphole, furan, pyridine, pyrazine, pyrimidine, triazine, pyridazine, quinoline, isoquinoline, carbazole, dibenzosilole or dibenzophosphole; and heterocyclic compounds in which the heterocyclic ring itself does not exhibit aromaticity but an aromatic ring is annelated on the heterocyclic ring, such as phenoxazine, phenothiazine, dibenzoborole, dibenzosilole or benzopyran.

### <Polymer compound>

### - Constitutional unit represented by formula (1a) -

The polymer compound of the present invention comprises the constitutional unit represented by the above-described formula (1a). It is sufficient that the constitutional unit represented by formula (1a) comprised in the polymer compound of the present invention be of one or more types.

In the above-described formula (1a), an aromatic hydrocarbon ring represented by ring A or ring B generally contains 6 to 14 carbon atoms that constitute the aromatic ring. The aromatic hydrocarbon ring includes a benzene ring, a naphthalene ring, a fluorene ring, an anthracene ring, and a phenanthrene ring. The aromatic hydrocarbon ring may have a substituent. It is to be noted that a bond exists on each of the above-described ring A and ring B.

In the above-described formula (1a), an aryl group represented by R² is an atomic group formed by removing one hydrogen atom from an aromatic hydrocarbon, and it includes those having a condensed ring. The aryl group generally contains approximately 6 to 60, preferably 6 to 48, more preferably 6 to 20, and further preferably 6 to 14 carbon atoms. The above-described number of carbon atoms does not include the number of carbon atoms of substituents. The aryl group includes phenyl group, 1-naphthyl group, 2-naphthyl group, 1-anthracenyl group, 2-anthracenyl group, 9-anthracenyl group, 1-tetracenyl group, 2-tetracenyl group, 5-tetracenyl group, 1-pyrenyl group, 2-pyrenyl group, 4-pyrenyl group, 2-perylenyl group, 3-perylenyl group, 2-fluorenyl group, 3-fluorenyl group, 4-fluorenyl group, 1-biphenylenyl group, 2-biphenylenyl group, 2-phenanthrenyl group, 9-phenanthrenyl group, 6-chrysenyl group, and 1-coronenyl group. The aryl group may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group.

In the above-described formula (1a), a monovalent aromatic heterocyclic group represented by R² generally contains approximately 3 to 60, and preferably 3 to 20 carbon atoms. The above-described number of carbon atoms does not include the number of carbon atoms of substituents. The monovalent aromatic heterocyclic group includes a 2-oxadiazole group, a 2-thiadiazole group, a 2-thiazole group, a 2-oxazole group, a 2-thienyl group, a 2-pyrrolyl group, a 2-furyl group, a 2-pyridyl group, a 3-pyridyl group, a 4-pyridyl group, a 2-pyrazyl group, a 2-pyrimidyl group, a 2-triazyl group, a 3-pyridazyl group, a quinolyl group, an isoquinolyl group, a 2-carbazolyl group, a 3-carbazolyl group, a 2-phenoxadinyl group, a 3-phenoxadinyl group, a 2-phenothiazinyl group, and a 3-phenothiazinyl group. The monovalent aromatic heterocyclic group may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group.
It is to be noted that the monovalent aromatic heterocyclic group is different from the group represented by the above-described formula (2).

### • Explanation of substituents

The above-described alkyl group may be any one of linear, branched and cyclic groups, and it generally contains approximately 1 to 20 carbon atoms. The alkyl group includes a methyl group, an ethyl group, a propyl group, an isopropyl group, a butyl group, an isobutyl group, a t-butyl group, a pentyl group, an isoamyl group, a hexyl group, a cyclohexyl group, a heptyl group, an octyl group, a 2-ethylhexyl group, a nonyl group, a decyl group, a 3,7-dimethyloctyl group, a dodecyl group, a trifluoromethyl group, a pentafluoroethyl group, a perfluorobutyl group, a perfluorohexyl group, and a perfluorooctyl group.

The above-described alkoxy group may be any one of linear, branched and cyclic groups, and it generally contains approximately 1 to 20 carbon atoms. The alkoxy group includes a methoxy group, an ethoxy group, a propyloxy group, an isopropyloxy group, a butoxy group, an isobutoxy group, a t-butoxy group, a pentyloxy group, a hexyloxy group, a cyclohexyloxy group, a heptyloxy group, an octyloxy group, a 2-ethylhexyloxy group, a nonyloxy group, a decyloxy group, a 3,7-dimethyloctyloxy group, a dodecyloxy group, a trifluoromethoxy group, a pentafluoroethoxy group, a perfluorobutoxy group, a perfluorohexyl group, a perfluorooctyl group, a methoxymethyloxy group, a 2-methoxyethyloxy group, and a 2-ethoxyethyloxy group.

The above-described alkylthio group may be any one of linear, branched and cyclic groups, and it generally contains approximately 1 to 20 carbon atoms. The alkylthio group includes a butylthio group, a hexylthio group, an octylthio group, a 2-ethylhexylthio group, a 3,7-dimethyloctylthio group, and a dodecylthio group.

The above-described substituted carbonyl group generally contains approximately 2 to 60 carbon atoms. The substituted carbonyl group includes a carbonyl group substituted with an alkyl group, an aryl group, an aralkyl group or a monovalent aromatic heterocyclic group. An acetyl group, a butyryl group, a benzoyl group and the like are preferable.

The above-described substituted carboxyl group generally contains approximately 2 to 60 carbon atoms. The substituted carboxyl group includes a carboxyl group substituted with an alkyl group, an aryl group, an aralkyl group or a monovalent aromatic heterocyclic group. A methoxycarbonyl group, an ethoxycarbonyl group, a butoxycarbonyl group, a phenoxycarbonyl group, a benzyloxycarbonyl group and the like are preferable.

The above-described aryl group is the same as that described and exemplified in the section of the aryl group represented by R².

The above-described aryloxy group generally contains approximately 6 to 60 carbon atoms. The aryloxy group includes a phenoxy group, a C₁-C₁₂ alkoxyphenoxy group (the term "C₁-C₁₂ alkoxy" means that the number of carbon atoms in the alkoxy portion is 1 to 12; the same applies below), a C₁-C₁₂ alkylphenoxy group (the term "C₁-C₁₂ alkyl" means the number of carbon atoms in the alkyl portion is 1 to 12; the same applies below), a 1-naphthyloxy group, a 2-naphthyloxy group, and a pentafluorophenyloxy group.

The above-described arylthio group generally contains approximately 6 to 60 carbon atoms. The arylthio group includes a phenylthio group, a C₁-C₁₂ alkoxyphenylthio group, a C₁-C₁₂ alkylphenylthio group, a 1-naphthylthio group, a 2-naphthylthio group, and a pentafluorophenylthio group.

The above-described aralkyl group generally contains approximately 7 to 60 carbon atoms. The aralkyl group includes a phenyl-C₁-C₁₂ alkyl group, a C₁-C₁₂ alkoxyphenyl-C₁-C₁₂ alkyl group, a C₁-C₁₂ alkylphenyl-C₁-C₁₂ alkyl group, a 1-naphthyl-C₁-C₁₂ alkyl group, and a 2-naphthyl-C₁-C₁₂ alkyl group.

In formula (1a), as a group represented by R², the above-described aryl group is preferable. From the viewpoint of the balance between the solubility of the polymer compound of the present invention in an organic solvent and heat resistance, and the like, an aryl group substituted with an alkyl group, an alkoxy group or an aryl group, or an unsubstituted aryl group is more preferable. An aryl group substituted with an alkyl group or an aryl group, or an unsubstituted aryl group is further preferable. Particularly preferred group represented by R² includes a phenyl group, a 4-tolyl group, a 4-butylphenyl group, a 4-t-butylphenyl group, a 4-hexylphenyl group, a 4-octylphenyl group, a 4-(2-ethylhexyl)phenyl group, a 4-(3,7-dimethyloctyl)phenyl group, a 3-tolyl group, a 3-butylphenyl group, a 3-t-butylphenyl group, a 3-hexylphenyl group, a 3-octylphenyl group, a 3-(2-ethylhexyl)phenyl group, a 3-(3,7-dimethyloctyl)phenyl group, a 3,5-dimethylphenyl group, a 3,5-di-(t-butyl)phenyl group, a 3,4-dihexylphenyl group, a 3,4-dioctylphenyl group, a 1-naphthyl group a 2-naphthyl group, a 2-fluorenyl group, a 9,9-dihexyl-2-fluorenyl group, a 9,9-dioctyl-2-fluorenyl group, and a 4-(4'-t-butylbiphenyl) group.

With regard to the group represented by R² in formula (1a), from the viewpoint of the easiness of the synthesis of the polymer compound of the present invention, the formula weight of the group represented by R² in formula (1a) (namely, gram per mole of the group represented by R²) is preferably 500 or less, more preferably 300 or less, and further preferably 200 or less. The lower limit is generally 77.

Next, the "group represented by formula (2)," which is represented by R¹ in formula (1a), will be described.
From the viewpoint of the easiness of the synthesis of the polymer compound of the present invention, the formula weight of the group represented by R¹ in formula (1a) (namely, gram per mole of the group represented by R¹) is preferably 2000 or less, more preferably 1500 or less, and further preferably 1200 or less. The lower limit is generally 407.

The arylene group represented by each of Ar¹ to Ar³ in formula (2) means an atomic group formed by removing two hydrogen atoms from aromatic hydrocarbon, and it includes those having a condensed ring. The arylene group generally contains 6 to 60 carbon atoms. The above-described number of carbon atoms does not include the number of carbon atoms of substituents. The arylene group includes: phenylene groups such as a 1,4-phenylene group, a 1,3-phenylene group or a 1,2-phenylene group; naphthalenediyl groups such as a naphthalene-1,4-diyl group, a naphthalene-1,5-diyl group or a naphthalene-2,6-diyl group; anthracenediyl groups such as an anthracene-1,4-diyl group, an anthracene-1,5-diyl group, an anthracene-2,6-diyl group or an anthracene-9,10-diyl group; phenanthrenediyl groups such as a phenanthrene-2,7-diyl group; naphthacenediyl groups such as a naphthacene-1,7-diyl group or a naphthacene-2,8-diyl group; and fluorenediyl groups such as a fluorene-2,7-diyl group, a 7H-benzo[c]fluorene-5,9-diyl group or a 6,12-dihydro-indeno[1,2-b]fluorene-2,8-diyl group. The arylene group may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom, a cyano group or the like.

In formula (2), a divalent group represented by each of Ar¹ to Ar³, in which two or more identical or different arylene groups are bound to one another by a single bond, includes: biphenyldiyl groups such as a biphenyl-4,4'-diyl group, a biphenyl-3,4'-diyl group or a biphenyl-3,3'-diyl group; and terphenyldiyl groups such as a [1,1';4',1"]terphenyl-4,4"-diyl group. The divalent group may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom, a cyano group or the like.

In formula (2), preferred groups as Ar¹, Ar² and Ar³ include a 1,4-phenylene group, a 1,3-phenylene group, a naphthalene-1,4-diyl group, a naphthalene-1,5-diyl group, a naphthalene-2,6-diyl group, an anthracene-2,6-diyl group, an anthracene-9,10-diyl group, a phenanthrene-2,7-diyl group, a 9,9-dialkylfluorene-2,7-diyl group, a 9,9-diarylfluorene-2,7-diyl group, a 7,7-dialkyl-benzo[c]fluorene-5,9-diyl group, a 7,7-diaryl-benzo[c]fluorene-5,9-diyl group, a 6,6,12,12-tetraalkyl-indeno[1,2-b]fluorene-2,8-diyl group, a 6,6,12,12-tetraaryl-indeno[1,2-b]fluorene-2,8-diyl group, a biphenyl-4,4'-diyl group, a biphenyl-3,4'-diyl group, a biphenyl-3,3'-diyl group and a [1,1';4',1"]terphenyl-4,4"-diyl group. From the viewpoint of the easiness of the synthesis of the polymer compound of the present invention, a 1,4-phenylene group, a 1,3-phenylene group, a biphenyl-4,4'-diyl group, a biphenyl-3,4-diyl group and a biphenyl-3,3'-diyl group are particularly preferable as Ar¹. From the viewpoint of the heat resistance and easy synthesis of the polymer compound of the present invention, a 1,4-phenylene group, a 1,3-phenylene group, an anthracene-9,10-diyl group, a 9,9-dialkylfluorene-2,7-diyl group, a 9,9-diarylfluorene-2,7-diyl group, a biphenyl-4,4'-diyl group, a biphenyl-3,3'-diyl group and a [1,1;4',1"]terphenyl-4,4"-diyl group are particularly preferable as Ar² and Ar³.

In formula (2), the aryl groups and the monovalent aromatic heterocyclic groups represented by Ar⁴, Ar⁵, Ar⁶ and Ar⁷ are the same as those described and exemplified as the aryl groups and the monovalent aromatic heterocyclic groups represented by the above-described R².

From the viewpoint of the heat resistance of the polymer compound of the present invention, as Ar⁴, Ar⁵, Ar⁶ and Ar⁷ in formula (2), an aryl group substituted with an alkyl group, an alkoxy group or an aryl group, or an unsubstituted aryl group is preferable, and an aryl group substituted with an alkyl group or an aryl group, or an unsubstituted aryl group is more preferable. A phenyl group, a C₁-C₁₂ alkoxyphenyl group, a C₁-C₁₂ alkylphenyl group, a 1-naphthyl group, a 2-naphthyl group and a C₁-C₁₂ alkylbiphenyl group are further preferable. A C₁-C₁₂ alkylphenyl group and a C₁-C₁₂ alkylbiphenyl group are particularly preferable.
The C₁-C₁₂ alkylphenyl group includes a 4-tolyl group, a 4-butylphenyl group, a 4-t-butylphenyl group, a 4-hexylphenyl group, a 4-octylphenyl group, a 4-(2-ethylhexyl)phenyl group, a 4-(3,7-dimethyloctyl)phenyl group, a 3-tolyl group, a 3-butylphenyl group, a 3-t-butylphenyl group, a 3-hexylphenyl group, a 3-octylphenyl group, a 3-(2-ethylhexyl)phenyl group, a 3-(3,7-dimethyloctyl)phenyl group, a 3,5-dimethylphenyl group, a 3,5-di-(t-butyl)phenyl group, a 3,4-dihexylphenyl group, a 3,4-dioctylphenyl group, a 1-naphthyl group, a 2-naphthyl group, a 2-fluorenyl group, a 9,9-dihexyl-2-fluorenyl group, and a 9,9-dioctyl-2-fluorenyl group. The above mentioned C₁-C₁₂ alkylbiphenyl group includes a 4-(4'-t-butylbiphenyl) group, and a 4-(4'-hexylbiphenyl) group.

In formula (2), with regard to k and kk, from the viewpoint of the easiness of the synthesis of the polymer compound of the present invention, the value of k + kk is preferably an integer of 1 to 4, more preferably an integer of 1 to 3, and further preferably 1 or 2.

In formula (2), when a group selected from among the groups represented by Ar³, Ar⁶ and Ar⁷ forms a 5- to 7-membered ring by being bound by a single bond or by -O-, -S-, - C(=O)-, -C(=O)-O-, -N(R⁶)-, -C(=O)-N(R⁶)- or -C(R⁶)(R⁶)- to a group that is selected from among the groups represented by Ar¹, Ar², Ar³, Ar⁴, Ar⁵, Ar⁶ and Ar⁷ and that is attached to a nitrogen atom which is the same as that to which the group selected from among the groups represented by Ar³, Ar⁶ and Ar⁷ is attached, the above two groups preferably are bound to each other by a single bond or by -O-, -S- or -C(R⁶)(R⁶)-, so as to form a 5- to 6-membered ring.
The above two groups more preferably are bound to each other by a single bond or by -O- or - C(R⁶)(R⁶)-, so as to form a 5- to 6-membered ring. For example, when both k and kk are 1, and both Ar² and Ar³ are phenyl groups, examples of such combination include: Ar² is bound to Ar³ by a single bond to form a carbazole ring (formula (2-105) as described later, etc.); Ar² is bound to Ar³ by -O- to form a phenoxazine ring (formula (2-107) as described later, etc.); and Ar² is bound to Ar³ by -C(R⁶)(R⁶)- to form a dihydroacridine ring (formula (2-108) as described later, etc.).

The alkyl group represented by R⁶ is the same as the alkyl group described and exemplified in the above-described section of substituents. The aryl group represented by R⁶ is the same as the aryl groups represented by Ar⁴ to Ar⁷, which are described and exemplified above. The monovalent aromatic heterocyclic group represented by R⁶ is the same as the monovalent aromatic heterocyclic group described and exemplified in the above-described section of substituents. Preferred groups represented by R⁶ include an alkyl group and an aryl group.

The group represented by formula (2) includes: those represented by formulae (2-001) to (2-035), (2-101) to (2-117), and (2-201) to (2-212) provided below; and the above mentioned groups substituted with a group selected from the group consisting of an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom and a cyano group.

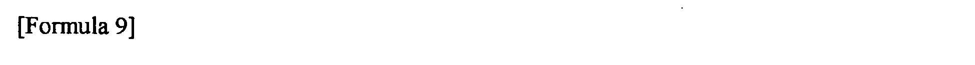

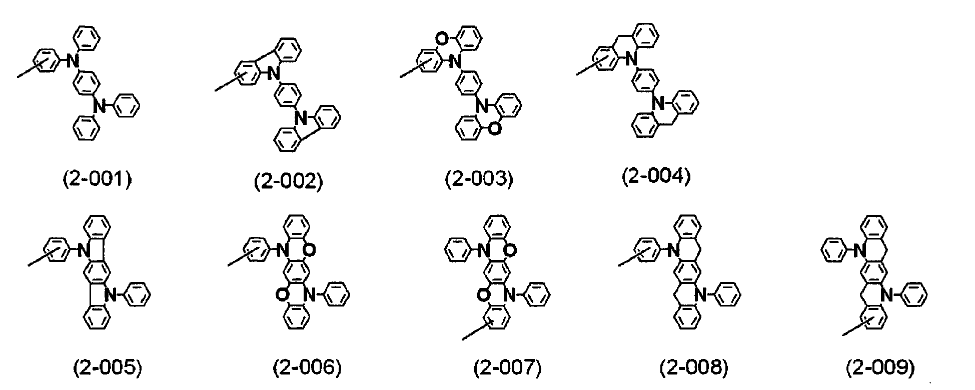

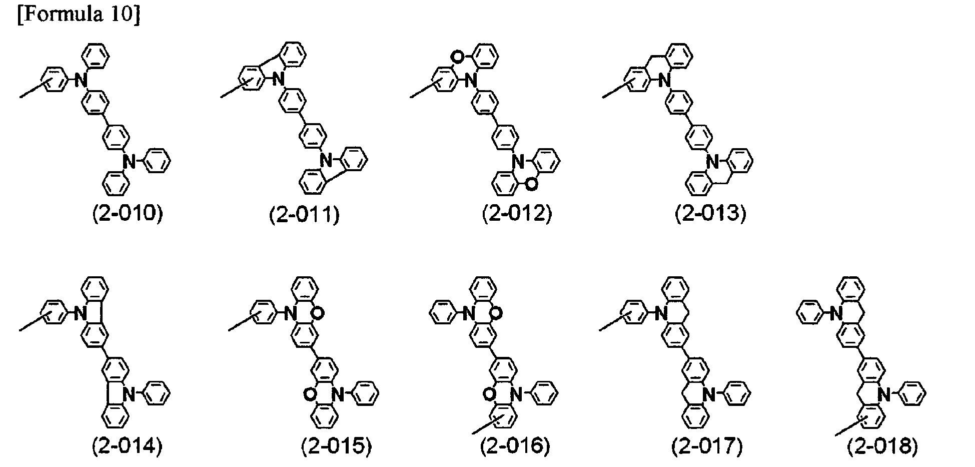

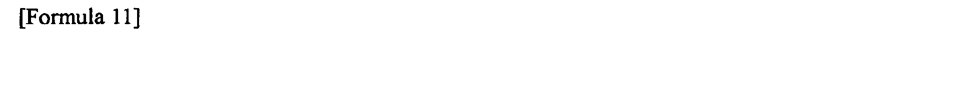

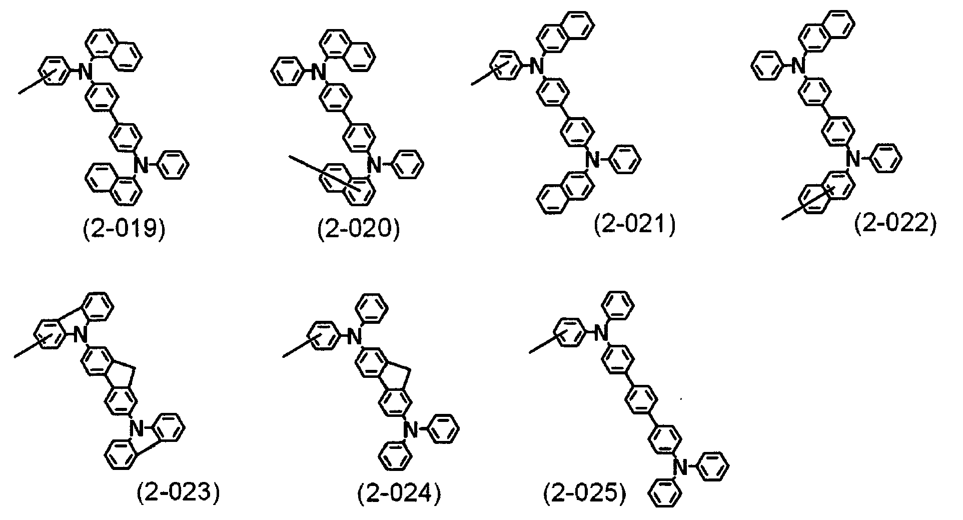

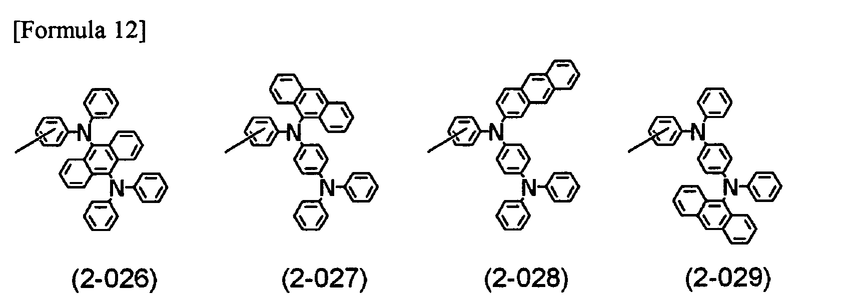

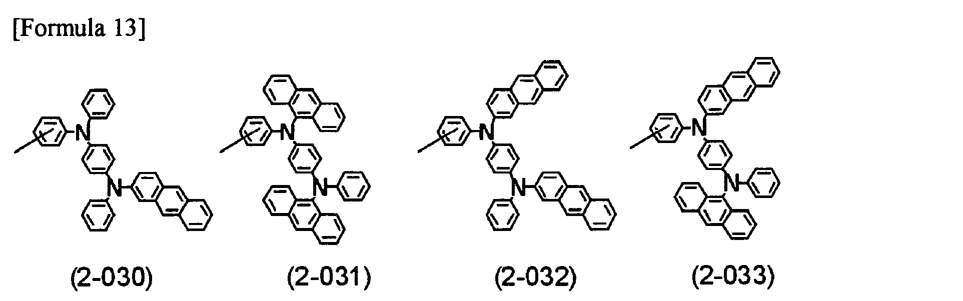

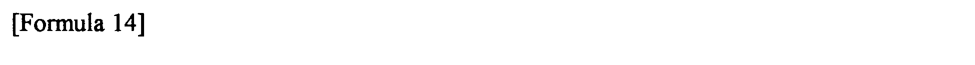

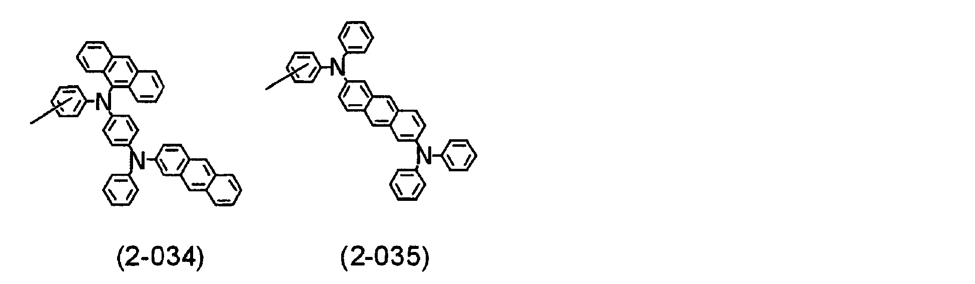

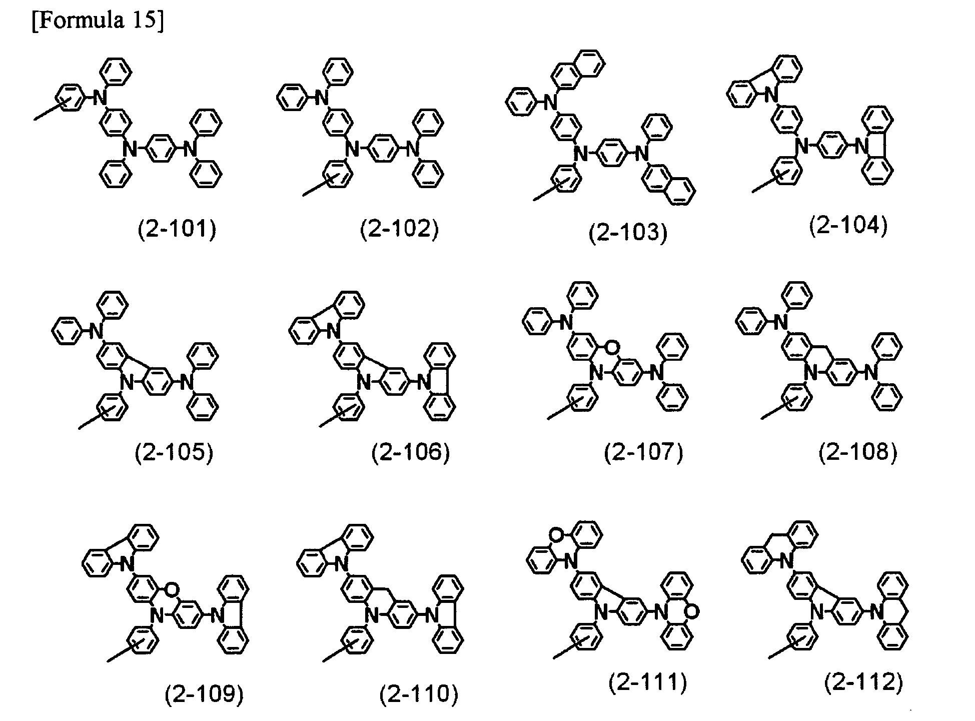

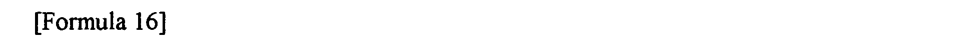

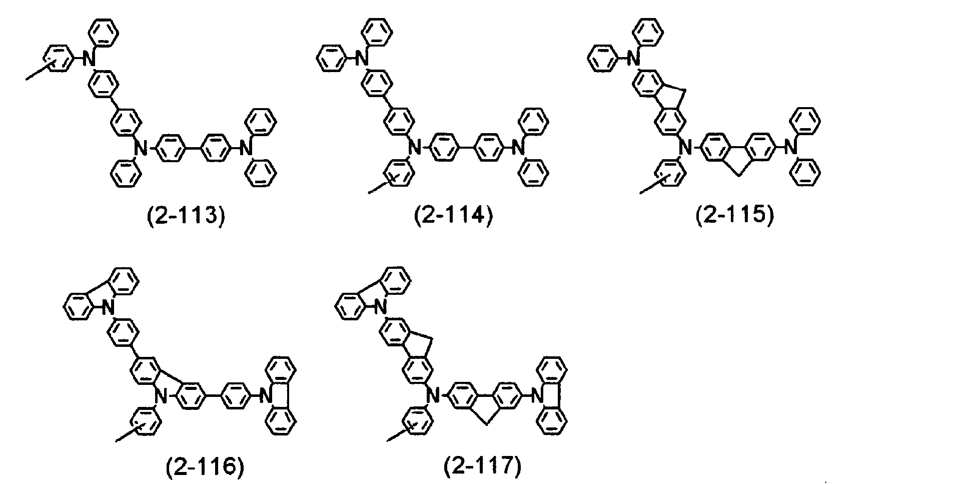

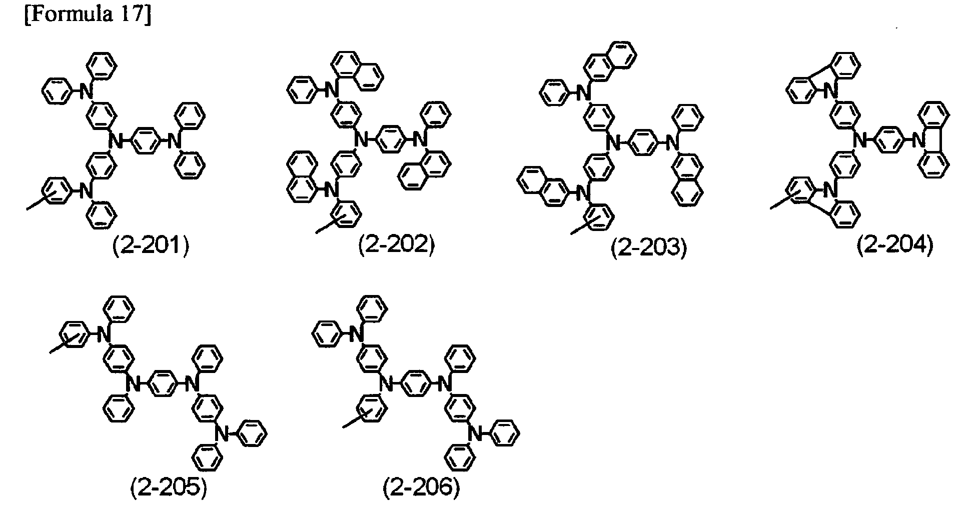

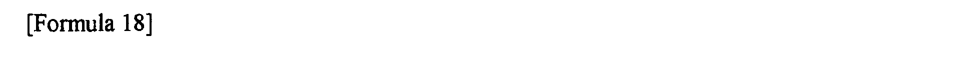

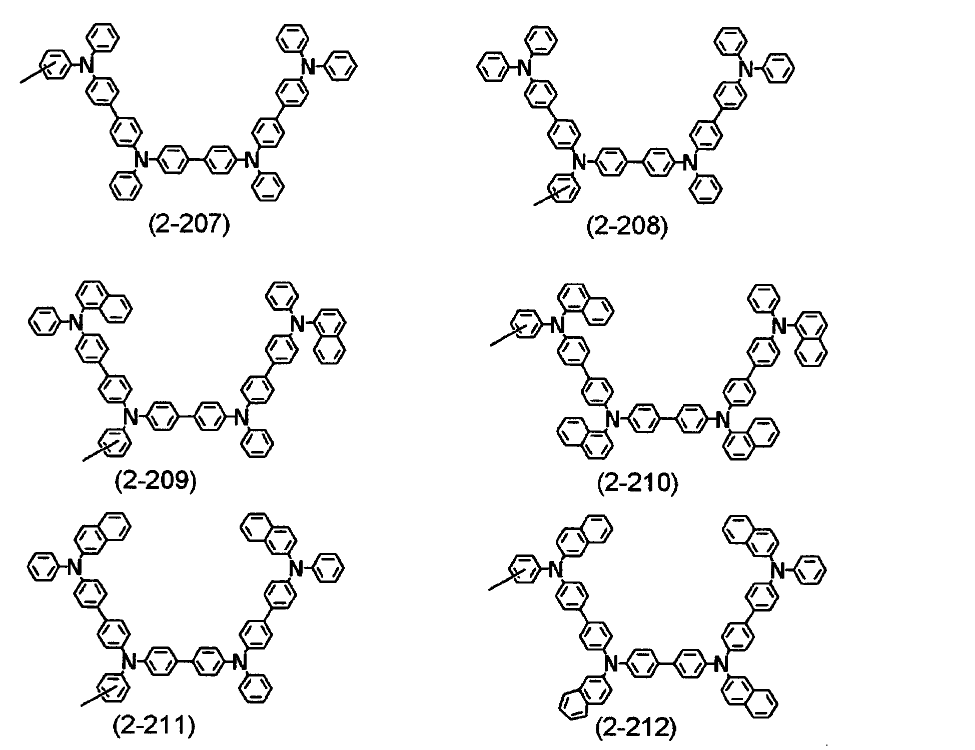

(In the formula, a bond extended from an aromatic ring represents directly the bond itself or a bond by an arylene group.)

### • Constitutional unit represented by formula (1)

From the viewpoint of the easiness of synthesis, the constitutional unit represented by the following formula (1) is preferable as a constitutional unit represented by the above formula (1a): 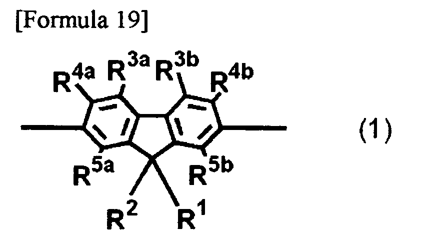 wherein R¹ and R² are the same as above in meaning; each of R^{3a}, R^{4a}, R^{5a}, R^{3b}, R^{4b} and R^{5b} independently represents a hydrogen atom, an alkyl group, an aryl group, a monovalent aromatic heterocyclic group, -N(R⁸)(R⁹), a fluorine atom or a cyano group; each of R⁸ and R⁹ independently represents a hydrogen atom, an alkyl group, an aryl group or a monovalent aromatic heterocyclic group; each of the aryl groups and the monovalent aromatic heterocyclic groups represented by R^{3a}, R^{4a}, R^{5a}, R^{3b}, R^{4b}, R^{5b}, R⁸ and R⁹ may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group; and each of R^{3a} and R^{4a}, R^{3b} and R^{4b}, R^{3a} and R^{3b}, and R⁸ and R⁹, may together form a ring.

In formula (1), the alkyl groups, the aryl groups and the monovalent aromatic heterocyclic groups represented by R^{3a}, R^{4a}, R^{5a}, R^{3b}, R^{4b}, R^{5b}, R⁸ and R⁹ are the same as the alkyl groups, the aryl groups and the monovalent aromatic heterocyclic groups described and exemplified in the above-described section of substituents. The alkyl group, the alkoxy group, the alkylthio group, the substituted carbonyl group, the substituted carboxyl group, the aryl group, the aryloxy group, the arylthio group, the aralkyl group and the monovalent aromatic heterocyclic group, which may be possessed as substituents by the groups represented by R^{3b}, R^{4b}, R^{5b}, R⁸ and R⁹, are the same as those described and exemplified in the above-described section of substituents.

From the viewpoint of the heat resistance of the polymer compound of the present invention, each of R⁸ and R⁹ is preferably a C₁-C₁₂ alkyl group, or an aryl group unsubstituted or substituted with a C₁-C₁₂ alkyl group, and is more preferably an aryl group unsubstituted or substituted with a C₁-C₁₂ alkyl group.

The group represented by -N(R⁸)(R⁹) includes a diphenylamino group, a di-4-tolylamino group, a di-3-tolylamino group, a di-(4-t-butylphenyl)amino group, a di-(4-hexylphenyl)amino group, a bis((3,5-di-t-butyl)phenyl)amino group, a phenyl-1-naphthylamino group, and a phenyl-2-naphthylamino group.

From the viewpoint of the easiness of the synthesis of the polymer compound of the present invention, as R^{3a}, R^{4a}, R^{5a}, R³⁶, R^{4b} and R^{5b}, a hydrogen atom, an alkyl group and an aryl group are preferable, and a hydrogen atom is more preferable.

The constitutional unit represented by formula (1) includes: those represented by formulae (1-001) to (1-018), (1-101) to (1-115), and (1-201) to (1-205) provided below; and the above mentioned groups substituted with a group selected from the group consisting of an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom and a cyano group. It is to be noted that, in the formulae as shown below, Me indicates a methyl group, n-Bu indicates an n-butyl group, and t-Bu indicates a t-Bu group, and that the same applies throughout the following paragraphs.

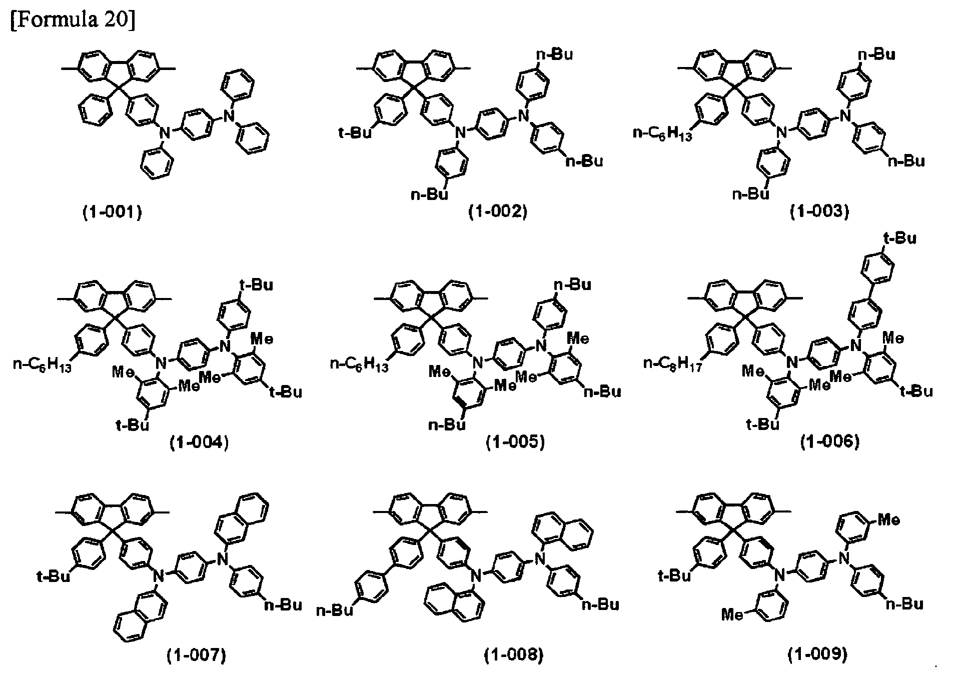

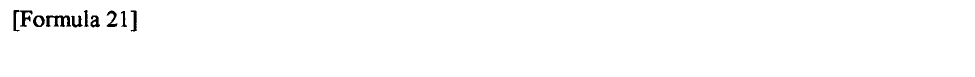

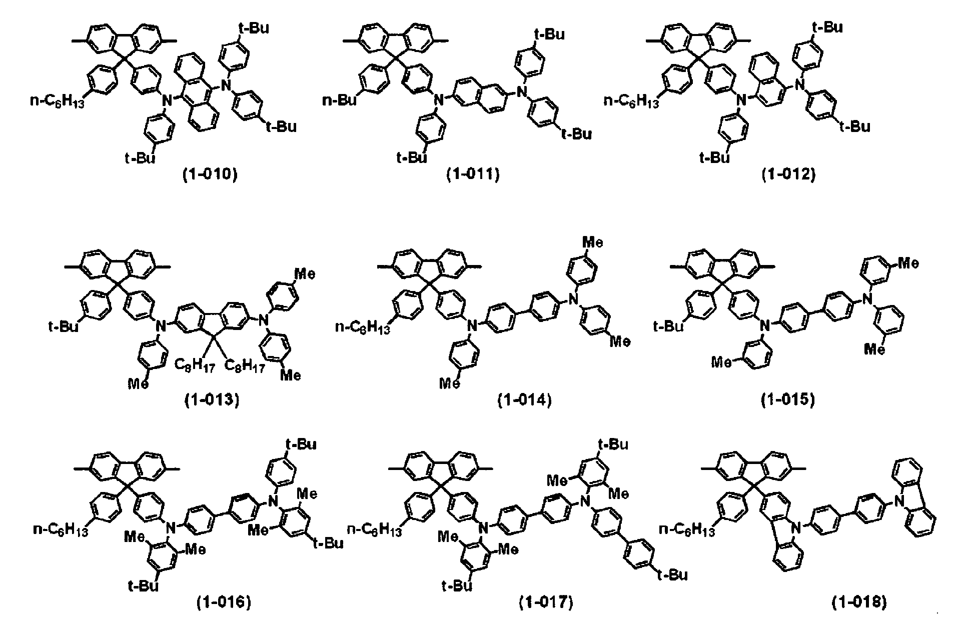

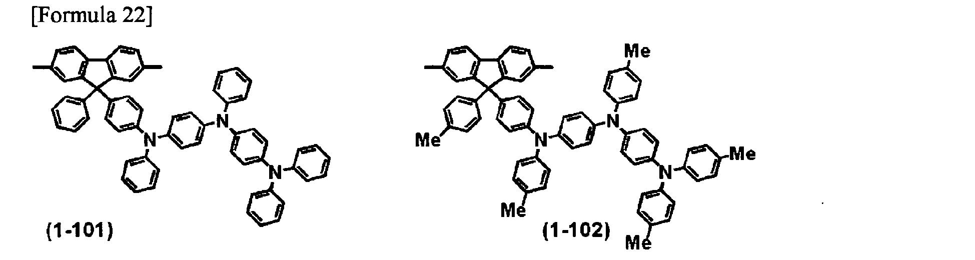

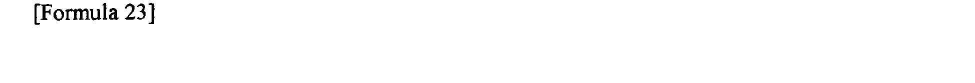

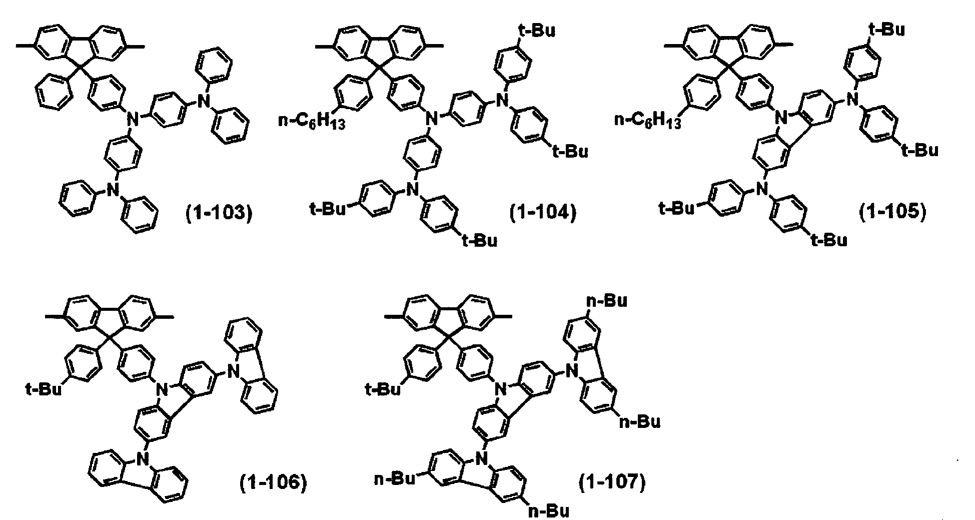

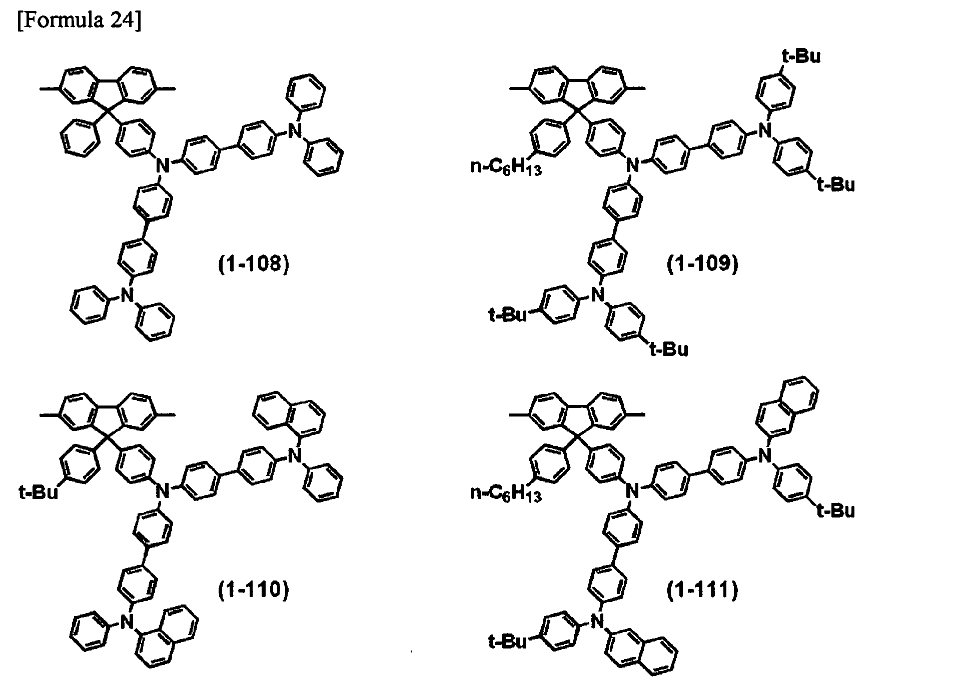

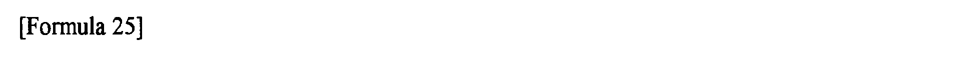

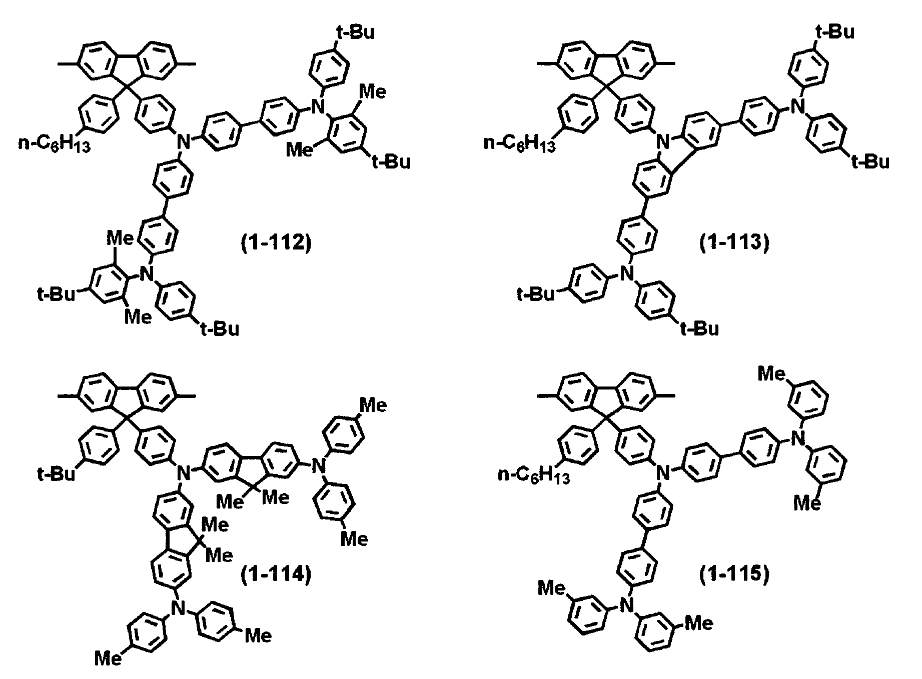

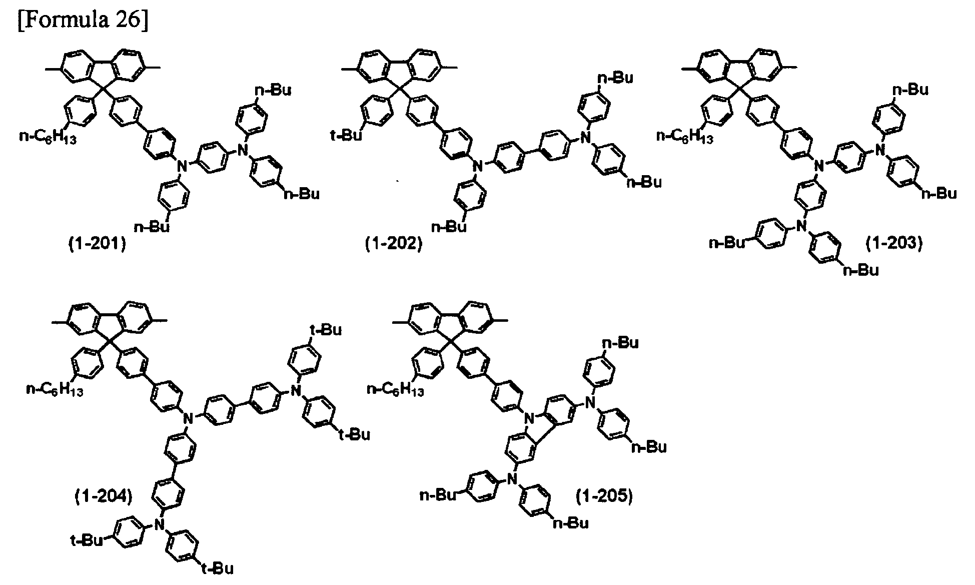

### - Constitutional units represented by formulae (3) to (5) -

The polymer compound of the present invention may further comprise a constitutional unit selected from among the constitutional units represented by the following formulae (3) to (5), as well as the constitutional unit represented by formula (1a).

[Formula 27] **-Ar⁸-** (3)

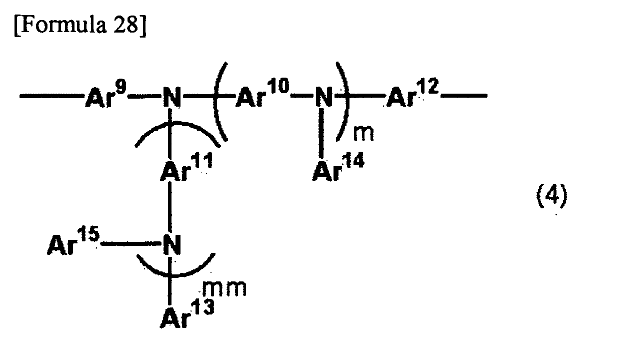

[Formula 29] **-Ar¹⁶-X¹-** (5)

In the formulae, each of Ar⁸ and Ar¹⁶ independently represents an arylene group, or a divalent aromatic heterocyclic group, or a divalent group in which two or more identical or different groups selected from the group consisting of the arylene groups and the divalent aromatic heterocyclic groups are bound to one another by a single bond; each of Ar⁹, Ar¹⁰, Ar¹¹ and Ar¹² independently represents an arylene group or a divalent group in which two or more identical or different arylene groups are bound to one another by a single bond; each of Ar¹³, Ar¹⁴ and Ar¹⁵ independently represents an aryl group or a monovalent aromatic heterocyclic group; each of arylene groups, divalent aromatic heterocyclic groups and the divalent groups represented by Ar⁸ and Ar¹⁶ may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, -N(R⁸)(R⁹), a fluorine atom or a cyano group; each of the groups represented by Ar⁹, Ar¹⁰, Ar¹¹, Ar¹², Ar¹³, Ar¹⁴ and Ar¹⁵ may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group; a group selected from among the groups represented by Ar¹¹, Ar¹⁴ and Ar¹⁵ may form a 5- to 7-membered ring by being bound by a single bond or by -O-, -S-, -C(=O)-, -C(=O)-O-, -N(R⁶)-, -C(=O)-N(R⁶)- or -C(R⁶)(R⁶)- to a group that is selected from among the groups represented by Ar⁹, Ar¹⁰, Ar¹¹, Ar¹², Ar¹³, Ar¹⁴ and Ar¹⁵ and that is attached to a nitrogen atom which is the same as that to which the group selected from among the groups represented by Ar¹¹, Ar¹⁴ and Ar¹⁵ is attached; each of m and mm independently represents 0 or 1; X¹ represents -C(R⁷)=C(R⁷)- or -C≡C-; R⁷ independently represents a hydrogen atom, an alkyl group, an aryl group, a monovalent aromatic heterocyclic group, a fluorine atom or a cyano group; the group represented by R⁷ may have a substituent; if a plurality of Ar¹⁰, Ar¹¹, Ar¹⁴ or Ar¹⁵ are present, they may be identical to or different from one another; and R⁶, R⁸ and R⁹ are the same as above in meaning.

### • Constitutional unit represented by formula (3)

The arylene group represented by Ar⁸ means an atomic group formed by removing two hydrogen atoms from aromatic hydrocarbon, and it includes those having a condensed ring. The arylene group generally contains approximately 6 to 60, preferably 6 to 48, more preferably 6 to 30, and further preferably 6 to 14 carbon atoms. The above-described number of carbon atoms does not include the number of carbon atoms of substituents. Specific examples of the arylene group include: phenylene groups such as a 1,4-phenylene group, a 1,3-phenylene group or a 1,2-phenylene group; naphthalenediyl groups such as a naphthalene-1,4-diyl group, a naphthalene-1,5-diyl group or a naphthalene-2,6-diyl group; anthracenediyl groups such as an anthracene-1,4-diyl group, an anthracene-1,5-diyl group, an anthracene-2,6-diyl group or an anthracene-9,10-diyl group; phenanthrenediyl groups such as a phenanthrene-2,7-diyl group; dihydrophenanthrenediyl groups such as a 4,5-dihydrophenanthrene-2,7-diyl group; naphthacenediyl groups such as a naphthacene-1,7-diyl group, a naphthacene-2,8-diyl group or a naphthacene-5,12-diyl group; fluorenediyl groups such as a fluorene-2,7-diyl group or a fluorene-3,6-diyl group; pyrenediyl groups such as a pyrene-1,6-diyl group, a pyrene-1,8-diyl group, a pyrene-2,7-diyl group or a pyrene-4,9-diyl group; perylenediyl groups such as a perylene-2,5-diyl group, a perylene-2,8-diyl group, a perylene-3,9-diyl group or a perylene-3,10-diyl group; benzofluorenediyl groups such as a 7H-benzo[c]fluorene-5,9-diyl group; and dihydroindenofluorenediyl groups such as a 6,12-dihydro-indeno[1,2-b]fluorene-2,8-diyl group. These arylene groups may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, - N(R⁸)(R⁹), a fluorine atom or a cyano group.

The divalent aromatic heterocyclic group represented by Ar⁸ means an atomic group formed by removing two hydrogen atoms from an aromatic heterocyclic compound, and it includes those having a condensed ring. The above-described divalent aromatic heterocyclic group generally contains approximately 3 to 60, and preferably 3 to 20 carbon atoms. The above-described number of carbon atoms does not include the number of carbon atoms of substituents. The divalent aromatic heterocyclic group includes: an oxadiazole-2,5-diyl group; a thiadiazole-2,5-diyl group; thiazolediyl groups such as a thiazole-2,5-diyl group; oxazolediyl groups such as an oxazole-2,5-diyl group; thiophenediyl groups such as a thiophene-2,5-diyl group; pyrrolediyl groups such as a pyrrole-2,5-diyl group; furandiyl groups such as a furan-2,5-diyl group; pyridinediyl groups such as a pyridine-2,5-diyl group or a pyridine-2,6-diyl group; pyrazinediyl groups such as a pyrazine-2,5-diyl group; pyrimidinediyl groups such as a pyrimidine-4,6-diyl group; a triazine-2,4-diyl group; pyridazinediyl groups such as a pyridazine-3,6-diyl group; quinolinediyl groups such as a quinoline-2,6-diyl group; isoquinolinediyl groups such as an isoquinoline-1,4-diyl group; quinoxalinediyl groups such as a quinoxaline-5,8-diyl group; carbazolediyl groups such as a carbazole-2,7-diyl group or a carbazole-3,6-diyl group; phenoxazinediyl group such as a phenoxazine-3,7-diyl group or a phenoxazine-2,8-diyl group; phenothiazinediyl groups such as a phenothiazine-3,7-diyl group or a phenothiazine-2,8-diyl group; benzothiadiazolediyl groups such as a benzo[1,2,5]thiadiazole-4,7-diyl group; benzothiazolediyl groups such as a benzothiazole-4,7-diyl group; and dibenzosilolediyl groups such as a dibenzosilole-2,7-diyl group or a dibenzosilole-3,6-diyl group. These divalent aromatic heterocyclic groups may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, - N(R⁸)(R⁹), a fluorine atom, a cyano group or the like.

In the above formula (3), Ar⁸ is preferably a 1,4-phenylene group, a 1,3-phenylene group, a naphthalene-1,4-diyl group, a naphthalene-1,5-diyl group, a naphthalene-2,6-diyl group, an anthracene-2,6-diyl group, an anthracene-9,10-diyl group, a fluorene-2,7-diyl group, a fluorene-3,6-diyl group, a pyrene-1,6-diyl group, a pyrene-1,8-diyl group, a perylene-3,9-diyl group, a 7H-benzo[c]fluorene-5,9-diyl group, a 6,12-dihydro-indeno[1,2-b]fluorene-2,8-diyl group, an oxadiazole-2,5-diyl group, a thiadiazole-2,5-diyl group, a thiophene-2,5-diyl group, a pyridine-2,5-diyl group, a quinoline-2,6-diyl group, an isoquinoline-1,4-diyl group, a quinoxaline-5,8-diyl group, a carbazole-2,7-diyl group, a carbazole-3,6-diyl group, a phenoxazine-2,7-diyl group, a phenoxazine-3,6-diyl group, a phenothiazine-2,7-diyl group, a phenothiazine-3,6-diyl group, or a benzo[1,2,5]thiadiazole-4,7-diyl group. Such Ar⁸ is more preferably a 1,4-phenylene group, a naphthalene-1,4-diyl group, a naphthalene-2,6-diyl group, an anthracene-2,6-diyl group, an anthracene-9,10-diyl group, a fluorene-2,7-diyl group, a pyrene-1,6-diyl group, a perylene-3,9-diyl group, a 7H-benzo[c]fluorene-5,9-diyl group, a 6,12-dihydroindeno[1,2-b]fluorene-2,8-diyl group, a quinoline-2,6-diyl group, a quinoxaline-5,8-diyl group, a phenoxazine-3,7-diyl group, a phenothiazine-3,7-diyl group, or a benzo[1,2,5]thiadiazole-4,7-diyl group. These groups may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, - N(R⁸)(R⁹), a fluorine atom, a cyano group or the like. Ar⁸ is preferably a divalent group represented by any one of the following formulae (3a) to (3g), more preferably a divalent group represented by any one of the following formulae (3a) to (3e), and particularly preferably a divalent group represented by the following formula (3b). In addition, Ar⁸ is also preferably a divalent group represented by the following formula (3f).

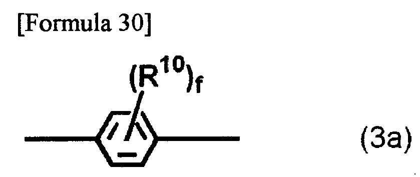

In the formula, R¹⁰ represents an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, -N(R⁸)(R⁹), a fluorine atom or a cyano group; f represents an integer of 0 to 4; and if a plurality of R¹⁰s are present, they may be identical to or different from one another.

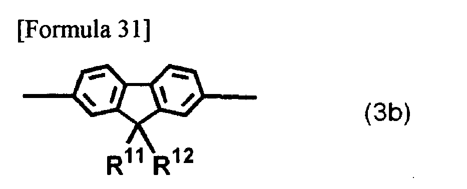

In the formula, each of R¹¹ and R¹² independently represents a hydrogen atom, an alkyl group, an aryl group, an aralkyl group or a monovalent aromatic heterocyclic group, and R¹¹ and R¹² may together form a ring.

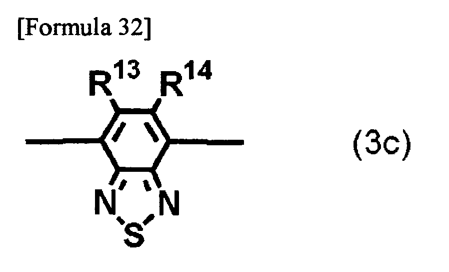

In the formula, each of R¹³ and R¹⁴ independently represents a hydrogen atom, an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, -N(R⁸)(R⁹), a fluorine atom or a cyano group.

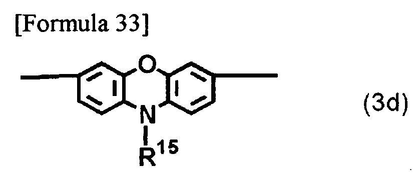

In the formula, R¹⁵ represents a hydrogen atom, an alkyl group, an aryl group, a monovalent aromatic heterocyclic group or an aralkyl group.

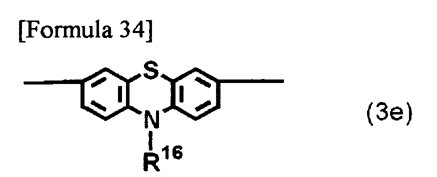

In the formula, R¹⁶ represents a hydrogen atom, an alkyl group, an aryl group, a monovalent aromatic heterocyclic group or an aralkyl group.

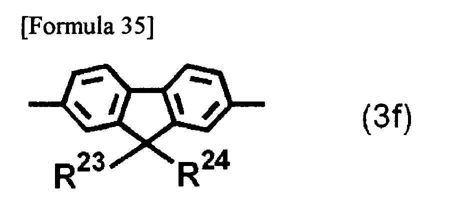

In the formula, R²³ represents a hydrogen atom, an alkyl group, an aryl group, an aralkyl group or a monovalent aromatic heterocyclic group; R²⁴ is a group represented by the following formula (2a); and R²³ and R²⁴ may be identical to or different from each other.

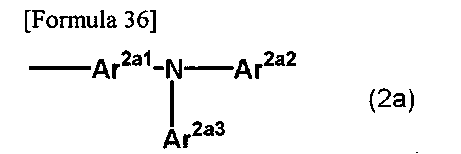

In the formula, Ar^{2a1} represents an arylene group, or a divalent group in which two or more identical or different arylene groups are bound to one another by a single bond; each of Ar^{2a2} and Ar^{2a3} independently represents an aryl group or a monovalent aromatic heterocyclic group; a group selected from among the groups represented by Ax^{2a2} and Ar^{2a3} may form a 5- to 7-membered ring by being bound by a single bond or by -O-, -S-, -C(=O)-, -C(=O)-O-, -N(R^{2a6})-, - C(=O)-N(R^{2a6})- or -C(R^{2a6})(R^{2a6})- to a group that is selected from among the groups represented by Ar^{2a1}, Ar^{2a2} and Ar^{2a3} and that is attached to a nitrogen atom which is the same as that to which the group selected from among the groups represented by Ar^{2a2} and Ar^{2a3} is attached; R^{2a6} represents a hydrogen atom, an alkyl group, an aryl group or a monovalent aromatic heterocyclic group; and each of the groups represented by Ar^{2a1}, Au^{2a2}, Ar^{2a3} and Ar^{2a6} may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group.

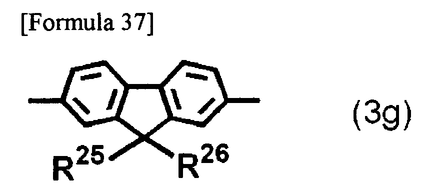

In the formula, each of R²⁵ and R²⁶ independently represents a group represented by the following formula (2b); and R²⁵ and R²⁶ may be identical to or different from each other.

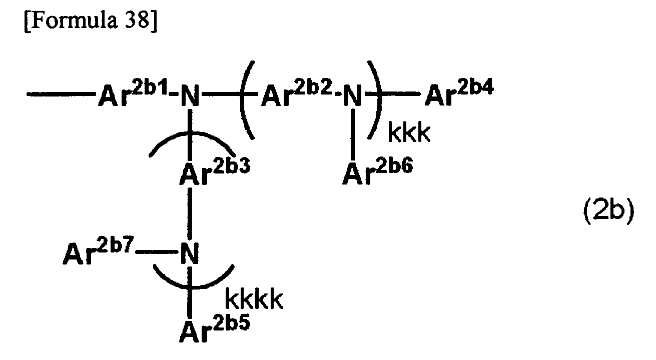

In the formula, each of Ar^{2b1}, Ar^{2b2} and Ar^{2b3} independently represents an arylene group, or a divalent group in which two or more identical or different arylene groups are bound to one another by a single bond, each of Ar^{2b4}, Ar^{2b5}, Ar^{2b6} and Ar^{2b7} independently represents an aryl group or a monovalent aromatic heterocyclic group; a group selected from among the groups represented by Ar^{2b3}, Ar^{2b6} and Ar^{2b7} may form a 5- to 7-membered ring by being bound by a single bond or by -O-, -S-, -C(=O)-, -C(=O)-O-, -N(R^{2b6})-, -C(=O)-N(R^{2b6})- or -C(W^{2b6})(R^{2b6})-to a group that is selected from among the groups represented by Ar^{2b1}, Ar^{2b2}, Ar^{2b3}, Ar^{2b4}, Ar^{2b5}, Ar^{2b6} and Ar^{2a7} and that is attached to a nitrogen atom which is the same as that to which the group selected from among the gruops represented by Ar^{2b3}, Ar^{2b6} and Ar^{2b7} is attached; R^{2b6} represents a hydrogen atom, an alkyl group, an aryl group or a monovalent aromatic heterocyclic group; and each of the groups represented by Ar^{2b1}, Ar^{2b2}, Ar^{2b3}, Ar^{2b4}, Ar^{2b5}, Ar^{2b6}, Ar^{2b7} and R^{2b6} may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group; each of kkk and kkkk independently represents an integer of 0 to 3; and when a plurality of A^{2b2}, Ar^{2b3}, Ar^{2b6}, Ar^{2b7} or R^{2b6} are present, they may be identical to or different from one another.

In the above formula (3a), R¹⁰ preferably represents an alkyl group, an alkoxy group, a substituted carbonyl group, an aryl group, an aryloxy group, an aralkyl group, a monovalent aromatic heterocyclic group or -N(R⁸)(R⁹). Such R¹⁰ is more preferably an alkyl group, an alkoxy group, an aryl group, an aralkyl group, a monovalent aromatic heterocyclic group or -N(R⁸)(R⁹), further preferably an alkyl group, an alkoxy group, an aryl group or a monovalent aromatic heterocyclic group, and particularly preferably an alkyl group, an alkoxy group or an aryl group. The above-described alkyl group, alkoxy group, alkylthio group, substituted carbonyl group, substituted carboxyl group, aryl group, aryloxy group, arylthio group, aralkyl group, and monovalent aromatic heterocyclic group are the same as those described and exemplified as substituents for R² in the above formula (1a). The groups represented by -N(R⁸)(R⁹) are the same as those described and exemplified in the description of the above formula (1).

In the above formula (3a), f is preferably an integer of 1 to 4, more preferably an integer of 1 to 3, further preferably an integer of 1 or 2, and particularly preferably 2.

R¹¹ and R¹² in the above formula (3b), R¹⁵ in the above formula (3d), R¹⁶ in the above formula (3e), and R²³ in the above formula (3f) are each preferably an alkyl group, an aryl group or a monovalent aromatic heterocyclic group, and more preferably an alkyl group or an aryl group. The above-described alkyl group, aryl group, monovalent aromatic heterocyclic group and aralkyl group are the same as those described and exemplified as substituents for R² in the above formula (1a).

The arylene groups, or the divalent groups in which two or more identical or different arylene groups are bound to one another by a single bond, which are represented by Ar^{2a1} in the above formula (2a) and by Ar^{2b1}, Ar^{2b2} and Ar^{2b3} in the above formula (2b), are the same as those described and exemplified as the above-described arylene groups, or the above-described divalent groups in which two or more identical or different arylene groups are bound to one another by a single bond, which are represented by Ar¹, Ar² and Ar³.

The aryl groups or the monovalent aromatic heterocyclic groups represented by Ar^{2a2} and Ar^{2a3} in the above formula (2a) and by Ar^{2b4}, Ar^{2b5}, Ar^{2b6} and Ar^{2b7} in the above formula (2b) are the same as those described and exemplified as the aryl groups and the monovalent aromatic heterocyclic groups represented by the above-described R².

R^{2a6} in the above formula (2a) and R^{2b6} in the above formula (2b) are each preferably an alkyl group, an aryl group or a monovalent aromatic heterocyclic group, and more preferably an alkyl group or an aryl group. The above-described alkyl group, aryl group, and monovalent aromatic heterocyclic group are the same as those described and exemplified as R⁶ in the above formula (2).

R¹³ and R¹⁴ in the above formula (3c) are each preferably a hydrogen atom, an alkyl group, an alkoxy group, a substituted carbonyl group, an aryl group, an aryloxy group, an aralkyl group or a monovalent aromatic heterocyclic group, more preferably a hydrogen atom or an alkyl group, and further preferably a hydrogen atom. The above-described alkyl group, alkoxy group, alkylthio group, substituted carbonyl group, substituted carboxyl group, aryl group, aryloxy group, arylthio group, aralkyl group and monovalent aromatic heterocyclic group are the same as those described and exemplified as substituents for R² in the above formula (1a). The group represented by -N(R⁸)(R⁹) is the same as that described and exemplified in the description of the above formula (1).

### • Constitutional unit represented by formula (4)

In the above formula (4), the arylene groups, or the divalent groups in which two or more identical or different arylene groups are bound to one another by a single bond, which are represented by Ar⁹ to Ar¹², are the same as those described and exemplified as Ar¹ to Ar³ in the above formula (2).

In the above formula (4), the aryl groups or the monovalent aromatic heterocyclic groups represented by Ar¹³ to Ar¹⁵ are the same as those described and exemplified as Ar⁴ to Ar⁷ in the above formula (2).

Examples of the constitutional unit represented by the above formula (4) include constitutional units represented by formulae (4a) to (4d) provided below. In these formulae, R^{a} represents a hydrogen atom, an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group. If a plurality of R^{a}s are present, they may be identical to or different from one another. The above-described alkyl group, alkoxy group, alkylthio group, substituted carbonyl group, substituted carboxyl group, aryl group, aryloxy group, arylthio group, aralkyl group and monovalent aromatic heterocyclic group are the same as those described and exemplified in the section of substituents in a case in which R² in the above formula (1a) has a substituent.

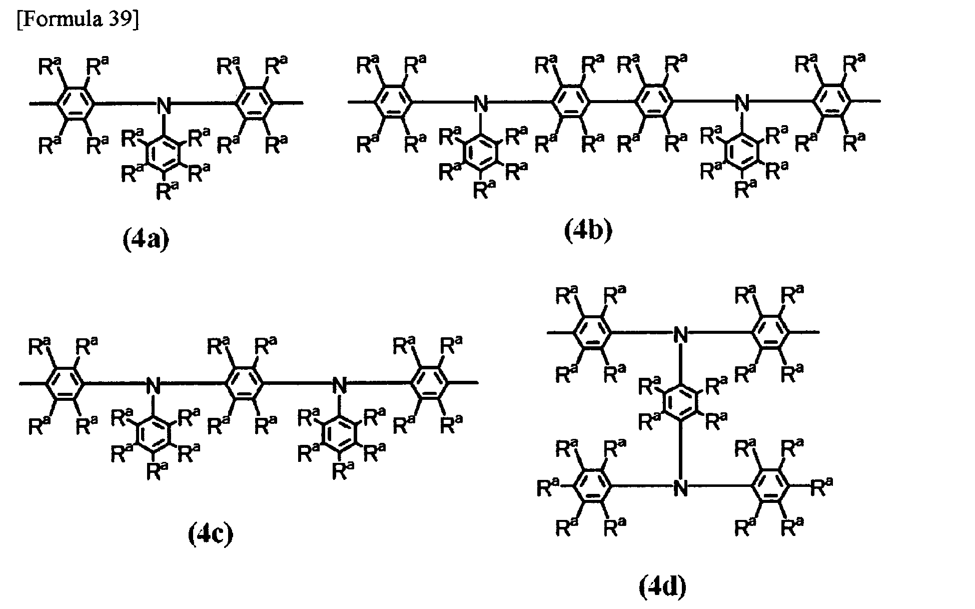

### • Constitutional unit represented by formula (5)

The arylene group, the divalent aromatic heterocyclic group, and the divalent group in which two or more identical or different groups selected from the group consisting of the arylene groups and the divalent aromatic heterocyclic groups are bound to one another by a single bond, which are represented by Ar¹⁶ in the above formula (5), are the same as those described and exemplified as the above-described Ar⁸.

In the above formula (5), R⁷ is preferably a hydrogen atom, an alkyl group or an aryl group, and more preferably a hydrogen atom or an aryl group. The above-described alkyl group and aryl group may have a substituent. The above-described alkyl group, aryl group, and monovalent aromatic heterocyclic group are the same as those described and exemplified as substituents for R² in the above formula (1a).

As the constitutional units represented by the above formula (5), those represented by the following formulae (5a) to (5k) are preferable.

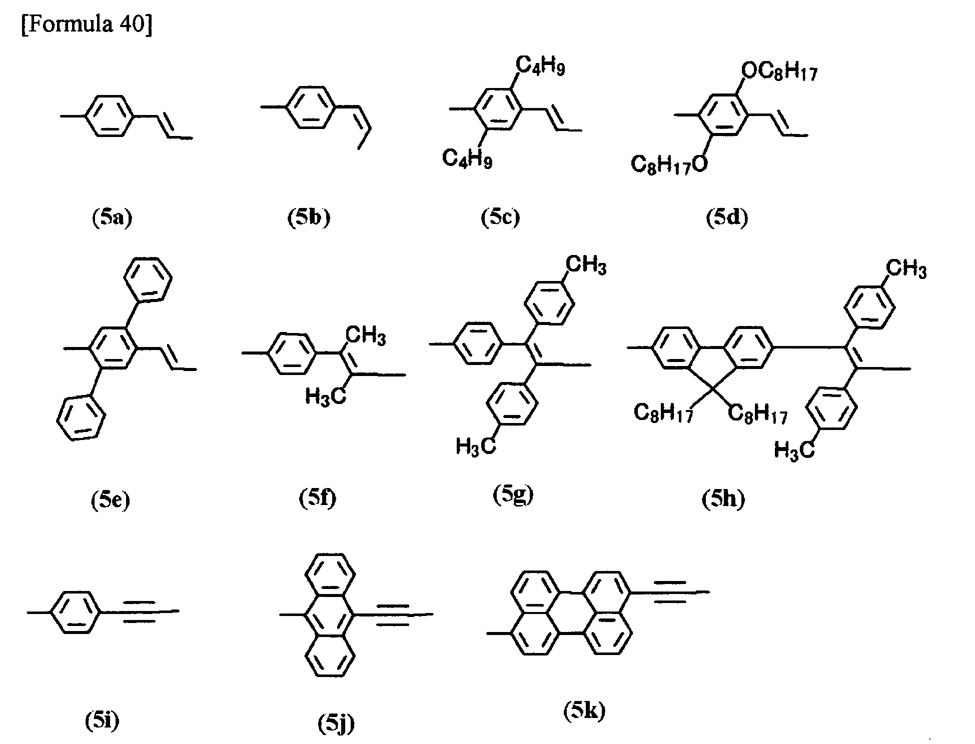

### - The polymer compound of the present invention -

From the viewpoint of the luminous efficiency of a light-emitting device, the number of moles of the constitutional unit represented by formula (1a) to the total number of moles of all constitutional units is preferably 1% to 100%, more preferably 2% to 80%, and further preferably 3% to 50% in the polymer compound of the present invention.

When the polymer compound of the present invention comprises the constitutional units represented by the above formulae (3) to (5) as well as the constitutional unit represented by the above formula (1a) (which further comprises other constitutional units in some cases), from the viewpoint of the luminous efficiency of a light-emitting device, the total number of moles of the constitutional units represented by formulae (1a) and (3) to (5) to the total number of moles of all constitutional units is preferably 90% to 100%, more preferably 95% to 100%, further preferably 98% to 100%, and particularly preferably 100%.

Moreover, When the polymer compound of the present invention comprises the constitutional units represented by the above formulae (3) and (4) as well as the constitutional unit represented by the above formula (1a) (which further comprises other constitutional units in some cases), from the viewpoint of the heat resistance of the polymer compound and the luminous efficiency of a light-emitting device, the total number of moles of the constitutional units represented by formulae (1a), (3) and (4) to the total number of moles of all constitutional units is preferably 90% to 100%, and more preferably 95% to 100%.

In addition to the above-described polymer compounds, the polymer compound of the present invention includes: a polymer compound consisting of the constitutional unit represented by the above formula (1); a polymer compound consisting of the constitutional units represented by the above formulae (1) and (3a); a polymer compound consisting of the constitutional units represented by the above formulae (1), (3a) and (3b); a polymer compound consisting of the constitutional units represented by the above formulae (1), (3a) and (4a); a polymer compound consisting of the constitutional units represented by the above formulae (1), (3a), (3b) and (4a); a polymer compound consisting of the constitutional units represented by the above formulae (1) and (3b); a polymer compound consisting of the constitutional units represented by the above formulae (1), (3b) and (3c); a polymer compound consisting of the constitutional units represented by the above formulae (1), (3b), (3c) and (3d); a polymer compound consisting of the constitutional units represented by the above formulae (1), (3b), (3c) and (4d); a polymer compound consisting of the constitutional units represented by the above formulae (1), (3b) and (3d); a polymer compound consisting of the constitutional units represented by the above formulae (1), (3b) and (3e); a polymer compound consisting of the constitutional units represented by the above formulae (1), (3b) and (4a); a polymer compound consisting of the constitutional units represented by the above formulae (1), (3b) and (4b); a polymer compound consisting of the constitutional units represented by the above formulae (1), (3b) and (4c); a polymer compound consisting of the constitutional units represented by the above formulae (1), (3b) and (4d); a polymer compound consisting of the constitutional units represented by the above formulae (1) and (4a); a polymer compound consisting of the constitutional units represented by the above formulae (1) and (4b); a polymer compound consisting of the constitutional units represented by the above formulae (1) and (4c); and a polymer compound consisting of the constitutional units represented by the above formulae (1) and (4d). Specific examples are given below. In the following formulae, each of v/w, v'/w'/x', and v"/w"/x" indicates the molar ratio among two or three constitutional units.

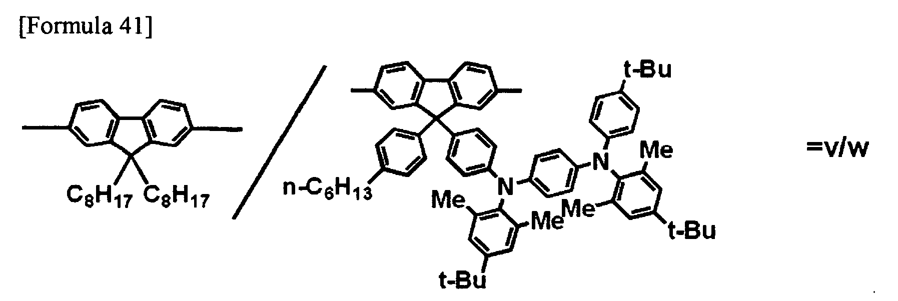

(In the formula, v represents a number of 0 to 0.99, w represents a number of 0.01 to 1, and v + w =1.) 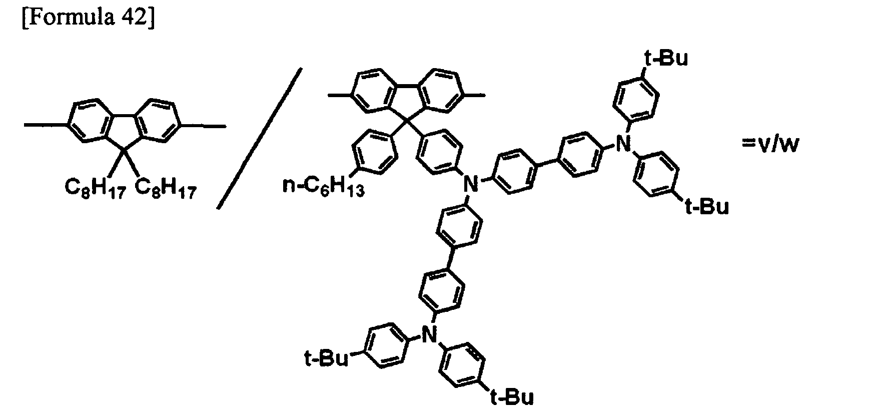 (In the formula, v represents a number of 0 to 0.99, w represents a number of 0.01 to 1, and v + w =1.) 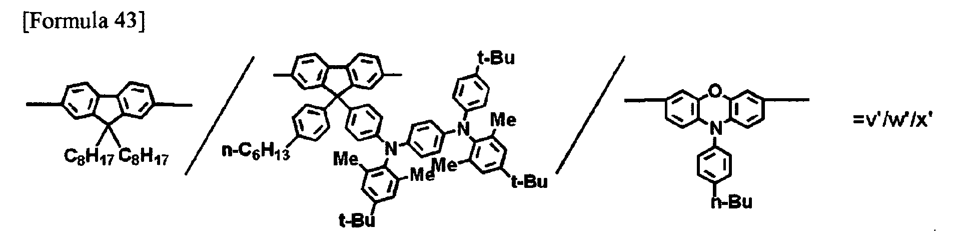 (In the formula, v' represents a number of 0 to 0.98, w' represents a number of 0.01 to 0.99, x' represents a number of 0.01 to 0.50, and v' + w' + x' =1.) 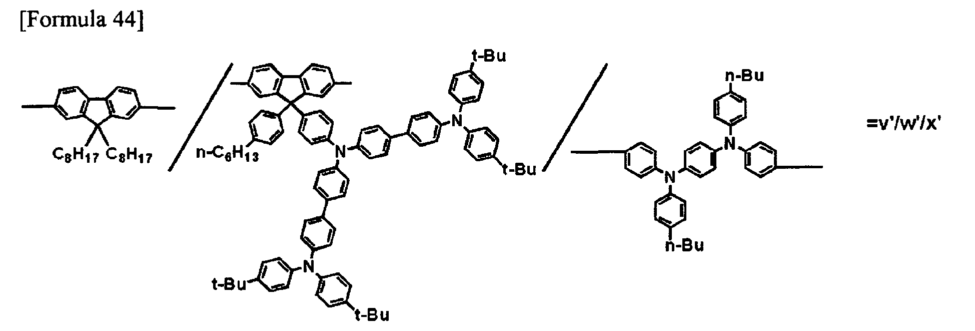 (In the formula, v' represents a number of 0 to 0.98, w' represents a number of 0.01 to 0.99, x' represents a number of 0.01 to 0.50, and v' + w' + x' =1.) 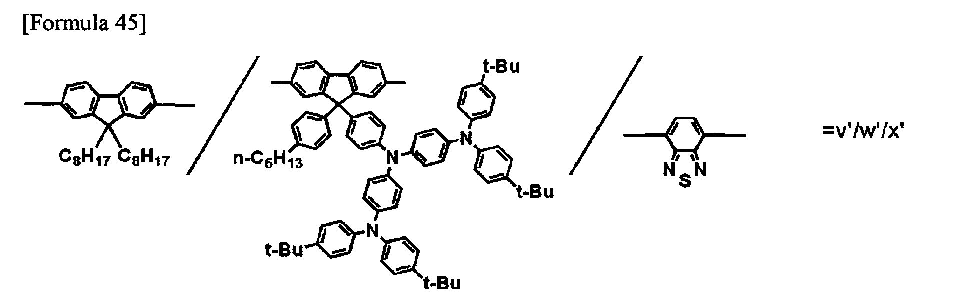 (In the formula, v' represents a number of 0 to 0.98, w' represents a number of 0.01 to 0.99, x' represents a number of 0.01 to 0.50, and v' + w' + x' =1.) 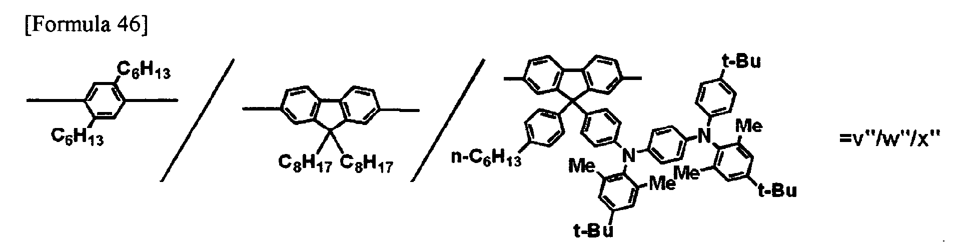 (In the formula, v" represents a number of 0.10 to 0.50, w" represents a number of 0 to 0.80, x" represents a number of 0.10 to 0.50, and v" + w" + x" =1.)

If a polymeric active group remains at the end group of the polymer compound of the present invention, the use of the aforementioned compound in the production of a light-emitting device can reduce its light-emitting properties or lifetime. Accordingly, the end group of the polymer compound of the present invention is preferably a stable group. A group bound to a main chain by a conjugated bond is preferable. An example is a structure in which such group is bound to an aryl group or a monovalent aromatic heterocyclic group by a carbon-carbon bond. The aryl group and the monovalent aromatic heterocyclic group exemplified as such end group are the same as those described and exemplified as the aryl group and the monovalent aromatic heterocyclic group represented by the above-described R².

The polymer compound of the present invention may comprise the constitutional unit represented by formula (1a) and the constitutional units represented by formulae (3) to (5), singly or in combination of two or more types.

The polymer compound of the present invention may be a polymer having any form, such as a linear polymer, a branched polymer, a hyper-branched polymer, a cyclic polymer, a comb-shaped polymer, a star polymer or a network polymer. In addition, it may also be a polymer having any composition and regularity, such as a homopolymer, an alternating copolymer, a periodic copolymer, a random copolymer, a block copolymer or a graft copolymer, having any of the above forms.

The polymer compound of the present invention is useful as a light-emitting material, a charge transport material and the like, and when used, it may be combined with other compounds (that is, it may be used as a composition as described later).

The polystyrene equivalent number average molecular weight (Mn) of the polymer compound of the present invention according to gel permeation chromatography (hereinafter referred to as "GPC") is generally approximately 1 × 10³ to 1 × 10⁸, and preferably 1 × 10⁴ to 1 × 10⁶. On the other hand, the polystyrene equivalent weight average molecular weight (Mw) of the polymer compound of the present invention is generally approximately 1 × 10³ to 1 × 10⁸. From the viewpoint of film-forming properties and the light-emitting properties of a light-emitting device, it is preferably 1 × 10⁴ to 5 × 10⁶, more preferably 3 × 10⁴ to 1 × 10⁶, and further preferably 5 × 10⁴ to 5 × 10⁵.

From the viewpoint of durability necessary for various processes for producing a light-emitting device and the like, and stability and heat resistance to heating during the operation of such light-emitting device, the glass transition temperature of the polymer compound of the present invention is preferably 100°C or higher.

In general, the polymer compound of the present invention emits fluorescence or phosphorescence in a solid state, and it is useful as a material for a light-emitting device. A light-emitting device comprising this polymeric compound has high performance capable of operation at high luminous efficiency. Accordingly, this light-emitting device is useful as a backlight for liquid crystal display, a curved or planar light source for lighting, a segment-type display device, and a display device such as a flat panel display of dot matrix. Moreover, the polymer compound of the present invention can also be used as a laser dye, a material for organic solar battery, an organic semiconductor for organic transistor, a conductive thin film, a material for conductive thin film, such as an organic semiconductor thin film, or a light-emitting thin film material that emits fluorescence or phosphorescence.

### • Method for producing polymer compound

The polymer compound of the present invention can be synthesized, for example, by dissolving a monomer represented by the above-described formula (A) having a functional group suitable for a polymerization reaction to be applied, and as necessary, a compound selected from among compounds represented by the formulae (M-1) to (M-3) as described below, in an organic solvent, as necessary, and then by performing polymerization or copolymerization according to a known polymerization method such as aryl coupling, using an alkali, a suitable catalyst, and a ligand.

[Formula 47] **X^{a}-Ar⁸-X^{b}** (M-1)

(In the formula, Ar⁸, X^{a} and X^{b} are the same as above in meaning.) 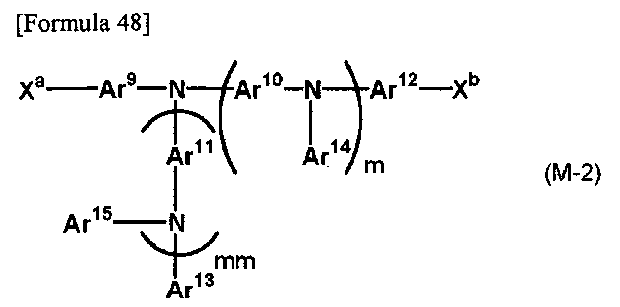 (In the formula, Ar⁹, Ar¹⁰, Ar¹¹, Ar¹², Ar¹³, Ar¹⁴, Ar¹⁵, m, mm, X^{a} and X^{b} are the same as above in meaning.)

[Formula 49] **X^{a}-Ar¹⁶-X¹-X^{b}** (M-3)

(In the formula, Ar¹⁶, X¹, X^{a} and X^{b} are the same as above in meaning.)

In the formula (A), each of the alkyl groups represented by R²⁰, R²¹ and R²² may independently be any one of linear, branched and cyclic groups. The number of carbon atoms is generally approximately 1 to 20, preferably 1 to 15, and more preferably 1 to 10.

In the formula (A), the alkyl group represented by the above-described R²⁰ is the same as that described and exemplified in the above-described description of substituents for the aryl group represented by R².

In the formula (A), the aryl group represented by R²⁰ is the same as that described and exemplified as the aryl group represented by R². From the viewpoint of the easiness of the synthesis of the polymer compound of the present invention and reactivity in polymerization, a phenyl group, a 4-tolyl group, a 4-methoxyphenyl group, a 4-nitrophenyl group, a 3-nitrophenyl group, a 2-nitrophenyl group, and a 4-trifluoromethylphenyl group are particularly preferable.

In the formula (A), the group represented by the above-described -O-S(=O)₂R²⁰ includes a methanesulfonate group, a trifluoromethanesulfonate group, a phenylsulfonate group, a 4-methylphenylsulfonate group, and a 4-trifluoromethylphenylsulfonate group.

In the formula (A), the group represented by the above-described -B(OR²¹)₂ includes groups represented by the following formulae.

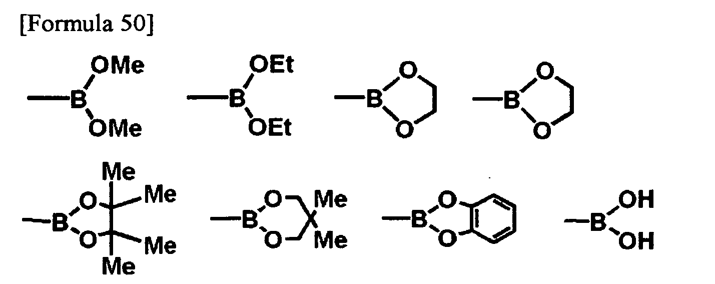

In the formula (A), the group represented by the above-described -BF₄Q¹ includes a group represented by the following formula.

[Formula 51] **-BF₄⁻K⁺**

In the formula (A), the group represented by the above-described -Sn(R²²)₃ includes a trimethylstannanyl group, a triethylstannanyl group, and a tributylstannanyl group.

The compounds represented by the formulae (A) and (M-1) to (M-3) may be previously synthesized and isolated and may be then used. Otherwise, the compounds may be synthesized in a reaction system and may be directly be used. When the polymer compound of the present invention is used for a light-emitting device, since the purity thereof has an influence on the performance of the device with light-emitting properties, it is preferable that, before polymerization, a monomer be purified by a method such as distillation, sublimation purification or recrystallization, and that it be then subjected to condensation polymerization.

Examples of the above-mentioned condensation polymerization method include: a polymerization method that utilizes a Suzuki coupling reaction (Chem. Rev., Vol. 95, pp. 2457-2483 (1995)); a polymerization method that utilizes a Grignard reaction (Bull. Chem. Soc. Jpn., Vol. 51, p. 2091 (1978)); a polymerization method using an Ni(0) catalyst (Progress in Polymer Science, Vol. 17, pp. 1153-1205, 1992); and a method that utilizes a Stille coupling reaction (European Polymer Journal), Vol. 41, pp. 2923-2933 (2005)). From the viewpoint of the easiness of the synthesis of raw materials and simple operations for polymerization reaction, the polymerization method that utilizes a Suzuki coupling reaction and the polymerization method using an Ni(0) catalyst are preferable. From the viewpoint of easy control of the structure of the polymer compound, the polymerization method that utilizes a Suzuki coupling reaction is more preferable.

In the above formulae (M-1) to (M-3), from the viewpoint of the easiness of the synthesis of the polymer compound of the present invention, each of the above-described X^{a} and X^{b} is preferably a bromine atom, an iodine atom, a chlorine atom, -B(OR²¹)₂, -BF₄Q¹ or - Sn(R²²)₃. In particular, when the polymerization method that utilizes a Suzuki coupling reaction is selected as the above-described condensation polymerization method, from the viewpoint of the simple synthesis of the compounds represented by the above-described formulae (A) and (M-1) to (M-3) and handleability, a bromine atom, an iodine atom, a chlorine atom, or -B(OR²¹)₂ is more preferable. Bromine atom or -B(OR²¹)₂ is further preferable.

As the above-described condensation polymerization method, there is applied a method of reacting the compounds represented by the above-described formulae (A) and (M-1) to (M-3), as necessary, together with an appropriate catalyst or an appropriate base. In particular, when the polymerization method that utilizes a Suzuki coupling reaction is selected as the above-described condensation polymerization method, in order to allow the polymer compound of the present invention to have a sufficient molecular weight, the ratio of the total number of moles of bromine atom, iodine atom and chlorine atom represented by X^{a} and X^{b} to the total number of moles of the group represented by -B(OR²¹)₂, possessed by the compound represented by the above-described formulae (A) and (M-1) to (M-3), is set at preferably 0.95 to 1.05, and more preferably 0.98 to 1.02.

An example of the above-described catalyst in the polymerization that utilizes a Suzuki coupling reaction is a catalyst consisting of a transition metal complex including a palladium complex such as palladium [tetrakis(triphenylphosphine)], [tris(dibenzylideneacetone)]dipalladium, palladium acetate or dichlorobis(triphenylphosphine)palladium, and as necessary, a ligand such as triphenylphosphine, tri(t-butylphosphine) or tricyclohexylphosphine. In addition, in polymerization using an Ni(0) catalyst, there is used a catalyst consisting of a transition metal complex including a nickel complex such as nickel [tetrakis(triphenylphosphine)], [1,3-bis(diphenylphosphino)propane]dichloronickel or [bis(1,4-cyclooctadiene)]nickel, and as necessary, a ligand such as triphenylphosphine, tri(t-butylphosphine), tricyclohexylphosphine, diphenylphosphinopropane or bipyridyl. The above-described catalyst may be previously synthesized and may be then used, or may be prepared in a reaction system and may be directly used. The above catalyst may be used singly or in combination of two or more types.

When the above-described catalyst is used, it is sufficient that the catalyst be used in an effective amount as a catalyst. The amount of a transition metal compound to the total number of moles of the compounds represented by the above-described formulae (A) and (M-1) to (M-3) is generally 0.00001 to 3 mole equivalents, preferably 0.00005 to 0.5 mole equivalents, and more preferably 0.0001 to 0.2 mole equivalents.

In the polymerization that utilizes a Suzuki coupling reaction, it is necessary to carry out the reaction in the presence of a base. The above-described base includes inorganic bases such as sodium carbonate, potassium carbonate, cesium carbonate, potassium fluoride, cesium fluoride or tripotassium phosphate; and organic bases such as tetrabutylammonium fluoride; tetrabutylammonium chloride, tetrabutylammonium bromide or tetrabutylammonium hydroxide.

The above-described base is used in an amount of generally 0.5 to 20 mole equivalents, and preferably 1 to 10 mole equivalents, based on the total number of moles of the compounds represented by the above-described formulae (A) and (M-1) to (M-3).

The above-described condensation polymerization may be carried out either in the absence of a solvent, or in the presence of a solvent. In general, this reaction is carried out in the presence of an organic solvent.

The above-described organic solvent includes toluene, xylene, mesitylene, tetrahydrofuran, 1,4-dioxane, dimethoxyethane, N,N-dimethylacetamide, and N,N-dimethylformamide. In general, in order to suppress side reactions, it is desired to carry out a deoxygenation treatment. The above-described organic solvent may be used singly or in combination of two or more types.

The amount of the above-described organic solvent used is such an amount that the total concentration of the compounds represented by the above-described formulae (A) and (M-1) to (M-3) is generally 0.1% to 90% by weight, preferably 1% to 50% by weight, and more preferably 2% to 30% by weight.

The reaction temperature of the above-described condensation polymerization is preferably -100°C to 200°C, more preferably -80°C to 150°C, and further preferably 0°C to 120°C.

The reaction time depends on conditions such as the reaction temperature. It is generally 1 hour or more, and preferably 2 to 500 hours.

When each of X^{a} and X^{b} in the above formulae (A) and (M-1) to (M-3) is a group represented by -MgY¹, the above-described condensation polymerization is carried out under dewatering conditions.

In the above-described condensation polymerization, in order to prevent a polymeric active group from remaining at the terminus of the polymer compound of the present invention, a compound represented by the formula (M-4) provided below may be used as a chain terminating agent, as necessary. Thereby, a polymer compound whose terminal is substituted with an aryl group or a monovalent aromatic heterocyclic group can be obtained.

[Formula 52] **X^{c}-Ar²⁴** (M-4)

(In the formula, Ar²⁴ represents an aryl group or a monovalent aromatic heterocyclic group, and X^{c} is the same as the above-described X^{a} and X^{b} in meaning.)

In the formula (M-4), the aryl group or the monovalent aromatic heterocyclic group represented by Ar²⁴ is the same as that described and exemplified as the aryl group or the monovalent aromatic heterocyclic group as the above-described R².

The post-treatment of the above-described condensation polymerization can be carried out by a known method. For example, there may be applied a method, which comprises adding a reaction solution obtained as a result of the above-described condensation polymerization to lower alcohol such as methanol, and then subjecting the obtained precipitate to filtration and drying.

When the purity of the polymer compound of the present invention is low, it may be purified by an ordinary method such as recrystallization, continuous extraction using a Soxhlet extractor, or column chromatography. When the polymer compound of the present invention is used for a light-emitting device, since the purity of the polymer compound has an influence on the performance of the device such as light-emitting properties, it is preferable to carried out a purification treatment such as reprecipitation purification or fractionation using chromatography, after completion of the condensation polymerization.

### <Monomer>

### - Compound represented by formula (A) -

The compound represented by the above formula (A) includes compounds exemplified as the constitutional units represented by the above formula (1) (compounds represented by the formulae (1-001) to (1-018), (1-101) to (1-115), and (1-201) to (1-205), and the substitution products thereof) in which either one of two bonds is substituted with a group represented by X^{a} and the other bond is substituted with a group represented by X^{b}.

Any type of method may be applied to synthesize the compound represented by the formula (A). Synthesis methods, which will be described as a first production method and a second production method below, are simple and preferable.

### • First production method

A first production method of the compound represented by the formula (A) comprises subjecting the compound represented by the formula (B) and the compound represented by the formula (E), which are dissolved or suspended in an organic solvent, as necessary, to a coupling reaction in the presence of a copper compound, a ligand and a base (scheme 1).

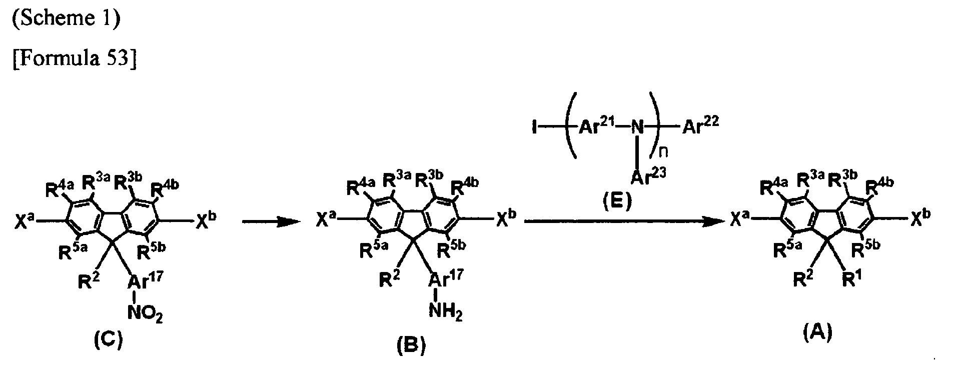

The compound represented by the formula (B) is synthesized by reducing a nitro group of the compound represented by the formula (C) using a reducing agent such as tin chloride or zinc chloride.

The arylene groups, which are represented by the above-described Ar¹⁷ in the formulae (B) and (C) and by the above-described Ar²¹ in the formula (E), are the same as those described and exemplified as the arylene groups represented by the above-described Ar¹ to Ar³.

In the formula (E), the "divalent group in which two or more identical or different arylene groups are bound to one another by a single bond" represented by the above-described Ar²¹ are the same as those described and exemplified as the "divalent group in which two or more identical or different arylene groups are bound to one another by a single bond" represented by the above-described Ar¹ to Ar³.

In the formula (E), the aryl group or the monovalent aromatic heterocyclic group represented by the above-described Ar²² and Ar²³ are the same as those described and exemplified as the aryl group or the monovalent aromatic heterocyclic group represented by the above-described Ar⁴.

The compound represented by the formula (E) is used in an amount of generally 1 to 3 moles with respect to 1 mole of the compound represented by the formula (B). From the viewpoint of the easiness of the purification of the obtained compound represented by the formula (A), the amount used is preferably 1.5 to 2.5 moles.

The copper compound used in the above-described coupling reaction includes copper chloride (I), copper bromide (I), and copper iodide (I). The above-described copper compound is preferably used in an amount of 0.1 to 10 moles with respect to 1 mole of the compound represented by the formula (B) from the viewpoint of reactivity and economy.

The ligand used in the above-described coupling reaction includes 1,10-phenanthroline. The above-described ligand is preferably used in an amount of 0.5 to 5 moles with respect to 1 mole of the above-described copper compound from the viewpoint of reactivity and economy.

The base used in the above-described coupling reaction includes an organic base and an inorganic base. Inorganic bases such as sodium carbonate, potassium carbonate, cesium carbonate, sodium hydroxide or potassium hydroxide; and organic bases such as t-butoxy sodium or t-butoxy potassium are preferable. Potassium hydroxide and t-butoxy potassium are more preferable.

When an organic solvent is used in the above-described coupling reaction, the organic base includes: aromatic hydrocarbon solvents such as toluene, xylene, mesitylene, chlorobenzene or o-dichlorobenzene; and polar solvents such as N,N-dimethylformamide, dimethyl solfoxide or dimethylacetamide. Aromatic hydrocarbon solvents such as toluene, xylene, mesitylene, chlorobenzene or o-dichlorobenzene are preferable. From the viewpoint of reactivity, it is preferable to use an organic solvent.

From the viewpoint of the stability of a material compound and a generated product, the above-described coupling reaction is preferably carried out under light-shielded conditions. The reaction temperature is generally 0°C to 200°C. However, since the above-described coupling reaction is promoted by heating, the reaction temperature is preferably 80°C to 200°C. When the above-described organic solvent is used, the reaction is preferably carried out under reflux conditions. Moreover, it is preferable to carry out the reaction, while removing water and alcohol obtained as a by-product during the reaction, for example, while performing azeotropy using a Dean-Stark apparatus.

After completion of the reaction, water is added to the reaction system, so as to wash bases, inorganic salts obtained as by-products, and the like, and to remove them by liquid separation and the like. Thereafter, ordinary operations such as column chromatography or recrystallization are carried out, so as to obtain the compound represented by the formula (A).

The compound represented by the formula (C) can be synthesized by allowing substituted benzene to react with a fluorenone derivative in the presence of an acid, for example.

### • Second production method

Moreover, as shown in scheme (2) below, the compound A of the present invention may be synthesized by subjecting the compound represented by the formula (D) and the compound represented by the formula (F), which are dissolved or suspended in a solvent, as necessary, to a coupling reaction in the presence of an acid. The compound represented by the formula (D) can be synthesized, for example, by allowing a Grignard reagent and an organic lithium reagent to react with a fluorenone derivative.

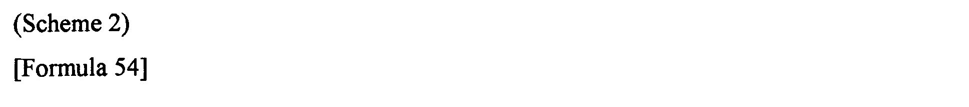

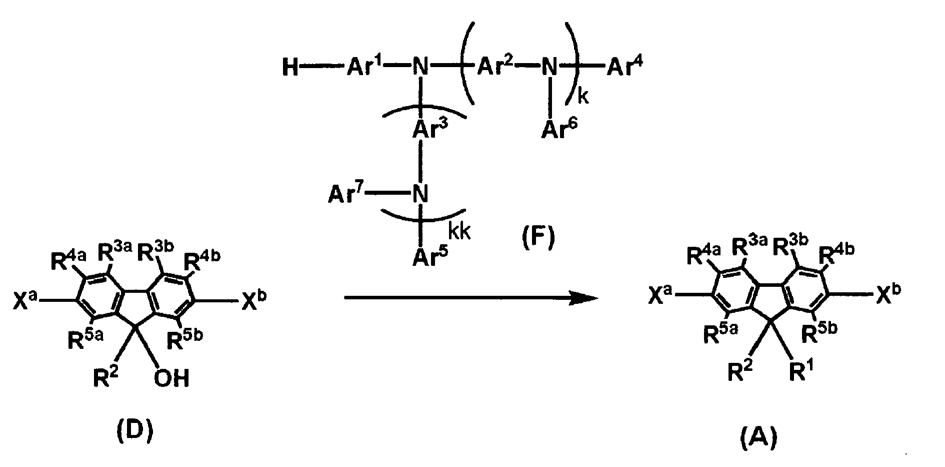

The compound represented by the formula (F) is used in an amount of generally 0.8 to 2 moles with respect to 1 mole of the compound represented by the formula (D). From the viewpoint of the easiness of the purification of the obtained compound represented by the formula (A), the amount used is preferably 0.9 to 1.5 moles.

The above-described acid includes a boron trifluoride diethyl ether complex, trifluoromethanesulfonic acid, methanesulfonic acid, trifluoroacetic acid, sulfuric acid, and polyphosphoric acid. Of these, a boron trifluoride diethyl ether complex is preferable. The amount of the above-described acid used depends on the type thereof. When such boron trifluoride diethyl ether complex is used, from the viewpoint of reactivity and economy, it is used in an amount of generally 1 to 10 moles, and preferably 1 to 2 moles, with respect to 1 mole of the compound represented by the formula (D).

When the above-described solvent is used, the solvent includes organic solvents such as toluene, xylene mesitylene, chlorobenzene, o-dichlorobenzene, dichloromethane, chloroform or carbon tetrachloride. When a boron trifluoride diethyl ether complex is used as the above-described acid, chlorobenzene, o-dichlorobenzene, dichloromethane, chloroform and carbon tetrachloride are preferable, and dichloromethane, chloroform and carbon tetrachloride are more preferable. In addition, from the viewpoint of handleability such as easy control of the reaction temperature, the combined use of a boron trifluoride diethyl ether complex with an organic solvent is preferable.

From the viewpoint of the stability of the compound represented by the formula (D) and the compound represented by the formula (A), the above-described reaction is preferably carried out under light-shielded conditions.

The reaction temperature of the above-described reaction is generally -50°C to 300°C. When the above-described boron trifluoride diethyl ether complex is used in combination with an organic solvent, the reaction temperature is preferably -20°C to 100°C. When the organic solvent is used, the reaction may be carried out under reflux conditions.

After completion of the above-described reaction, water, alcohol or the like is added to the reaction system to terminate the reaction. Thereafter, it is removed by washing, liquid separation and the like, and ordinary operations such as column chromatography, or recrystallization are then carried out, so as to obtain the compound represented by the formula (A).

### <Composition>

The polymer compound of the present invention is combined with at least one selected from among a hole transport material, an electron transport material and a light-emitting material, so that it can be used as a light-emitting material, a hole transport material or an electron transport material even in the form of a composition.

With regard to the ratio between at least one selected from among the hole transport material, the electron transport material and the light-emitting material, and the polymer compound of the present invention in the above-described composition, when the composition of the present invention is used as a light-emitting material, each of the weights of the above-described hole transport material, electron transport material and light-emitting material is generally 1 to 400 parts by weight, and preferably 5 to 150 parts by weight, with respect to 100 parts by weight of the polymer compound of the present invention.

The polystyrene equivalent number average molecular weight of the composition of the present invention is generally approximately 1 × 10³ to 1 × 10⁸, and preferably 1 × 10⁴ to 1 × 10⁶. In addition, the polystyrene equivalent weight average molecular weight of the composition of the present invention is generally approximately 1 × 10³ to 1 × 10⁸. From the viewpoint of film-forming properties and the light-emitting properties of the obtained light-emitting device, it is preferably 1 × 10⁴ to 5 × 10⁶. The term "the average molecular weight of the composition of the composition of the present invention" is used herein to mean a value obtained by analyzing the composition by GPC.

### <Solution>

The solution of the present invention includes a solution comprising the polymer compound of the present invention and a solvent, and the composition of the present invention containing a solvent. This solution is useful for printing processes, and it may generally be called ink, an ink composition, or the like. The solvent of the present invention may comprise a hole transport material (a material used for a hole transport layer as described later), an electron transport material (a material used for an electron transport layer as described later), a light-emitting material, a stabilizer, a thickener (a high-molecular-weight compound used to increase viscosity), a low-molecular-weight compound to decrease viscosity, a surfactant, an antioxidant, high-molecular-weight compounds other than the above-described polymeric compound and the like, as necessary. It is to be noted that ingredients contained in the solution of the present invention may be of one type or may be in combination of two or more types.

With regard to the ratio of the polymer compound of the present invention to the solution of the present invention, generally 0.1 to 99 parts by weight of, preferably 0.5 to 40 parts by weight of, and more preferably 0.5 to 20 parts by weight of the polymer compound is used with respect to 100 parts by weight of the solution as a whole.

The viscosity of the solution of the present invention may be adjusted depending on the type of a printing process. When the solution passes through a discharge device, such as in an ink jet printing process, in order to prevent clogging or the bentness of ink lines during discharge, the solution preferably has a viscosity of 1 to 20 mPa·s at 25°C.

The type of the above-described thickener is not limited, as long as it is soluble in the same solvent as that for the polymer compound of the present invention, and it does not inhibit light emission or charge transport. For example, high-molecular-weight polystyrene, high-molecular-weight polymethyl methacrylate and the like can be used. The compound used as the above-described thickener has a polystyrene equivalent weight average molecular weight of preferably 5 × 10⁵ or more, and more preferably 1 × 10⁶ or more.

The above-described antioxidant is used to improve the preservation stability of the solution of the present invention. The type of the antioxidant is not limited, as long as it is soluble in the same solvent as that for the polymer compound of the present invention and it does not inhibit light emission or charge transport. For example, a phenolic antioxidant, a phosphorus antioxidant and the like can be used.

The solvent used for the solution of the present invention is preferably a solvent, in which a solid used as a solute can be dissolved or uniformly dispersed. The solvent includes: chlorine solvents such as chloroform, methylene chloride, 1,2-dichloroethane, 1,1,2-trichloroethane, chlorobenzene or o-dichlorobenzene; ether solvents such as tetrahydrofuran, dioxane or anisole; aromatic hydrocarbon solvents such as toluene or xylene; aliphatic hydrocarbon solvents such as cyclohexane, methylcyclohexane, n-pentane, n-hexane, n-heptane, n-octane, n-nonane or n-decane; ketone solvents such as acetone, methyl ethyl ketone, cyclohexanone, benzophenone or acetophenone; ester solvents such as ethyl acetate, butyl acetate, ethyl cellosolve acetate, methyl benzoate or phenyl acetate; polyhydric alcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin or 1,2-hexanediol, and the derivatives thereof; alcohol solvents such as methanol, ethanol, propanol, isopropanol or cyclohexanol; sulfoxide solvents such as dimethyl sulfoxide; and amide solvents such as N-methyl-2-pyrrolidone or N-N-dimethylformamide. These solvents may be used singly or in combination of two or more types.

From the viewpoint of a film-forming property and device characteristics, the above-described solvent may be used preferably in combination of two or more types, more preferably in combination of two or three types, and particularly preferably in combination of two types.

When two types of solvents are contained in the solution of the present invention, either one type of the solvents may be in a solid state at 25°C. From the viewpoint of a film-forming property, either one type of solvent has a boiling point of preferably 180°C or higher, and more preferably 200°C or higher. In addition, from the viewpoint of viscosity, it is preferable that 1% by weight or more of the polymer compound of the present invention be dissolved in both of the two types of solvents at 60°C, and it is more preferable that 1% by weight or more of the polymer compound of the present invention be dissolved in either one of the two types of solvents at 25°C.

When two or more types of solvents are contained in the solution of the present invention, from the viewpoint of viscosity and a film-forming property, a solvent having the highest boiling point is used at a weight percentage of preferably 40% to 90%, more preferably 50% to 90%, and further preferably 65% to 85%, based on the total weight of all solvents in the solution.

The solution of the present invention may further comprise water, metals and the salts thereof, silicon, phosphorus, fluorine, chlorine, bromine, and the like, in an amount of 1 to 1000 ppm on a weight basis. The above-described metals include lithium, sodium, calcium, potassium, iron, copper, nickel, aluminum, zinc, chrome, manganese, cobalt, platinum, and iridium.

### <Thin film>

The thin film of the present invention comprises the polymer compound of the present invention. The above-described thin film includes a light-emitting thin film, a conductive thin film, and an organic semiconductor thin film, for example.

The thin film of the present invention can be produced, for example, by a spin coating method, a casting method, a micro gravure coating method, a gravure coating method, a bar coating method, a roll coating method, a wire-bar coating method, a dip coating method, a spray coating method, a screen printing method, a flexographic printing method, an offset printing method, an ink jet printing method, a capillary coating method, a nozzle coating method and the like.

When a thin film is produced using the solution of the present invention, since the glass transition temperature of the polymer compound of the present invention contained in the solution is high, it is possible to perform baking at a temperature of 100°C or higher. Even if baking is performed at a temperature of 130°C, device characteristics are hardly reduced. In addition, depending on the type of the polymer compound, it is also possible to perform baking at a temperature of 160°C or higher.

From the viewpoint of the luminance of the device, light emission voltage, and the like, in the case of the above-described light-emitting thin film, the quantum yield of light emission is preferably 30% or more, more preferably 50% or more, further preferably 60% or more, and particularly preferably 70% or more.

The above-described conductive thin film has a surface resistance of, preferably 1 kΩ/sq. or less, more preferably 100 Ω/sq. or less, and further preferably 10 Ω/sq. or less. A Lewis acid, an ionic compound or the like can be doped to the conductive thin film, so as to increase electric conductivity.

In the case of the above-described organic semiconductor thin film, either an electron mobility or a hole mobility, which is greater, is preferably 10⁻⁵ cm²N/sec or more, more preferably 10⁻³ cm²/V/sec or more, and further preferably 10⁻¹ cm²/V/sec or more. The organic semiconductor thin film is formed on an Si substrate, on which an insulator film such as SiO₂ and a gate electrode are formed, and a source electrode and a drain electrode are formed with Au and the like, thereby obtaining an organic transistor.

### <Light-emitting device>

It is sufficient that the light-emitting device of the present invention be a light-emitting device which comprises electrodes consisting of an anode and a cathode, and an organic layer containing the above-described polymer compound established between the electrodes.

At least one of the above-described anode and cathode is generally transparent or semi-transparent. The above-described organic layer may consist of a single layer or two or more layers. When the organic layer consists of two or more layers, it is sufficient that at least one of them comprise the above-described polymer compound. In addition, the organic layer containing the above-described polymer compound generally functions as a light-emitting layer (a layer having a light-emitting function), a hole transport layer, or an electron blocking layer. The organic layer preferably functions as a light-emitting layer. The light-emitting device of the present invention may also comprise other layers between the anode and the light-emitting layer, and between the cathode and the light-emitting layer, as well as the cathode, the anode and the light-emitting layer. In the light-emitting device of the present invention, each layer may consist of a single layer, or two or more layers. Moreover, materials and compounds that constitute such individual layers may be used singly or in combination of two or more types.

The layer(s) established between the anode and the light-emitting layer includes a hole injection layer, a hole transport layer, and an electron blocking layer. When only a single layer is established between the anode and the light-emitting layer, it is a hole injection layer. On the other hand, when two or more layers are established between the anode and the light-emitting layer, the layer adjacent to the anode is a hole injection layer, and another layer is a hole transport layer. The hole injection layer has the function of improving hole injection efficiency from the cathode. The hole transport layer has the function of improving hole injection from the hole injection layer or a layer closer to the anode. When such hole injection layer or hole transport layer has the function of blocking electron transport, these layers are considered to be electron blocking layers. The fact that the layer has the function of blocking electron transport can be confirmed, for example, by producing a device that supplies only electronic current and then confirming the blocking effects based on a decrease in the current value.

The layer(s) established between the cathode and the light-emitting layer includes an electron injection layer, an electron transport layer, and a hole blocking layer. When only a single layer is established between the cathode and the light-emitting layer, it is an electron injection layer. On the other hand, when two or more layers are established between the cathode and the light-emitting layer, the layer adjacent to the cathode is an electron injection layer, and another layer is an electron transport layer. The electron injection layer has the function of improving electron injection efficiency from the cathode. The electron transport layer has the function of improving electron injection from the electron injection layer or a layer closer to the cathode. When such electron injection layer or electron transport layer has the function of blocking hole transport, these layers are sometimes referred to as a hole blocking layer. The fact that the layer has the function of blocking hole transport can be confirmed, for example, by producing a device that supplies only hole current and then confirming the blocking effects based on a decrease in the current value.

The light-emitting device of the present invention has the following structures (a) to (d), for example:
(a) anode/light-emitting layer/cathode;
(b) anode/hole transport layer/light-emitting layer/cathode;
(c) anode/light-emitting layer/electron transport layer/cathode; and
(d) anode/hole transport layer/light-emitting layer/electron transport layer/cathode,
   (wherein the symbol "/" indicates that two layers adjacent to each other via such "/" are adjacently laminated, and the same applies below).

Among the hole transport layer and the electron transport layer established adjacent to the electrode, the layer having the function of improving charge (hole or electron) injection efficiency from the electrode and having the effect of decreasing driving voltage of the device may be called a charge injection layer (hole injection layer or electron injection layer) at times.

Furthermore, for the improvement of adhesion to the electrode or the improvement of charge injection from the electrode, it may also be possible to establish the above-described charge injection layer or an insulating layer adjacent to the electrode. Further, for the improvement of adhesion at the interface, the prevention of mixing, and the like, a thin buffer layer may be inserted into the interface of the above-described charge transport layer or light-emitting layer. The order or number of layers to be laminated and the thickness of each layer may be adjusted, while taking into consideration the luminous efficiency or the lifetime of the device.

The light-emitting device of the present invention comprising a charge injection layer has the following structures (e) to (p), for example:
(e) anode/charge injection layer/light-emitting layer/cathode;
(f) anode/light-emitting layer/charge injection layer/cathode;
(g) anode/charge injection layer/light-emitting layer/charge injection layer/cathode;
(h) anode/charge injection layer/hole transport layer/light-emitting layer/cathode;
(i) anode/hole transport layer/light-emitting layer/charge injection layer/cathode;
(j) anode/charge injection layer/hole transport layer/light-emitting layer/charge injection layer/cathode;
(k) anode/charge injection layer/light-emitting layer/charge transport layer/cathode;
(l) anode/light-emitting layer/electron transport layer/charge injection layer/cathode;
(m) anode/charge injection layer/light-emitting layer/electron transport layer/charge injection layer/cathode;
(n) anode/charge injection layer/hole transport layer/light-emitting layer/charge transport layer/cathode;
(o) anode/hole transport layer/light-emitting layer/electron transport layer/charge injection layer/cathode; and
(p) anode/charge injection layer/hole transport layer/light-emitting layer/electron transport layer/charge injection layer/cathode.

### • Anode

The above-described anode is generally transparent or semi-transparent, and is generally composed of a thin film of a metal oxide, a metal sulfide or a metal, which has high electric conductivity. Among others, the anode is preferably composed of a material having high transmittance. Materials used as the above-described anode include a film (NESA, etc.) produced using a conductive inorganic compound including indium oxide, zinc oxide, tin oxide, and the complex thereof such as indium/tin/oxide (ITO) or indium/zinc/oxide, gold, platinum, silver, and copper. Of these, ITO, indium/zinc/oxide, and tin oxide are preferable. For the production of the above-described anode, a vacuum evaporation method, a sputtering method, an ion plating method, a plating method, or the like can be applied. Moreover, as the above-described anode, an organic transparent conductive film such as polyaniline and a derivative thereof, or polythiophene and a derivative thereof, may be used.

The thickness of the above-described anode can be appropriately selected, while taking into consideration the tarnsmittance of light and electric conductivity. It is generally 10 nm to 10 µm, preferably 20 nm to 1 µm, and more preferably 50 nm to 500 nm.

### • Hole injection layer

Materials used for the above-described hole injection layer include phenylamines; starburst amines; phthalocyanines; oxides such as vanadium oxide, molybdenum oxide, ruthenium oxide or aluminum oxide; conductive polymers such as amorphous carbons, polyanilines and derivatives thereof, and polythiophenes and derivatives thereof; and the polymer compound of the present invention.

When the materials used for the above-described hole injection layer are a conductive polymer and the polymer compound of the present invention, in order to improve the electric conductivity of the conductive polymer and the polymer compound, anions such as polystyrene sulfonate ions, alkylbenzene sulfonate ions or camphor sulfonate ions may be doped, as necessary.

### • Hole transport layer

Materials used for the above-described hole transport layer include polyvinylcarbazoles and derivatives thereof, polysilanes and derivatives thereof, polysiloxane derivatives having an aromatic amine on the side chain or main chain thereof, pyrazoline derivatives, arylamine derivatives, stilbene derivatives, triphenyldiamine derivatives, polyanilines and derivatives thereof, polythiophenes and derivatives thereof, polyarylamines and derivatives thereof, polypyrroles and derivatives thereof, poly(p-phenylenevinylene)s and derivatives thereof, poly(2,5-thienylenevinylene)s and derivatives thereof, and the polymer compound of the present invention. Of these, polymeric hole transport materials such as polyvinylcarbazoles and derivatives thereof, polysilanes and derivatives thereof, polysiloxane derivatives having an aromatic amine compound group on the side chain or main chain thereof, polyanilines and derivatives thereof, polythiophenes and derivatives thereof, polyarylamines and derivatives thereof, poly(p-phenylenevinylene)s and derivatives thereof, poly(2,5-thienylenevinylene)s and derivatives thereof, and the polymer compound of the present invention, are preferable. Polyvinylcarbazoles and derivatives thereof, polysilanes and derivatives thereof, polysiloxane derivatives having an aromatic amine on the side chain or main chain thereof, polyarylamines and derivatives thereof, and the polymer compound of the present invention are more preferable. When the materials used for the above-described hole transport layer are low-molecular-weight compounds, it is preferable that they be dispersed in a polymeric binder and be then used.

As a method for forming a film as a hole transport layer, when the material used for the above-described hole transport layer is a low-molecular-weight compound, such film is formed from a solution in which the low-molecular-weight compound and a polymeric binder are mixed. On the other hand, when the material used for the above-described hole transport layer is a high-molecular-weight compound, such film is formed from a solution.

The type of a solvent used in film formation from the solution is not limited, as long as it dissolves materials used for the hole transport layer. The solvent includes chlorine solvents such as chloroform, methylene chloride or dichloroethane; ether solvents such as tetrahydrofuran; aromatic hydrocarbon solvents such as toluene or xylene, ketone solvents such as acetone or methyl ethyl ketone; and ester solvents such as ethyl acetate, butyl acetate or ethyl cellosolve acetate.

For film formation from the solution, methods involving coating from the solution, such as a spin coating method, a casting method, a micro gravure coating method, a gravure coating method, a bar coating method, a roll coating method, a wire-bar coating method, a dip coating method, a spray coating method, a screen printing method, a flexographic printing method, an offset printing method or an ink jet printing method can be applied.

As the above-described polymeric binder, a polymeric binder, which does not strongly inhibit charge transport, is preferable. Moreover, a polymeric binder, which absorbs only a small amount of visible light, is preferably used. The polymeric binder includes polycarbonates, polyacrylate, polymethyl acrylate, polymethyl methacrylate, polystyrene, polyvinyl chloride, and polysiloxanes.

The thickness of the hole transport layer may be selected, while taking into consideration driving voltage and luminous efficiency. The hole transport layer should have at least a thickness necessary for preventing the generation of pinholes. If the thickness is too high, driving voltage of the device becomes high, and thus it is not favorable. Accordingly, the film thickness of the hole transport layer is generally 1 nm to 1 µm, preferably 2 nm to 500 nm, and more preferably 5 nm to 200 nm.

### • Light-emitting layer

The light-emitting layer is generally formed from an organic compound (a low-molecular-weight compound or a high-molecular-weight compound) emitting fluorescence or phosphorescence, and as necessary, a dopant that supports this compound. For the light emitting layer in the light-emitting device of the present invention, there can be used light-emitting materials including polymeric materials such as: the polymer compound of the present invention; pigment materials such as a cyclopendamine derivative, a tetraphenylbutadiene derivative compound, a triphenylamine derivative, an oxadiazole derivative, a pyrazoloquinoline derivative, a distyrylbenzene derivative, a distyrylarylene derivative, a pyrrole derivative, a thiophene ring compound, a pyridine ring compound, an anthracene derivative, a perylene derivative, an oligothiophene derivative, a trifumanylamine derivative, an oxadiazole dimer, or a pyrazoline dimer; metal complex materials including metal complexes having light emission from a triplet excited state, such as an iridium complex or a platinum complex, and metal complexes having, as a central metal, Al, Zn, Be, etc. or rare earth metals such as Tb, Eu or Dy, and having, as a ligand, an oxadiazole, thiadiazole, phenylpyridine, phenylbenzoimidazole or quinoline structure or the like, for example, an alumiquinolinol complex, a benzoquinolinol beryllium complex, a benzooxazolyl zinc complex, a benzothiazole zinc complex, an azomethyl zinc complex, a porphyrin zinc complex, a europium complex, and the like; a polyparaphenylenevinylene derivative, a polythiophene derivative, a polyparaphenylene derivative, a polysilane derivative, a polyacetylene derivative, a polyfluorene derivative, a polyvinylcarbazole derivative, products formed by polymerizing the above-described pigment materials or metal complex light-emitting materials, and the like.

Among the above-described light-emitting materials, materials emitting blue light include distyrylarylene derivatives and the polymers thereof, oxadiazole derivatives and the polymers thereof, polyvinylcarbazole derivatives, polyparaphenylene derivatives, and polyfluorene derivatives. Polyvinylcarbazole derivatives, polyparaphenylene derivatives, polyfluorene derivatives and the like are preferable.
Among the above-described light-emitting materials, materials emitting green light include quinacridon derivatives, coumarin derivatives, anthracene derivatives, their polymers, polyparaphenylenevinylene derivatives, and polyfluorene derivatives. Polyparaphenylenevinylene derivatives, polyfluorene derivatives and the like are preferable.
Among the above-described light-emitting materials, materials emitting red light include coumarin derivatives and the polymers thereof, thiophene compounds and the polymers thereof, polyparaphenylenevinylene derivatives, polythiophene derivatives, and polyfluorene derivatives. Polyparaphenylenevinylene derivatives, polythiophene derivatives, polyfluorene derivatives and the like are preferable.

In order to improve luminous efficiency or to change the wavelength of light emitted, a dopant may be added to the above-described light-emitting layer. The above-described dopant includes anthracene derivatives, perylene derivatives, coumarin derivatives, rubrene derivatives, quinacridon derivatives, squarium derivatives, porphyrin derivatives, styryl pigments, tetracene derivatives, pyrazolone derivatives, decacyclene, and phenoxazone.

The thickness of the above-described light-emitting layer may be selected, while taking into consideration driving voltage and luminous efficiency. It is generally approximately 20 to 2000 A.

In order to form a film used as the above-described light-emitting layer, a method of applying a solution containing a light-emitting material onto a substrate or to the upper portion thereof, a vacuum evaporation method, a transfer method and the like can be applied. A solvent used in film formation from such solution is the same as those described and exemplified in the section regarding film formation from a hole transport layer solution. In order to apply a solution containing a light-emitting material onto a substrate or to the upper portion thereof, printing methods such as a spin coating method, a dip coating method, an ink jet printing method, a flexographic printing method, a gravure printing method or a slit coating method can be applied. In the case of using a subliming low-molecular-weight compound, a vacuum evaporation method can be applied. A method of forming a light-emitting layer at a desired position by transfer with laser or thermal transfer can also be applied.

### • Electron transport layer

Materials used for the above-described electron transport layer include oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, metal complexes of 8-hydroxyquinoline and derivatives thereof, polyquinoline and derivatives thereof, polyquinoxaline and derivatives thereof, and polyfluorene and derivatives thereof. Of these, oxadiazole derivatives, benzoquinone and derivatives thereof, anthraquinone and derivatives thereof, metal complexes of 8-hydroxyquinoline and derivatives thereof, polyquinoline and derivatives thereof, polyquinoxaline and derivatives thereof, and polyfluorene and derivatives thereof are preferable. Of these, 2-(4-biphenylyl)-5-(4-t-butylphenyl)-1,3,4-oxadiazole, benzoquinone, anthraquinone, tris(8-quinolinol)aluminum, and polyquinoline are more preferable.

In order to form a film used as the above-described electron transport layer, when the material used for the above-described electron transport layer is a low-molecular-weight compound, a vacuum evaporation method in which the film is formed from powders and a method of forming a film from a solution or a fused state are applied. When the material used for the above-described electron transport layer is a high-molecular-weight compound, a method of forming a film from a solution or a fused state is applied. A polymeric binder may be used for such film formation from a solution or a fused state. Film formation from a solution may be carried out in the same manner as the above-described method of forming a film used as a hole transport layer from a solution.

The thickness of the electron transport layer may be adjusted, while taking into consideration driving voltage and luminous efficiency. The thickness of the electron transport layer should be at least a thickness necessary for preventing the generation of pinholes. If the thickness is too high, driving voltage of the device becomes high, and thus it is not favorable. Accordingly, the film thickness of the electron transport layer is generally 1 nm to 1 µm, preferably 2 nm to 500 nm, and more preferably 5 nm to 200 nm.

### • Electron injection layer

Depending on the type of the light-emitting layer, the above-described electron injection layer is an electron injection layer consisting of a monolayer structure of a Ca layer, or an electron injection layer that has a laminated structure consisting of a layer formed with one or two or more types selected from among metals of the IA and IIA groups of the periodic table, from which Ca is excluded and which have a work function of 1.5 to 3.0 eV, the oxides thereof, the halides thereof and the carbonates thereof, and a Ca layer. The metal of the IA group of the periodic table having a work function of 1.5 to 3.0 eV, the oxide thereof, the halide thereof and the carbonate thereof include lithium, lithium fluoride, sodium oxide, lithium oxide, and lithium carbonate. The metal of the IIA group of the periodic table from which Ca is excluded and which has a work function of 1.5 to 3.0 eV, the oxide thereof, the halide thereof and the carbonate thereof include strontium, magnesium oxide, magnesium fluoride, strontium fluoride, barium fluoride, strontium oxide, and magnesium carbonate.

The electron injection layer may be formed by an evaporation method, a sputtering method, a printing method, or the like. The thickness of the electron injection layer is preferably approximately 1 nm to 1 µm.

### • Cathode material

As the above-described cathode material, a material that has a small work function and facilitates electron injection into the light-emitting layer is preferable. The above-described cathode material includes: metals such as lithium, sodium, potassium, rubidium, cesium, beryllium, magnesium, calcium, strontium, barium, aluminum, scandium, vanadium, zinc, yttrium, indium, cerium, samarium, europium, terbium or ytterbium; alloys consisting of two or more of the above-mentioned metals; alloys consisting of one or more of them and one or more from gold, silver, platinum, copper, manganese, titanium, cobalt, nickel, tungsten and tin; and graphite or an intercalated graphite. The above-described alloys include a magnesium-silver alloy, a magnesium-indium alloy, a magnesium-aluminum alloy, an indium-silver alloy, a lithium-aluminum alloy, a lithium-magnesium alloy, a lithium-indium alloy, and a calcium-aluminum alloy.

When the above-described cathode has a laminated structure consisting of two or more layers, a laminated structure consisting of the above-described metal, metal oxide, metal fluoride or the alloy thereof, and metal such as aluminum, silver or chromium, is preferable.

The thickness of the above-described cathode may be selected, while tanking into consideration electric conductivity or durability. It is generally 10 nm to 10 µm, preferably 20 nm to 1 µm, and more preferably 50 nm to 500 nm.

For the production of the above-described cathode, a vacuum evaporation method, a sputtering method, a lamination method involving the thermal compression bond of a metal thin film, and the like are applied. After completion of the production of the cathode, a protecting layer that protects the light-emitting device may be mounted. In order to use the light-emitting device stably for a long period of time, a protecting layer and/or a protecting cover is preferably mounted to protect the above-described light-emitting device from the outside.

As the above-described protecting layer, a high-molecular-weight compound, a metal oxide, a metal fluoride, a metal boride and the like can be used. As the above-described protecting cover, a metal plate, a glass plate, a plastic plate whose surface has been subjected to a low water permeability treatment, and the like can be used. In order to carry out such protection, there can be applied a method of adhering the protecting cover to a device substrate using a thermosetting resin or a photo-curing resin so as to hermitically seal the substrate. If a space is maintained using a spacer, the device is easily prevented from being damaged. If the space is filled with inactive gas such as nitrogen or argon, the oxidation of the cathode can be prevented. Moreover, by placing a drying agent such as barium oxide in the space, it becomes easy to reduce damage to the device caused by water adsorbed during the production process or a trace amount of water passing through the cured resin and entering the device. It is preferable to adopt any one or more methods from the above-described methods.

The light-emitting device of the present invention can be used as a planar light source, a segment display device, a dot matrix display device, a backlight of a liquid crystal display device, or the like. In order to achieve planar light emission using the light-emitting device of the present invention, a planar anode and a planar cathode may be disposed such that they overlap each other. In order to achieve patterned light emission, a method of establishing a mask having a patterned window on the surface of the above-described planar light-emitting device, a method of forming an extremely thick organic layer on a non-light-emitting portion so as to convert it to be substantially non-illuminant, and a method of forming either one of the anode and the cathode or the two electrodes in a patterned state are applied. A pattern is formed by any one of these methods, and several electrodes are then disposed such that they can be independently switched ON/OFF, so that a segment-type display device capable of displaying numbers, characters, simple symbols and the like can be obtained. Further, in order to produce a dot matrix device, each of the anode and the cathode may be disposed to form stripes, and they may be then orthogonalized. A partial color display and a multicolor display can be realized by a method of applying several types of polymer compounds having different light colors or by a method using a color filter or a fluorescence conversion filter. The dot matrix device may be passively driven, or may be actively driven in combination with TFT and the like. These display devices can be used as display devices for a computer, a television, a mobile terminal, a mobile phone, a car navigation system, a view finder of a video camera, etc. Moreover, the above-described planar light-emitting device is self-luminous low-profile device, and it can be preferably used as a planar light source for the backlight of a liquid crystal display device, a planar light source for lighting, and the like. Furthermore, if a flexible substrate is used, the light-emitting device can also be used as a curved light source or a display device.

### EXAMPLES

The present invention will be described more in detail in the following examples. However, these examples are not intended to limit the scope of the present invention.

### (Number average molecular weight and weight average molecular weight)

In the following examples, polystyrene equivalent number average molecular weight (Mn) and polystyrene equivalent weight average molecular weight (Mw) were obtained by GPC (manufactured by Shimadzu Corporation; product name: LC-10Avp). A polymer compound to be measured was dissolved in tetrahydrofuran to a concentration of approximately 0.5% by weight, and 30 µL of the obtained solution was then poured into GPC.
Tetrahydrofuran was used as a mobile phase in GPC, and it was supplied at a flow rate of 0.6 mL/min. As a column, two TSKgel SuperHM-H (manufactured by Tosoh Corporation) were connected with one TSKgel SuperH2000 (manufactured by Tosoh Corporation) in series. As a detector, a differential refractive index detector (manufactured by Shimadzu Corporation; product name: RID-10A) was used.

### (High performance liquid chromatograph (HPLC))

In the following examples, as a HPLC area percent used as an indicator for the purity of a compound, the value at 254 nm was obtained by high performance liquid chromatograph (HPLC; manufactured by Shimadzu Corporation; product name: LC-20A), unless otherwise specified. A compound to be measured was dissolved in tetrahydrofuran or chloroform to a concentration of 0.01% to 0.2% by weight, and 1 to 10 µl of the solution was poured to HPLC, depending on the concentration thereof. Acetonitrile and tetrahydrofuran were used as a mobile phase in the HPLC, and it was supplied at a flow rate of 1 mL/min as a gradient analysis of acetonitrile/tetrahydrofuran = 100/0 to 0/100 (volume ratio). As a column, Kaseisorb LC ODS 2000 (manufactured by Tokyo Chemical Industry Co., Ltd.) was used. As a detector, a photodiode array detector (manufactured by Shimadzu Corporation; product name: SPD-M20A) was used.

### (Glass transition temperature)

In the following examples, a glass transition temperature (Tg) was obtained using a differential scanning calorimeter (DSC; manufactured by TA Instruments; product name: DSC2920). As measurement conditions, a sample was retained at 200°C for 5 minutes, it was then quenched to -50°C, and it was then retained for 30 minutes. Thereafter, the temperature was increased to 30°C, and the measurement was then carried out at a temperature-increasing rate of 5°C/min up to 300°C.

### (Evaluation of fluorescence characteristics)

In the following examples, fluorescence characteristics (the fluorescence peak wavelength of the thin film of a polymer compound) were evaluated by the following method. A polymer compound was dissolved in toluene (manufactured by Kanto Kagaku Kabushiki Kaisha; grade for electronic industry). This time, a solid was prepared to have a concentration of 0.8% by weight, and the obtained solution was spin-coated on a quartz plate at a rotation rate of 1500 rpm, so as to produce a thin film of polymer compound. This thin film was excited at a wavelength of 350 nm, and a fluorescence spectrum was then measured using a fluorospectrophotometer (manufactured by JOBINYVON-SPEX; product name: Fluorolog).

### (Photoluminescence quantum yield (PLQY) measurement)

In the following examples, a photoluminescence quantum yield was measured using the C9920-02 Absolute PL Quantum Yield Measurement System manufactured by Hamamatsu Photonics K.K., at an excitation center wavelength of 325 nm in an excitation wavelength range of 315-335 nm and in a measurement wavelength range of 390-800 nm.

### <Example 1> (Synthesis of compound M1)

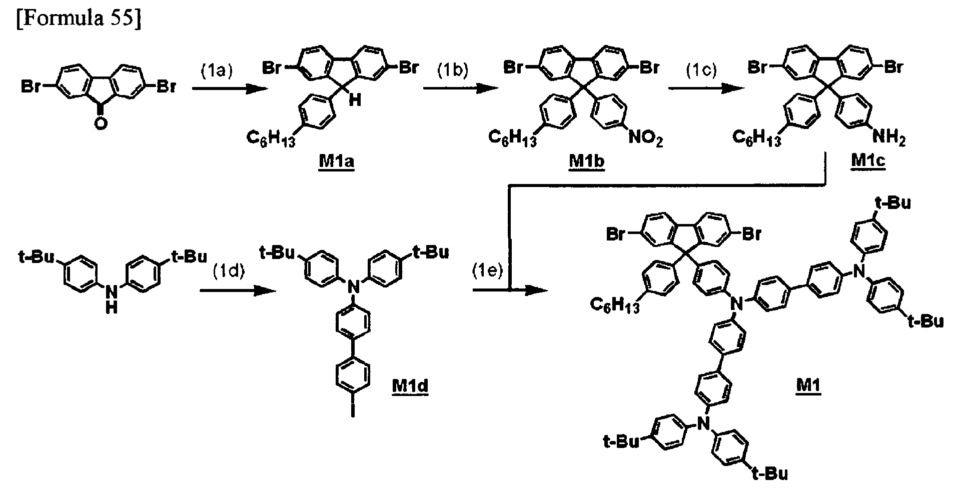

### • Step (1a)

Under a nitrogen gas atmosphere, 2,7-dibromofluorenone (75 g, 0.22 mol), hexylbenzene (334 ml, 1.78 mol), and trifluoromethanesulfonic acid (42 ml) were stirred at a room temperature. Thereafter, sodium mercaptosulfonate (8.1 g, 44 mmol) was then added to the solution. The obtained mixture was stirred at 45°C for 9 hours. Thereafter, the obtained solution was cooled to a room temperature, and it was then poured into 1 L of hexane. Residual hexylbenzene was removed by distillation under a reduced pressure (105.5°C, 20 hPa), and the resultant was then diluted with hexane. Thereafter, it was poured into methanol, and the precipitated 2,7-dibromofluorenone was removed by filtration. The obtained filtrate was concentrated, and it was then diluted with toluene. Isopropyl alcohol was added to the solution, so as to precipitate a solid. The obtained solid was recrystallized from toluene/isopropyl alcohol, so as to obtain a compound M1a (53 g; yield: 49%; HPLC area percent: 99.5%) in the form of a white crystal.
¹H-NMR (300 MHz, CDCl₃) δ 0.88 (t, 3H), 1.20-1.45 (m, 6H), 1.54-1.62 (m, 2H), 2.57 (t, 2H), 4.96 (s, 1H), 6.94 (d, 2H), 7.10 (d, 2H), 7.42 (s, 2H), 7.48 (dd, 2H), 7.60 (d, 2H)

### • Step (1b)

Under a nitrogen gas atmosphere, the compound M1a (10 g, 20.6 mmol), 4-fluoronitrobenzene (3.5 g, 24.8 mmol), and potassium carbonate (4.3 g, 31.0 mmol) were stirred in anhydrous N,N-dimethylformamide (35 ml) under heating to reflux for 6 hours. The reaction solution was cooled to a room temperature, and while stirring, 300 ml of water was slowly added to the obtained solution. The obtained solution was stirred overnight at a room temperature. Thereafter, the precipitated solid was obtained by filtration under a reduced pressure, and the solid on a filter was then washed with a large amount of water. The obtained solid was subjected to vacuum drying, so as to obtain a compound M1b (13.6 g, quantitative).
¹H-NMR (300 MHz, THF-d8) δ 0.91 (t, 3H), 1.24-1.42 (m, 6H), 1.55-1.61 (m, 2H), 2.59 (t, 2H), 7.07-7.16 (m, 4H), 7.43 (d, 2H), 7.59 (dd, 2H), 7.64 (s, 2H), 7.82 (d, 2H), 8.11 (d, 2H)

### • Step (1c)

Under a nitrogen gas atmosphere, a mixture of the compound M1b (12.9 g, 21 mmol), ethanol (153 ml) and tin(II) chloride dihydrate (18.6 g, 8 mmol) was stirred under heating to reflux for 6 hours. Thereafter, the reaction solution was cooled to a room temperature, and it was then concentrated under a reduced pressure until it became approximately 60 g. The obtained solution was added to ice water (150 g), while stirring. After the ice had been melted, a 40-weight-% sodium hydroxide aqueous solution was added to the obtained aqueous solution, until the pH of the aqueous solution exceeded pH 10. The obtained solution was extracted twice with 200 ml of toluene. The obtained organic layer was dried over anhydrous sodium sulfate, and it was then subjected to vacuum concentration. The resultant was recrystallized (toluene-hexane), so as to obtain a compound M1c (10g; yield: 97%). ¹H-NMR (300 MHz, CDCl₃) δ 0.87 (t, 3H), 1.20-1.40 (m, 6H), 1.52-1.57 (m, 2H), 2.54 (t, 2H), 6.54 (d, 2H), 6.91 (d, 2H), 7.02-7.06 (m, 4H), 7.42-7.48 (m, 4H), 7.54 (d, 2H)
LC-MS M=573

### • Step (1d)

Under an argon gas atmosphere, bis(t-butylphenyl)amine (98.5 g, 350 mmol), 4-4'-diiodobiphenyl (144.9 g, 357 mmol), potassium hydroxide (157.2 g, 2.8 mol), 1,10-phenanthroline (15.8 g, 87.5 mmol), and copper(I) chloride (8.66 g, 87.5 mmol) were suspended in anhydrous toluene (525 mL), and it was stirred for 6 hours while heating in an oil bath at 130°C. The obtained reaction mixture was diluted with toluene (1050 mL), and it was then cooled to a room temperature. The resultant was filtrated with Celite to remove insoluble matter. The solvent was removed by vacuum concentration, and while the precipitated solid was suspended in chloroform, it was intensively stirred to extract a product of interest. Insoluble matter was removed by filtration. The filtrate was subjected to vacuum concentration, and it was then purified by silica gel column chromatography (hexane/chloroform = 98/2 to 70/30 (volume ratio)). The resultant was recrystallized (chloroform-methanol), and it was then subjected to vacuum drying, so as to obtain a compound M1d (79.5 g; yield: 41%) in the form of a white crystal.
¹H-NMR (300 MHz, THF-d8) δ 1.33 (s, 18H), 7.00-7.07 (m, 6H), 7.29-7.48 (m, 8H), 7.73 (d, 2H)

### • Step (1e)

Under an argon gas atmosphere, the compound M1c (20.1 g, 35 mmol), the compound M1d (39.2 g, 70 mmol), potassium hydroxide (15.7 g, 280 mmol), 1,10-phenanthroline (3.15 g, 17.5 mmol), and copper(I) chloride (1.73 g, 17.5 mmol) were suspended in anhydrous toluene (70 mL) under light-shielded conditions, and it was stirred for 10 hours, while heating in an oil bath at 130°C. The obtained reaction mixture was diluted with toluene (490 mL), and it was then cooled to a room temperature. The resultant was filtrated with Celite to remove insoluble matter. The solvent was removed by vacuum concentration, and the resultant was then suspended in hexane that had been heated to 60°C. Insoluble matter was removed by filtration. The filtrate was subjected to vacuum concentration, and it was then purified by silica gel column chromatography (hexane/chloroform = 98/2 to 65/35 (volume ratio)). The resultant was recrystallized (hexane), and it was then subjected to vacuum drying, so as to obtain a compound M1 (19.3 g; yield: 55%; HPLC area percent: 99% or more) in the form of a white solid.
¹H-NMR (300 MHz, THF-d8) δ 0.90 (t, 3H), 1.21-1.47 (m, 42H), 1.53-1.61 (m, 2H), 2.57 (t, 2H), 7.00-7.17 (m, 24H), 7.31 (d, 8H), 7.43-7.56 (m, 10H), 7.63 (d, 2H), 7.77 (d, 2H) LC-MS M=1435

### <Example 2> (Synthesis of compound M2)

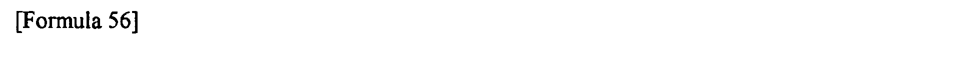

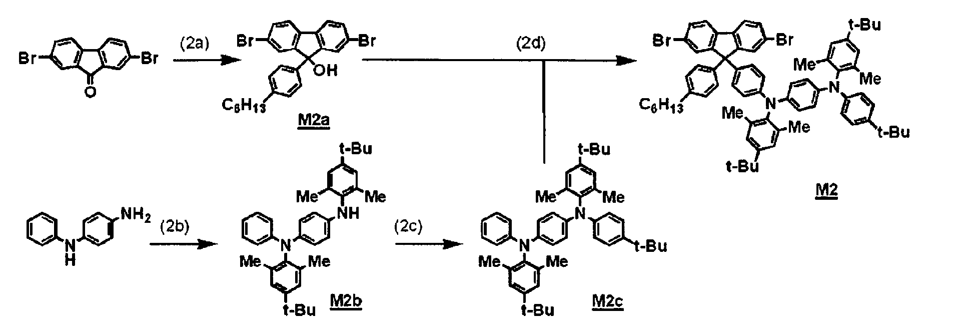

### • Step (2a)

Under an argon gas atmosphere, while a piece of magnesium (2.9 g, 120 mmol) was stirred in a small amount of anhydrous THF (approximately 3 mL), 1,2-dibromoethane (0.6 g, 3.2 mmol) was added thereto. After the activation of magnesium had been confirmed by the generation of heat, a solution obtained by diluting 4-bromohexylbenzene (28.9 g, 120 mmol) with anhydrous THF (40 mL) was added dropwise to the solution over approximately 30 minutes, such that a moderate reflux could be maintained in the reaction system. Thereafter, the obtained mixture was heated to reflux in an oil bath for 1 hour, and it was then diluted with 30 mL of anhydrous THF, so as to prepare a Grignard solution.

At the same time, under an argon gas atmosphere, 2,7-dibromofluorenone (27.0 g, 80 mmol) was suspended in 320 mL of anhydrous diethyl ether. While the obtained solution was intensively stirred at a room temperature, the above-described Grignard solution was added dropwise thereto over approximately 30 minutes. Thereafter, the obtained mixture was heated to reflux in an oil bath for 1 hour. The reaction solution was cooled in an ice water bath, and a saturated ammonium chloride aqueous solution (80 mL) was then added dropwise thereto. The obtained mixture was stirred for 1 hour. Thereafter, liquid separation was performed, and extraction from a water layer was then carried out using diethyl ether. After organic layers had been gathered, it was successively washed with water (3 times) and then with a 15-weight-% saline (once), and it was then dried over anhydrous magnesium sulfate. The resultant was subjected to vacuum concentration, and it was then purified by silica gel column chromatography (hexane/chloroform = 98/2 to 50/50 (volume ratio)), so as to obtain a compound M2a (28 g; yield: 70%) in the form of a light yellow oily product.
¹H-NMR (300 MHz, Acetone-d6) δ 0.90 (t, 3H), 1.25-1.40 (m, 6H), 1.55-1.63 (m, 2H), 2.60 (t, 2H), 5.51 (s, 1H), 7.17 (d, 2H), 7.30 (d, 2H), 7.46 (s, 2H), 7.59 (d, 2H), 7.81 (d, 2H)
LC-MS M=498

### • Step (2b)

Under an argon gas atmosphere, 4-aminodiphenylamine (51.8 g), 2,6-dimethyl-4-t-butylbromobenzene (139 g), t-butoxy sodium (59.4 g), tris(dibenzylideneacetone)dipalladium(0) (5.15 g), and tri(t-butyl)phosphine (2.28 g) were reacted at 100°C for 5 hours in anhydrous toluene (1500 mL). Subsequently, the reaction solution was cooled to a room temperature, and insoluble matter was then removed by filtration with Celite. The filtrate was subjected to vacuum concentration, and it was then supplied to a silica gel short column (toluene), followed by vacuum concentration, so as to obtain 137.3 g of a crude product. The above-described crude product (137.3 g), 2,6-dimethyl-4-t-butylbromobenzene (51.9 g), t-butoxy sodium (22.8 g), and tris(dibenzylideneacetone)dipalladium(0) (3.94 g), and tri(t-butyl)phosphine (1.74 g) were reacted at 100°C for 3 hours in anhydrous toluene (1500 mL). Subsequently, the reaction solution was cooled to a room temperature, and insoluble matter was then removed by filtration with Celite. The filtrate was subjected to vacuum concentration, and it was then supplied to a silica gel short column (toluene), followed by vacuum concentration. The resultant was purified by silica gel column chromatography (hexane/ethyl acetate = 50/1 (volume ratio)), so as to obtain a compound M2b (55.7 g).

### • Step (2c)

Under an argon gas atmosphere, the compound M2b (54.7 g), 4-t-butylbromobenzene (22.3 g), t-butoxy sodium (11.5 g), tris(dibenzylideneacetone)dipalladium(0) (1.83 g), and tri(t-butyl)phosphine (0.81 g) were reacted at 60°C for 1 hour in anhydrous toluene (530 mL). Subsequently, the reaction solution was cooled to a room temperature, and insoluble matter was then removed by filtration with Celite. The filtrate was subjected to vacuum concentration, and it was then supplied to a silica gel short column (toluene), followed by vacuum concentration. Thereafter, methanol was added to the resultant, so as to crystallize a solid. The obtained crystal was recrystallized twice (toluene-methanol and hexane), and it was then subjected to vacuum drying, so as to obtain a compound M2c (43.3 g; yield: 24%) in the form of a white crystal.
¹H-NMR (300 MHz, THF-d8) δ 1.28 (s, 9H), 1.34 (s, 18H), 2.03 (s, 12H), 6.73-6.86 (m, 9H), 7.05-7.21 (m, 8H)
LC-MS M=636

### • Step (2d)

Under an argon gas atmosphere, the compound M2a (10.0 g, 20.0 mmol) and the compound M2c (12.7 g, 20.0 mmol) were dissolved in anhydrous dichloromethane (600 mL) under light-shielded conditions at a room temperature. While the obtained solution was stirred, a solution of boron trifluoride diethyl ether complex (2.9 mL, 23.4 mmol) diluted with anhydrous dichloromethane (136 mL) was added dropwise thereto over 45 minutes. The obtained mixture was further stirred at a room temperature for 5 hours. Thereafter, ethanol (50 mL) and water (50 mL) were added to the reaction mixture, and the obtained mixture was then stirred for 1 hour. Thereafter, a water layer was removed by liquid separation, and the remaining organic layer was successively washed with a 5-weight-% sodium bicarbonate aqueous solution (twice), with water, and then with a 15-weight-% saline (twice). It was dried over anhydrous magnesium sulfate, and was then subjected to vacuum concentration. Methanol was added to the resultant to precipitate a solid. The obtained solid was subjected to vacuum drying, and it was then supplied to a silica gel short column (hexane/toluene = 1/1 (volume ratio)), followed by vacuum concentration. The resultant was recrystallized 4 times (hexane-ethanol, acetone-methanol (twice), and toluene-acetone-methanol), and it was then subjected to vacuum drying, so as to obtain a compound M2 (15.2 g; yield: 70%; HPLC area percent: 99% or more) in the form of a white solid.
¹H-NMR (300 MHz, THF-d8) δ 0.90 (t, 3H), 1.28 (s, 9H), 1.30-1.33 (m, 24H), 1.50-1.62 (m, 2H), 1.01 (s, 6H), 2.03 (s, 6H), 2.56 (t, 2H), 6.70-7.19 (m, 20H), 7.48-7.57 (m, 4H), 7.73 (d, 2H)
LC-MS M=1116

### <Comparative Example 1> (Synthesis of compound CM1)

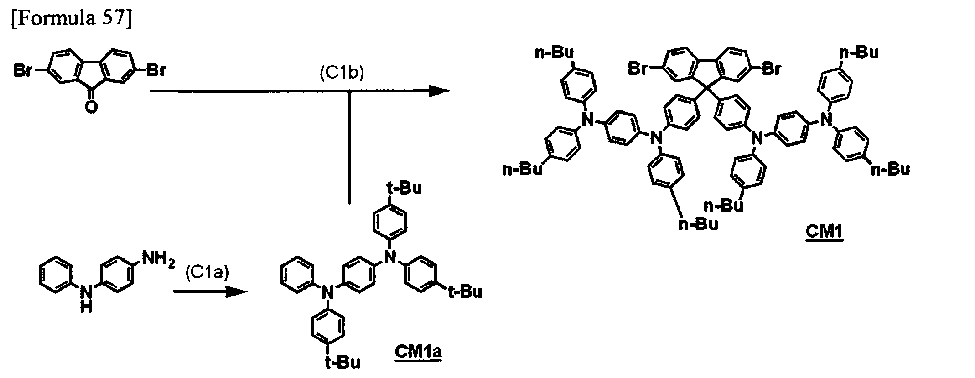

### • Step (C1a)

Under an argon gas atmosphere, 4-aminodiphenylamine (5.53 g, 30 mmol), 4-n-butylbromobenzene (25.6 g, 120 mmol), t-butoxy sodium (8.65 g, 90 mmol), tris(dibenzylideneacetone)dipalladium(0) (0.82 g, 0.9 mmol), and tri(t-butyl)phosphine (0.36 g, 1.8 mmol) were reacted at 100°C for 3 hours in anhydrous toluene (120 mL). Thereafter, the reaction solution was cooled to a room temperature, and toluene (200 mL) was then added thereto. The obtained mixture was successively washed with a saturated saline (100 mL × 3) and then with water (100 mL), and it was then dried over anhydrous sodium sulfate, followed by vacuum concentration. The resultant was purified twice by silica gel column chromatography (hexane/toluene = 75/25 (volume ratio), and hexane/toluene = 100/0 to 75/25 (volume ratio)), so as to obtain a compound CM1a (10.2 g).
¹H-NMR; δ 0.97 (9H, t), 1.37 (6H, m), 1.58 (6H, m), 2.55 (6H, t), 6.85-7.07 (18H, m), 7.17 (2H, t)

### • Step (C1b)

Under an argon gas atmosphere, 2,7-dibromofluorenone (0.379 g, 1.1 mmol), the compound CM1a (1.37 g, 2.3 mmol), methanesulfonic acid (in an amount as a catalyst), and toluene (6 mL) were mixed, and the obtained mixture was then heated to reflux for 15 hours. Thereafter, the reaction solution was cooled to a room temperature, and it was then diluted with toluene (30 mL). The resultant was washed with a sodium bicarbonate aqueous solution (100 mL × 3), and with water (100 mL), and it was then dried over anhydrous sodium sulfate, followed by vacuum concentration. The resultant was purified by silica gel column chromatography (hexane/toluene = 1/6 (volume ratio)), so as to obtain a compound CM1 (0.82 g; HPLC area percent: 99% or more).
LC-MS (APCI method); m/z 1482.5 ([M+H]⁺)

### <Comparative Example 2> (Synthesis of compound CM2)

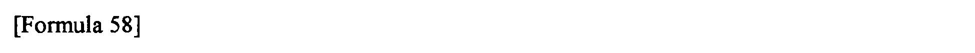

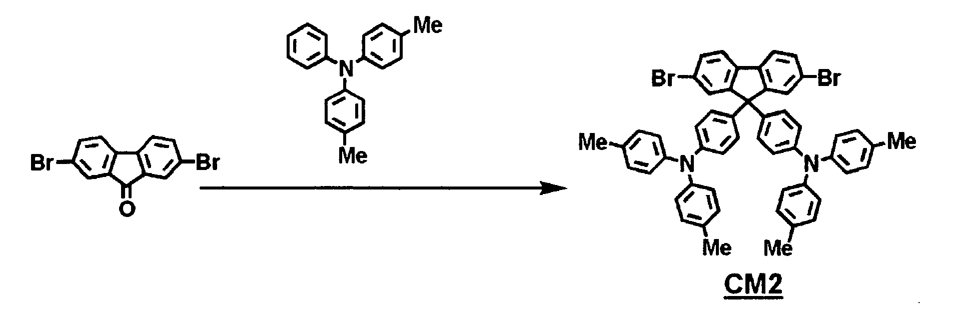

Under a nitrogen gas atmosphere, 2,7-dibromofluorenone (4.97 g, 14.7 mmol), 4,4'-dimethyltriphenylamine (10.5 g, 36.8 mmol), and methanesulfonic acid (1.41 g, 14.7 mmol) were stirred at 140°C for 5 hours under light-shielded conditions. Thereafter, the reaction solution was cooled to 50°C, and chloroform (50 ml) was then added thereto. The mixed solution was washed twice with a 10-weight-% sodium carbonate aqueous solution (50 ml), and it was then dried over anhydrous magnesium sulfate. The resultant was subjected to vacuum concentration, and it was then recrystallized (chloroform-acetone). The obtained solid was dissolved in methylene chloride (330 g) under heating, and activated carbon (3 g) was then added to the obtained solution. The obtained mixture was stirred at a room temperature for 1 hour. Thereafter, the activated carbon was removed using a filter that had been pre-coated with silica gel. Acetone was slowly added to the obtained solution for crystallization, followed by vacuum drying, so as to obtain a compound CM2 (9.8 g; yield: 77%; HPLC area percent: 99% or more) in the form of a white solid. ¹H-NMR (300 MHz, THF-d8) δ 2.26 (s, 12H), 6.80-7.20 (m, 24H), 7.5-7.8 (m, 6H) LC-MS M=864

### <Example 3> (Synthesis of polymer compound P1)

Under an argon gas atmosphere, 2,7-bis(1,3,2-dioxaborolane-2-yl)-9,9-dioctylfluorene (0.6537 g, 1.233 mmol), 2,7-dibromo-9,9-dioctylfluorene (0.5484 g, 1.000 mmol), the compound M1 (0.3597 g, 0.250 mmol), and trioctylmethylammonium chloride (manufactured by Aldrich; product name: Aliquat336) (0.16 g, 0.40 mmol) were dissolved in toluene (10 mL). Argon gas was bubbled into the solution, and the temperature was then increased to 80°C. A toluene suspension (5 mL) of dichlorobistriphenylphosphine palladium (1.8 mg, 2.5 µmol) was added to the solution, and a 17.5-weight% sodium carbonate aqueous solution (3.4 mL, 5.6 mmol) was further added thereto. The obtained mixture was reacted under reflux for 7 hours. Thereafter, the reaction solution was once cooled, and a solution prepared by dissolving phenylboric acid (0.0156 g, 0.128 mmol) in 2 mL of toluene was then added thereto. The mixture was further reacted under reflux for 2 hours. Thereafter, a water layer was removed, and a 9-weight-% sodium N,N-diethyldithiocarbamate aqueous solution (10 mL) was then added to the residue. The obtained mixture was stirred at 90°C for 2 hours, and an organic layer was successively washed with ion exchanged water (20 mL) twice, then with a 3-weight-% acetic acid aqueous solution (20 mL) twice, and then with ion exchanged water (20 mL) twice. Subsequently, the resultant was added dropwise to methanol (230 mL), and the obtained mixture was then stirred for 30 minutes, so as to precipitate a polymer. The polymer was collected by suction filtration, and it was then washed with methanol (45 mL), followed by vacuum drying, so as to obtain a crude polymer (1.163 g).

This crude polymer was dissolved in toluene (47 mL), and it was then supplied to a column filled with alumina (6.5 g) and silica gel (15 g). Thereafter, toluene (72 mL) was also supplied. The obtained solution was slowly added to methanol (230 mL) while stirring, and the obtained mixture was further stirred for 30 minutes, so as to precipitate a polymer. The polymer was collected by suction filtration, and it was then washed with methanol (45 mL), followed by vacuum drying, so as to obtain a polymer compound P1 (1.068 g; yield: 90.5%) as a polymer. The polystyrene equivalent number average molecular weight (Mn) of the polymer compound P1 was 6.7 × 10⁴, and the polystyrene equivalent weight average molecular weight (Mw) thereof was 1.7 × 10⁵. The glass transition temperature was 114°C, and the fluorescence peak wavelength of a thin film was 446 nm.

From the raw materials used, the polymer compound P1 was assumed to comprise the following repeating units at the following ratio (molar ratio).

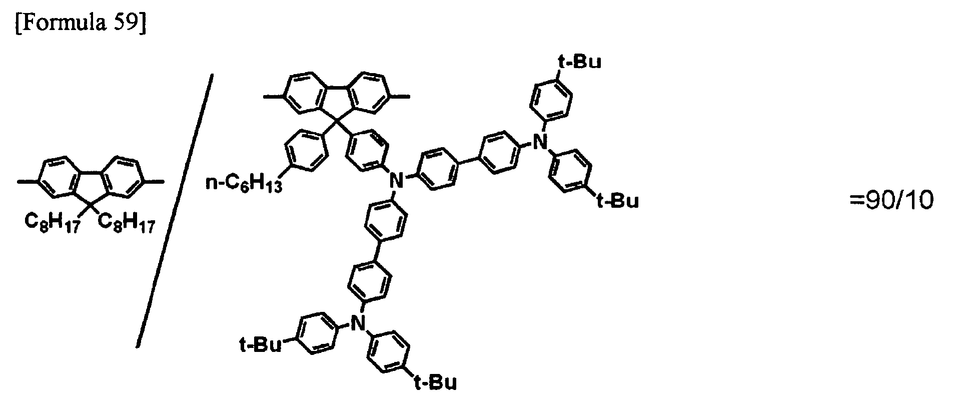

### <Example 4> (Synthesis of polymer compound P2)

Under an argon gas atmosphere, 2,7-bis(1,3,2-dioxaborolane-2-yl)-9,9-dioctylfluorene (0.7836 g, 1.478 mmol), 2,7-dibromo-9,9-dioctylfluorene (0.6581 g, 1.200 mmol), the compound M2 (0.3358 g, 0.300 mmol), and trioctylmethylammonium chloride (manufactured by Aldrich; product name: Aliquat336) (0.19 g, 0.47 mmol) were dissolved in toluene (10 mL). Argon gas was bubbled into the solution, and the temperature was then increased to 80°C. A toluene suspension (5 mL) of dichlorobistriphenylphosphine palladium (1.1 mg, 1.5 µmol) was added to the solution, and a 17.5-weight-% sodium carbonate aqueous solution (4.1 mL, 6.8 mmol) was further added thereto. The obtained mixture was reacted under reflux for 6 hours. Thereafter, the reaction solution was once cooled, and a solution prepared by dissolving phenylboric acid (0.0156 g, 0.128 mmol) in 4 mL of toluene was then added thereto. The mixture was further reacted under reflux for 2 hours. Thereafter, a water layer was removed, and a 9-weight-% sodium N,N-diethyldithiocarbamate aqueous solution (10 mL) was then added to the residue. The obtained mixture was stirred at 90°C for 2 hours, and an organic layer was successively washed with ion exchanged water (20 mL) twice, then with a 3-weight-% acetic acid aqueous solution (20 mL) twice, and then with ion exchanged water (20 mL) twice. Subsequently, the resultant was added dropwise to methanol (230 mL), and the obtained mixture was then stirred for 30 minutes, so as to precipitate a polymer. The polymer was collected by suction filtration, and it was then washed with methanol (45 mL), followed by vacuum drying, so as to obtain a crude polymer (1.23 g).

This crude polymer was dissolved in toluene (47 mL), and it was then supplied to a column filled with alumina (6.5 g) and silica gel (15 g). Thereafter, toluene (72 mL) was also supplied. The obtained solution was slowly added to methanol (230 mL) while stirring, and the obtained mixture was further stirred for 30 minutes, so as to precipitate a polymer. The polymer was collected by suction filtration, and it was then washed with methanol (45 mL), followed by vacuum drying, so as to obtain a polymer compound P2 (1.14 g; yield: 86.4%) as a polymer. The polystyrene equivalent number average molecular weight (Mn) of the polymer compound P2 was 1.2 × 10⁵, and the polystyrene equivalent weight average molecular weight (Mw) thereof was 2.5 × 10⁵. The glass transition temperature was 104°C, and the fluorescence peak wavelength of a thin film was 447 nm.

From the raw materials used, the polymer compound P2 was assumed to comprise the following repeating units at the following ratio (molar ratio).

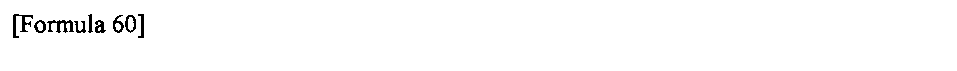

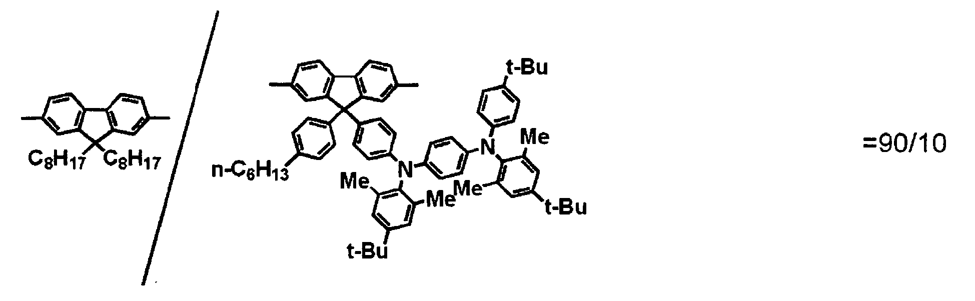

### <Example 5> (Synthesis of polymer compound P3)

Under an argon gas atmosphere, 2,7-bis(1,3,2-dioxaborolane-2-yl)-9,9-dioctylfluorene (0.7836 g, 1.478 mmol), 2,7-dibromo-9,9-dioctylfluorene (0.4938 g, 0.900 mmol), the compound M2 (0.6715 g, 0.453 mmol), and trioctylmethylammonium chloride (manufactured by Aldrich; product name: Aliquat336) (0.21 g, 0.52 mmol) were dissolved in toluene (10 mL). Argon gas was bubbled into the solution, and the temperature was then increased to 80°C. A toluene suspension (5 mL) of dichlorobistriphenylphosphine palladium (1.2 mg, 1.7 µmol) was added to the solution, and a 17.5-weight-% sodium carbonate aqueous solution (4.1 mL, 6.8 mmol) was further added thereto. The obtained mixture was reacted under reflux for 6 hours. Thereafter, the reaction solution was once cooled, and a solution prepared by dissolving phenylboric acid (0.0200 g, 0.164 mmol) in 3 mL of toluene was then added thereto. The mixture was further reacted under reflux for 2 hours. Thereafter, a water layer was removed, and a 9-weight-% sodium N,N-diethyldithiocarbamate aqueous solution (10 mL) was then added to the residue. The obtained mixture was stirred at 90°C for 2 hours, and an organic layer was successively washed with ion exchanged water (20 mL) twice, then with a 3-weight-% acetic acid aqueous solution (20 mL) twice, and then with ion exchanged water (20 mL) twice. Subsequently, the resultant was added dropwise to methanol (230 mL), and the obtained mixture was then stirred for 30 minutes, so as to precipitate a polymer. The polymer was collected by suction filtration, and it was then washed with methanol (45 mL), followed by vacuum drying, so as to obtain a crude polymer (1.41 g).

This crude polymer was dissolved in toluene (47 mL), and it was then supplied to a column filled with alumina (6.5 g) and silica gel (15 g). Thereafter, toluene (72 mL) was also supplied. The obtained solution was slowly added to methanol (230 mL) while stirring, and the obtained mixture was further stirred for 30 minutes, so as to precipitate a polymer. The polymer was collected by suction filtration, and it was then washed with methanol (45 mL), followed by vacuum drying, so as to obtain a polymer compound P3 (1.30 g; yield: 87.2%) as a polymer. The polystyrene equivalent number average molecular weight (Mn) of the polymer compound P3 was 5.5 × 10⁴, and the polystyrene equivalent weight average molecular weight (Mw) thereof was 1.2 × 10⁵. The glass transition temperature was 128°C, and the fluorescence peak wavelength of a thin film was 449 nm.

From the raw materials used, the polymer compound P3 was assumed to comprise the following repeating units at the following ratio (molar ratio).

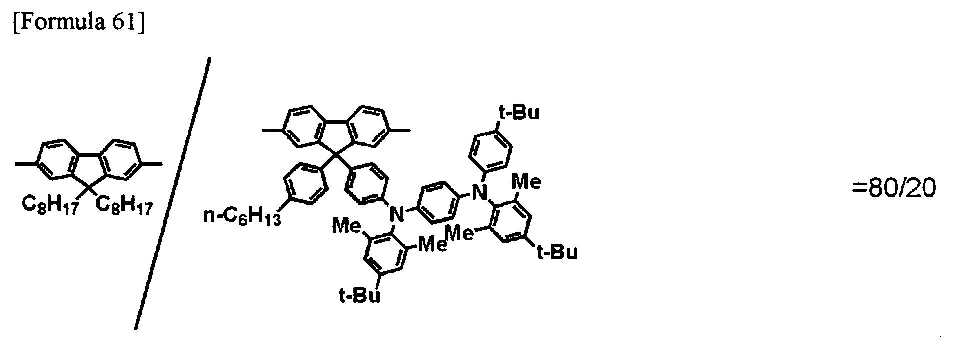

### <Example 6> (Synthesis of polymer compound P4)

Under an argon gas atmosphere, the compound M2 (1.12 g, 1.00 mmol) and 2,2'-bipyridyl (0.375 g, 2.40 mmol) were dissolved in commercially available anhydrous tetrahydrofuran (74 ml). Argon gas was bubbled into the solution, and the temperature was then increased to 60°C. Thereafter, [bis(1,4-cyclooctadiene)]nickel (0.66 g, 2.40 mmol) was added to the solution, and the obtained mixture was then reacted at 60°C for 2 hours. Thereafter, the reaction solution was cooled to a room temperature, and it was then poured into a mixed solution of 4-weight-% ammonia water (112 ml) and methanol (73 ml), so as to precipitate a polymer. The polymer was collected by filtration, and it was then subjected to vacuum drying, so as to obtain a crude polymer (1.07 g).

This crude polymer was dissolved in toluene (72 ml), and insoluble matter was then removed by filtration with Celite. The residue on the filter was washed with toluene (22 ml) 3 times, and it was then gathered with the above-described filtrate. The obtained mixture was supplied to a column filled with alumina (5.5 g) and silica gel (16.5 g), and toluene (100 ml) was further supplied thereto. The obtained solution was slowly added to methanol (450 ml), while stirring. The obtained mixture was further stirred for 30 minutes, so as to precipitate a polymer. The polymer was collected by suction filtration, and it was then washed with methanol (100 ml), followed by vacuum drying, so as to obtain a polymer compound P4 (0.85 g; yield: 89%) as a polymer. The polystyrene equivalent number average molecular weight (Mn) of the polymer compound P4 was 8.9 × 10⁴, and the polystyrene equivalent weight average molecular weight (Mw) thereof was 3.9 × 10⁵. The glass transition temperature was 269°C, and the fluorescence peak wavelength of a thin film was 453 nm.

From the raw materials used, the polymer compound P4 was assumed to comprise the following repeating units at the following ratio (molar ratio).

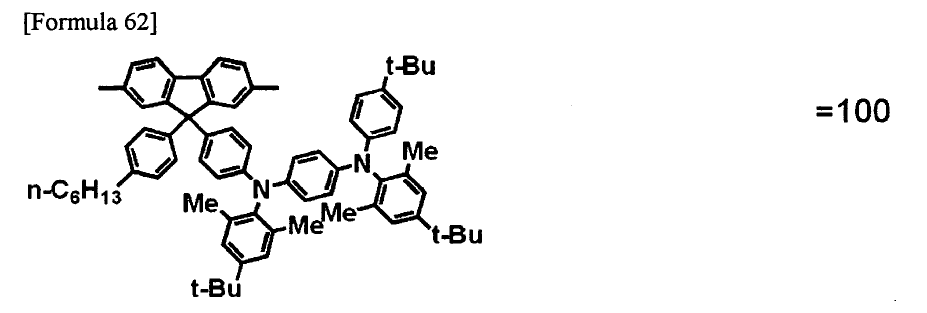

### <Comparative Example 3> (Synthesis of polymer compound CP1)

Under an argon gas atmosphere, 2,7-bis(1,3,2-dioxaborolane-2-yl)-9,9-dioctylfluorene (0.9323 g, 1.700 mmol), 2,7-dibromo-9,9-dioctylfluorene (0.9588 g, 1.717 mmol), and trioctylmethylammonium chloride (manufactured by Aldrich; product name: Aliquat336) (0.68 g, 1.7 mmol) were dissolved in toluene (27 mL). Argon gas was bubbled into the solution, and the temperature was then increased to 80°C. A toluene suspension (5 mL) of tetrakistriphenylphosphine palladium(0) (3.9 mg, 3.4 µmol) was added to the solution, and a 2.5-weight-% potassium carbonate aqueous solution (29.7 ml, 5.36 mmol) was further added thereto. The obtained mixture was reacted under reflux for 17 hours. Thereafter, the reaction solution was once cooled, and a solution prepared by dissolving bromobenzene (0.035 g, 0.22 mmol) in 3 mL of toluene was then added thereto. The mixture was further reacted under reflux for 2 hours. Thereafter, the reaction solution was cooled to a room temperature, and it was then added dropwise to methanol (200 mL). The mixture was stirred for 30 minutes, so as to precipitate a polymer. The polymer was collected by suction filtration, and it was then washed with methanol (70 mL), followed by vacuum drying, so as to obtain a crude polymer.

This crude polymer was dissolved in toluene (35 mL), and it was then supplied to a column filled with alumina (10 g) and silica gel (20 g). Thereafter, toluene (50 mL) was also supplied. The obtained solution was slowly added to methanol (120 mL) while stirring, and the obtained mixture was further stirred for 30 minutes, so as to precipitate a polymer. The polymer was collected by suction filtration, and it was then washed with methanol (70 mL), followed by vacuum drying, so as to obtain a polymer compound CP1 (1.04 g; yield: 78%) as a polymer. The polystyrene equivalent number average molecular weight (Mn) of the polymer compound CP1 was 4.8 × 10⁴, and the polystyrene equivalent weight average molecular weight (Mw) thereof was 1.1 × 10⁵. The glass transition temperature was 69°C, and the fluorescence peak wavelength of a thin film was 439 nm.

From the raw materials used, the polymer compound CP1 was assumed to comprise the following repeating units at the following ratio (molar ratio).

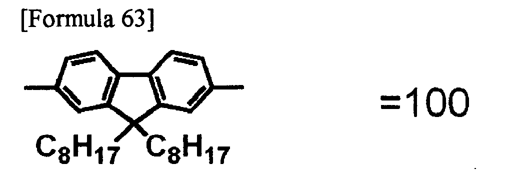

### <Comparative Example 4> (Synthesis of polymer compound CP2)

Under an argon gas atmosphere, 2,7-bis(1,3,2-dioxaborolane-2-yl)-9,9-dioctylfluorene (1.1814 g, 2.228 mmol), 2,7-dibromo-9,9-dioctylfluorene (0.9872 g, 1.800 mmol), the compound CM2 (0.3900 g, 0.450 mmol), and trioctylmethylammonium chloride (manufactured by Aldrich; product name: Aliquat336) (0.29 g, 0.72 mmol) were dissolved in toluene (17.5 mL). Argon gas was bubbled into the solution, and the temperature was then increased to 80°C. A toluene suspension (5 mL) of dichlorobistriphenylphosphine palladium (1.6 mg, 2.3 µmol) was added to the solution, and a 20-weight-% tetraethylammonium aqueous solution (7.3 ml, 10.4 mmol) was further added thereto. The obtained mixture was reacted under reflux for 7 hours. Thereafter, the reaction solution was once cooled, and a solution prepared by dissolving phenylboric acid (0.27 g, 2.25 mmol) in 3 mL of toluene was then added thereto. The mixture was further reacted under reflux for 2 hours. Thereafter, toluene (22 ml) was added to the reaction solution for dilution. Subsequently, a water layer was removed, and a 9-weight-% sodium N,N-diethyldithiocarbamate aqueous solution (14 mL) was then added to the residue. The obtained mixture was stirred at 90°C for 2 hours, and an organic layer was successively washed with ion exchanged water (30 mL) twice, then with a 3-weight-% acetic acid aqueous solution (30 mL) twice, and then with ion exchanged water (30 mL) twice. Subsequently, the resultant was added dropwise to methanol (350 L), and the obtained mixture was then stirred for 30 minutes, so as to precipitate a polymer. The polymer was collected by suction filtration, and it was then washed with methanol (70 mL), followed by vacuum drying, so as to obtain a crude polymer (1.89 g).

This crude polymer was dissolved in toluene (70 mL), and it was then supplied to a column filled with alumina (15 g) and silica gel (35 g). Thereafter, toluene (110 mL) was also supplied. The obtained solution was slowly added to methanol (350 mL) while stirring, and the obtained mixture was further stirred for 30 minutes, so as to precipitate a polymer. The polymer was collected by suction filtration, and it was then washed with methanol (70 mL), followed by vacuum drying, so as to obtain a polymer compound CP2 (1.65 g; yield: 88%) as a polymer. The polystyrene equivalent number average molecular weight (Mn) of the polymer compound CP2 was 2.8 × 10⁴, and the polystyrene equivalent weight average molecular weight (Mw) thereof was 6.1 × 10⁴. The glass transition temperature was 89°C, and the fluorescence peak wavelength of a thin film was 438 nm.

From the raw materials used, the polymer compound CP2 was assumed to comprise the following repeating units at the following ratio (molar ratio).

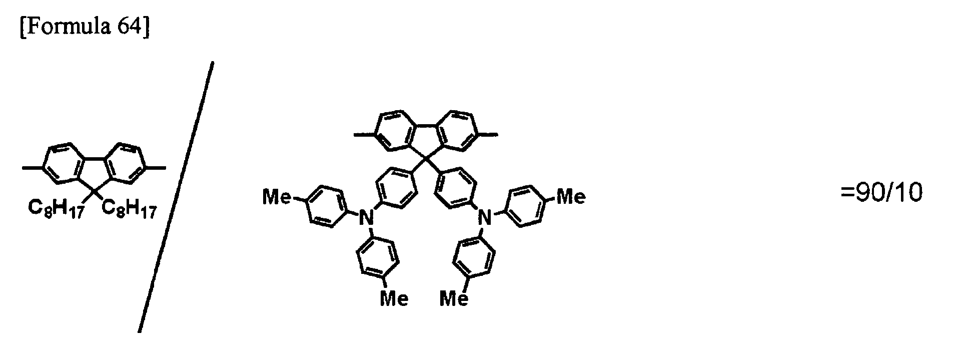

### <Comparative Example 5> (Synthesis of polymer compound CP3)

Under a nitrogen gas atmosphere, 2,7-bis(1,3,2-dioxaborolane-2-yl)-9,9-dioctylfluorene (0.424 g, 0.800 mmol), 2,7-dibromo-9,9-dioctylfluorene (0.254 g, 0.464 mmol), the compound CM1 (0.516 g, 0.348 mmol), trioctylmethylammonium chloride (manufactured by Aldrich; product name: Aliquat336) (0.20 g, 0.49 mmol), palladium acetate (0.5 mg, 2.4 µmol), tris(2-methylphenyl)phosphine (2.0 mg, 6.5 µmol), toluene (10 mL), and a 17.5-weight-% sodium carbonate aqueous solution (2 ml, 3.3 mmol) were reacted under reflux for 3 hours. Thereafter, the reaction solution was once cooled, and a mixed solution of phenylboric acid (20 mg, 0.16 mmol) and tetrahydrofuran (2 ml) was added to the solution. The obtained mixture was further reacted under reflux for 3 hours. Subsequently, a sodium N,N-diethyldithiocarbamate aqueous solution was added to the reaction solution, and the obtained mixture was stirred at 85°C for 4 hours. Thereafter, an organic layer was washed with water (30 ml) twice, then with a 3-weight-% acetic acid aqueous solution (30 ml) 4 times, and then with water (30 ml) 3 times. Subsequently, the resultant was supplied to a column filled with alumina and silica gel. The obtained solution was slowly added to methanol (250 ml) while stirring, and the obtained mixture was further stirred for 1 hour, so as to precipitate a polymer. The polymer was collected by suction filtration, and it was then washed with methanol, followed by vacuum drying, so as to obtain a polymer compound CP3 (719 mg; yield: 74.9%) as a polymer. The polystyrene equivalent number average molecular weight (Mn) of the polymer compound CP3 was 4.5 × 10⁴, and the polystyrene equivalent weight average molecular weight (Mw) thereof was 1.1 × 10⁵. The glass transition temperature was 90°C, and the fluorescence peak wavelength of a thin film was 481 nm.

From the raw materials used, the polymer compound CP3 was assumed to comprise the following repeating units at the following ratio (molar ratio).

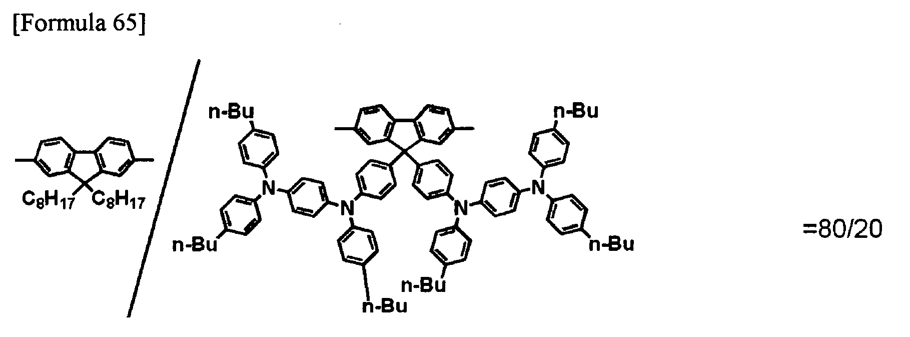

### <Comparative Example 6> (Synthesis of polymer compound CP4)

The compound CM1 (0.500 g, 0.34 mmol) and 2,2'-bipyridyl (0.126 g, 0.81 mmol) were dissolved in anhydrous tetrahydrofuran (24 ml) that had previously been bubbled with argon gas. Subsequently, [bis(1,4-cyclooctadiene)]nickel(0){Ni(COD)₂} (0.223 g, 0.81 mmol) was added to the solution, and the obtained mixture was then stirred. This solution was heated to 60°C, and it was then reacted for 3 hours. Thereafter, the reaction solution was cooled to a room temperature, and it was then added dropwise to a mixed solution of 25-weight-% ammonia water (1 ml), methanol (24 ml), and ion exchange water (24 ml). The obtained solution was stirred for 1 hours. Thereafter, the obtained precipitate was filtrated, and it was then subjected to vacuum drying. Subsequently, the resultant was dissolved in toluene (10 ml), and it was then filtrated with Celite to remove insoluble matter. The filtrate was supplied to an alumina column. Thereafter, 4-weight-% ammonia water (approximately 20 ml) was added thereto, and the mixture was stirred for 2 hours. Then, a water layer was removed. Further, ion exchanged water (approximately 20 ml) was added to an organic layer, the obtained mixture was then stirred for 1 hour, and water layer was then removed. Thereafter, the organic layer was added to methanol (60 ml) while stirring, and the obtained mixture was further stirred for 30 minutes, so as to precipitate a polymer. The polymer was collected by suction filtration, followed by vacuum drying, so as to obtain a polymer compound CP4 (0.28 g; yield: 63%) as a polymer. The polystyrene equivalent number average molecular weight (Mn) of the polymer compound CP4 was 9.8 × 10⁴, and the polystyrene equivalent weight average molecular weight (Mw) thereof was 2.3 × 10⁵. The glass transition temperature was 125°C, and the fluorescence peak wavelength of a thin film was 494 nm.

From the raw materials used, the polymer compound CP4 was assumed to comprise the following repeating units at the following ratio (molar ratio).

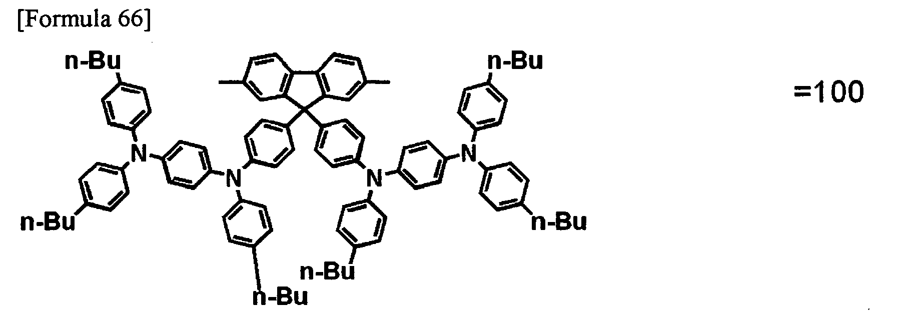

### <Comparative Example 7> (Synthesis of polymer compound CP5)

Under an argon gas atmosphere, 2,7-bis(1,3,2-dioxaborolane-2-yl)-9,9-dioctylfluorene (0.7836 g, 1.478 mmol), 2,7-dibromo-9,9-dioctylfluorene (0.6583 g, 1.200 mmol), the compound CM1 (0.4444 g, 0.300 mmol), and trioctylmethylammonium chloride (manufactured by Aldrich; product name: Aliquat336) (0.20 g, 0.49 mmol) were dissolved in toluene (10 mL). Argon gas was bubbled into the solution, and the temperature was then increased to 80°C. A toluene suspension (5 mL) of dichlorobistriphenylphosphine palladium (1.1 mg, 1.5 µmol) was added to the solution, and a 17.5-weight-% sodium carbonate aqueous solution (4.1 mL, 6.8 mmol) was further added thereto. The obtained mixture was reacted under reflux for 6 hours. Thereafter, the reaction solution was once cooled, and a solution prepared by dissolving phenylboric acid (0.0196 g, 0.161 mmol) in 3 mL of toluene was then added thereto. The mixture was further reacted under reflux for 2 hours. Thereafter, a water layer was removed, and a 9-weight-% sodium N,N-diethyldithiocarbamate aqueous solution (10 mL) was then added to the residue. The obtained mixture was stirred at 90°C for 2 hours, and an organic layer was successively washed with ion exchanged water (20 mL) twice, then with a 3-weight-% acetic acid aqueous solution (20 mL) twice, and then with ion exchanged water (20 mL) twice. Subsequently, the resultant was added dropwise to methanol (230 mL), and the obtained mixture was then stirred for 30 minutes, so as to precipitate a polymer. The polymer was collected by suction filtration, and it was then washed with methanol (45 mL), followed by vacuum drying, so as to obtain a crude polymer (1.29 g).

This crude polymer was dissolved in toluene (47 mL), and it was then supplied to a column filled with alumina (6.5 g) and silica gel (15 g). Thereafter, toluene (72 mL) was also supplied. The obtained solution was slowly added to methanol (230 mL) while stirring, and the obtained mixture was further stirred for 30 minutes, so as to precipitate a polymer. The polymer was collected by suction filtration, and it was then washed with methanol (45 mL), followed by vacuum drying, so as to obtain a polymer compound CP5 (1.19 g; yield: 83.2%) as a polymer. The polystyrene equivalent number average molecular weight (Mn) of the polymer compound CP5 was 7.3 × 10⁴, and the polystyrene equivalent weight average molecular weight (Mw) thereof was 1.8 × 10⁵. The glass transition temperature was 81°C, and the fluorescence peak wavelength of a thin film was 480 nm.

From the raw materials used, the polymer compound CP5 was assumed to comprise the following repeating units at the following ratio (molar ratio).

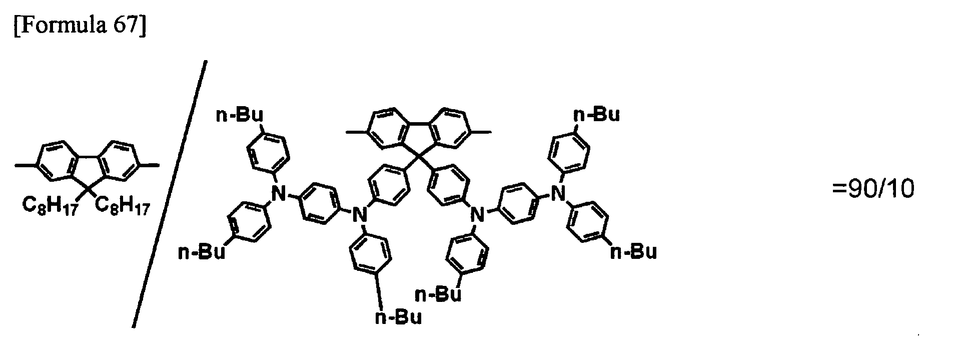

### <Example 7> (Production of light-emitting device DP1)

The polymer compound P1 was dissolved in xylene (manufactured by Kanto Kagaku Kabushiki Kaisha; grade for electronic industry). This time, the solution was prepared such that the concentration of a solid was set at 1.3% by weight. Using a poly(3,4-ethylenedioxythiophene)/polystyrenesulfonic acid solution (manufactured by Bayer; product name: BaytronP CH8000), a film having a thickness of 65 nm was formed by spin-coating on a glass substrate on which an 150-nm thick ITO film had been formed by a sputtering method, and it was then dried on a hot plate at 200°C for 10 minutes. Subsequently, using the above prepared xylene solution, a film was formed by spin-coating at a rotation rate of 1700 rpm. The thickness of the film was found to be approximately 100 nm. This film was dried at 130°C for 20 minutes under a nitrogen gas atmosphere, and thereafter, as a cathode, approximately 4 nm lithium fluoride, then approximately 5 nm calcium, and finally approximately 100 nm aluminum were applied thereto via evaporation, so as to produce a light-emitting device DP1. The device was composed of ITO/BaytronP (65 nm)/polymer compound P1/LiF/Ca/Al. After the degree of vacuum had reached 1 × 10⁻⁴ Pa or less, the vapor deposition of the metals was initiated.

Voltage was applied to the light-emitting device DP1. As a result, the device emitted deep blue light having a peak wavelength (EL) derived from the polymer compound P1 at 450 nm. The maximum luminous efficiency was 1.1 cd/A. At that time, the voltage was 3.8 V, and the external quantum yield was 1.8%. The voltage at a luminance of 100 cd/m² was 4.4 V, and the chromaticity coordinate C.I.E. 1931 was (x, y) = (0.16, 0.07). The luminous efficiency was 1.1 cd/A, and the external quantum yield was 1.8%. These results are shown in Table 1.

### <Example 8> (Production of light-emitting device DP2)

A light-emitting device DP2 was produced in the same manner as that of Example 7, with the exceptions that a 1.2-weight-% xylene solution of the polymer compound P2 was used instead of a 1.3-weight-% xylene solution of the polymer compound P1, and that the rotation rate in spin-coating was set at 2500 rpm instead of 1700 rpm.

Voltage was applied to the light-emitting device DP2. As a result, the device emitted deep blue light having a peak wavelength (EL) derived from the polymer compound P2 at 450 nm. The maximum luminous efficiency was 1.9 cd/A. At that time, the voltage was 3.8 V, and the external quantum yield was 1.7%. The voltage at a lumiance of 100 cd/m² was 5.5 V, and the chromaticity coordinate C.I.E. 1931 was (x, y) = (0.16, 0.13). The luminous efficiency was 1.6 cd/A, and the external quantum yield was 1.5%. These results are shown in Table 1.

### <Example 9> (Production of light-emitting device DP3)

A light-emitting device DP3 was produced in the same manner as that of Example 7, with the exceptions that a 1.5-weight-% xylene solution of the polymer compound P3 was used instead of a 1.3-weight% xylene solution of the polymer compound P1, and that the rotation rate in spin-coating was set at 2500 rpm instead of 1700 rpm.

Voltage was applied to the light-emitting device DP3. As a result, the device emitted deep blue light having a peak wavelength (EL) derived from the polymer compound P3 at 450 nm. The maximum luminous efficiency was 1.6 cd/A. At that time, the voltage was 4.6 V, and the external quantum yield was 1.3%. The voltage at a luminance of 100 cd/m² was 4.5 V, and the chromaticity coordinate C.I.E. 1931 was (x, y) = (0.16, 0.15). The luminous efficiency was 1.6 cd/A, and the external quantum yield was 1.3%. These results are shown in Table 1.

### <Comparative Example 8> (Production of light-emitting device DCP1)

The polymer compound CP1 was dissolved in toluene (manufactured by Kanto Kagaku Kabushiki Kaisha; grade for electronic industry). This time, the solution was prepared such that the concentration of a solid was set at 1.5% by weight. Using a poly(3,4-ethylenedioxythiophene)/polystyrenesulfonic acid solution (manufactured by Bayer; product name: BaytronP CH8000), a film having a thickness of 70 nm was formed by spin-coating on a glass substrate on which an 150-nm thick ITO film had been formed by a sputtering method, and it was then dried on a hot plate at 200°C for 10 minutes. Subsequently, using the above prepared toluene solution, a film was formed by spin-coating at a rotation rate of 1000 rpm. The thickness of the film was found to be approximately 93 nm. This film was dried at 130°C for 20 minutes under a nitrogen gas atmosphere, and thereafter, as a cathode, approximately 4 nm lithium fluoride, then approximately 5 nm calcium, and finally approximately 72 nm aluminum were applied thereto via evaporation, so as to produce a light-emitting device DCP1. The device was composed of ITO/BaytronP (70 nm)/polymer compound CP1/LiF/Ca/Al. After the degree of vacuum had reached 1 × 10⁻⁴ Pa or less, the vapor deposition of the metals was initiated.

Voltage was applied to the light-emitting device DCP1. As a result, the device emitted deep blue light having a peak wavelength (EL) derived from the polymer compound CP1 at 440 nm. The maximum luminous efficiency was 0.44 cd/A. At that time, the voltage was 5.4 V, and the external quantum yield was 0.30%. The voltage at a luminance of 100 cd/m² was 4.9 V, and the chromaticity coordinate C.I.E. 1931 was (x, y) = (0.15, 0.10). The luminous efficiency was 0.36 cd/A, and the external quantum yield was 0.16%. These results are shown in Table 1.

### <Comparative Example 9> (Production of light-emitting device DCP2)

A light-emitting device DCP2 was produced in the same manner as that of Example 7, with the exceptions that a 1.7-weight-% xylene solution of the polymer compound CP2 was used instead of a 1.3-weight-% xylene solution of the polymer compound P1, and that the rotation rate in spin-coating was set at 2200 rpm instead of 1700 rpm.

Voltage was applied to the light-emitting device DCP2. As a result, the device emitted deep blue light having a peak wavelength (EL) derived from the polymer compound CP2 at 450 nm. The maximum luminous efficiency was 0.18 cd/A. At that time, the voltage was 5.6 V, and the external quantum yield was 0.29%. The voltage at a luminance of 100 cd/m² was 4.8 V, and the chromaticity coordinate C.I.E. 1931 was (x, y) = (0.16, 0.07). The luminous efficiency was 0.17 cd/A, and the external quantum yield was 0.29%. These results are shown in Table 1.

### <Comparative Example 10> (Production of light-emitting device DCP3)

A light-emitting device DCP3 was produced in the same manner as that of Example 7, with the exceptions that a 1.5-weight% xylene solution of the polymer compound CP3 was used instead of a 1.3-weight-% xylene solution of the polymer compound P1, and that the rotation rate in spin-coating was set at 1600 rpm instead of 1700 rpm.

Voltage was applied to the light-emitting device DCP3. As a result, the device emitted blue green light having a peak wavelength (EL) derived from the polymer compound CP3 at 480 nm. The maximum luminous efficiency was 1.0 cd/A. At that time, the voltage was 5.4 V, and the external quantum yield was 0.55%. The voltage at a luminance of 100 cd/m² was 4.2 V, and the chromaticity coordinate C.I.E. 1931 was (x, y) = (0.20, 0.29). The luminous efficiency was 0.80 cd/A, and the external quantum yield was 0.43%. These results are shown in Table 1.

### <Comparative Example 11> (Production of light-emitting device DCP4)

A light-emitting device DCP4 was produced in the same manner as that of Comparative Example 8, with the exceptions that a 1.5-weight-% toluene solution of the polymer compound CP4 was used instead of a 1.5-weight-% toluene solution of the polymer compound CP1, and that the rotation rate in spin-coating was set at 1600 rpm instead of 1700 rpm.

Voltage was applied to the light-emitting device DCP4. As a result, the device emitted green light having a peak wavelength (EL) derived from the polymer compound CP4 at 500 nm. The maximum luminous efficiency was 0.58 cd/A. At that time, the voltage was 8.4 V, and the external quantum yield was 0.24%. The voltage at a luminance of 100 cd/m² was 5.2 V, and the chromaticity coordinate C.I.E. 1931 was (x, y) = (0.34, 0.45). The luminous efficiency was 0.35 cd/A, and the external quantum yield was 0.16%. These results are shown in Table 1.

[Table 1]

**Table 1**

| | Polymer compound | Maximum external quantum yield (%) | Value at luminance of 100cd/m² | | Peak wavelength | |
|---|---|---|---|---|---|---|
| | | | External quantum yield (%) | Chromaticity CIE (x,y) | λ max | |
| | | | | | EL | PL |
| Example 7 | P1 | 1.8 | 1.8 | (0.16, 0.07) | 450nm | 446nm |
| Example 8 | P2 | 1.7 | 1.5 | (0.16, 0.13) | 450nm | 447nm |
| Example 9 | P3 | 1.3 | 1.3 | (0.16, 0.15) | 450nm | 449nm |
| Comparative Example 8 | CP1 | 0.30 | 0.16 | (0.15, 0.10) | 440nm | 439nm |
| Comparative Example 9 | CP2 | 0.29 | 0.29 | (0.16, 0.07) | 450nm | 438nm |
| Comparative Example 10 | CP3 | 0.55 | 0.43 | (0.20, 0.29) | 480nm | 481nm |
| Comparative Example 11 | CP4 | 0.24 | 0.16 | (0.34, 0.45) | 500nm | 494nm |

### <Example 10> (Synthesis of compound M3)

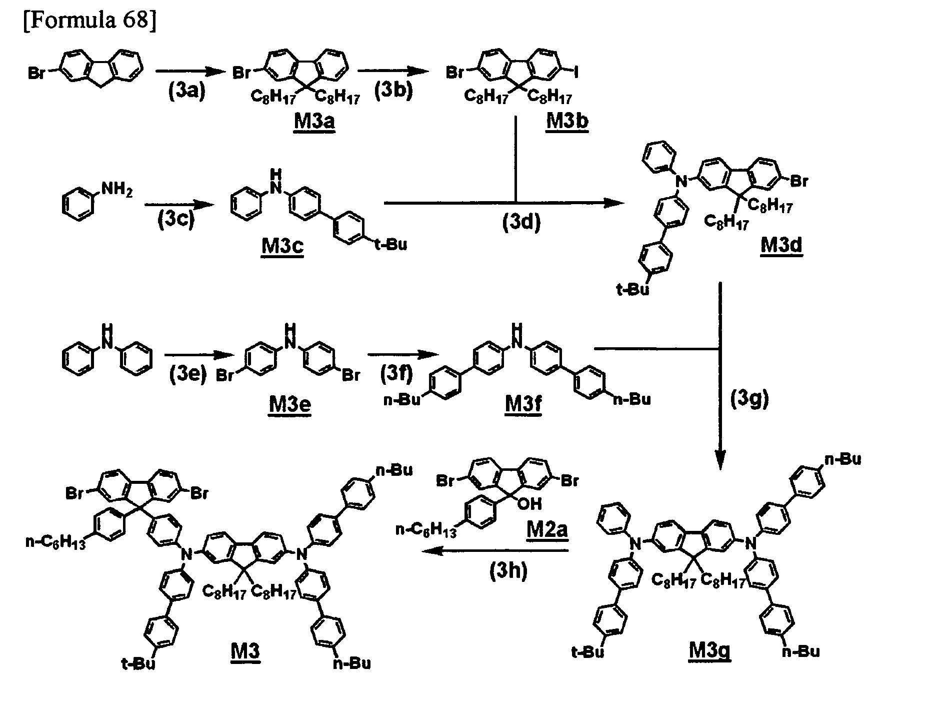

### • Step (3a)

In a 500-ml four-necked flask, 2-bromofluorene (17.16 g, 70 mmol), octyl bromide (27.71 g, 141 mmol), potassium hydroxide (16.69 g, 298 mmol) ground in a mortar, potassium iodide (1.16 g, 7.0 mmol) ground in a mortar, and DMSO (169 ml) were mixed, and the obtained mixture was then stirred at a room temperature for 7 hours. After completion of the reaction, ion exchange water (300 ml) and toluene (400 ml) were added to the reaction solution, followed by liquid separation. The resultant was washed with a saturated sodium chloride aqueous solution (200 ml × 5), and an organic layer was dried over sodium sulfate, followed by vacuum concentration. The obtained oil was purified using an intermediate pressure fractionation column (silica gel, hexane), so as to obtain a compound M3a (28.2 g, 86%).

### • Step (3b)

Under an argon atmosphere, the compound M3a (20.19 g, 43 mmol), iodine (6.55 g, 26 mmol), periodic acid (1.65 g, 8.6 mmol), concentrated sulfuric acid (2 ml), water (8 ml), and acetic acid (75 ml) were mixed in a 300-mL four-necked flask, and the obtained mixture was then stirred at 50°C for 8 hours. Thereafter, iodine (6.55 g, 26 mmol) was added to the reaction solution, and the obtained mixture was then stirred at 50°C for 6 hours. After completion of the reaction, water (100 ml) and toluene (300 ml) were added to the reaction solution, and a water layer was removed. Thereafter, an organic layer was washed with a sodium thiosulfate aqueous solution (100 ml) and ion exchanged water (300 ml × 3), and it was then dried over sodium sulfate, followed by vacuum drying. The obtained oil was purified using an intermediate pressure fractionation column (silica gel, hexane), so as to obtain a compound M3b (23.3 g, 91%).
¹H-NMR (270 MHz, THF-d₈): δ=0.68-0.86 (m, 4H), 1.01 (t, 6H), 1.14-1.52 (m, 20H), 2.12-2.22 (m, 4H), 7.63 (dd, 1H), 7.69 (d, 1H), 7.74 (d, 1H), 7.80-7.86 (m, 2H), 7.94 (d, 1H) ppm.
¹³C-NMR (270 MHz, THF-d₈): δ=15.6, 24.6, 31.2, 31.3, 31.9, 33.9, 41.8, 57.7, 94.7, 123.4, 123.5, 128.2, 132.2, 133.1, 134.2, 138.2, 139.2, 141.5, 142.0, 154.5, 154.8 ppm.

### • Step (3c)

Under an argon atmosphere, aniline (5.59 g, 60 mmol), 4-bromo-4'-butyl-biphenyl (17.29 g, 60 mmol), sodium-t-butoxide (8.65 g, 90 mmol), tri-o-tolylphosphine (1.46 mg, 4.8 mmol), and toluene (180 ml) were mixed in a 500-ml four-necked flask. Tris(dibenzylideneacetone)dipalladium(0) (1.10 g, 1.2 mmol) was further added thereto, and the obtained mixture was then heated to 120°C for 5 hours. After completion of the reaction, chloroform (300 ml) was added to the reaction solution, and the obtained mixture was then washed with ion exchanged water (100 ml × 3). An organic layer was dried over sodium sulfate, followed by vacuum concentration. Thereafter, toluene (200 ml) was added to the obtained composition, and it was then dissolved therein by reflux. The obtained toluene solution was passed through Celite and silica gel, and it was then intensively washed with toluene (150 ml) that had been heated to 80°C. The resultant was consolidated by concentration. The obtained solid was recrystallized from toluene (30 ml), so as to obtain a compound M3 c (9.18 g, 51%).
¹H-NMR (270 MHz, TBF-d₈): δ=1.51 (s, 9H), 6.98 (dd, 1H), 7.27 (dd, 4H), 7.36 (dd, 2H), 7.58 (m, 3H), 7.65 (m, 4H) ppm.
¹³C-NMR (270 MHz, THF-d₈): δ=31.0, 117.4, 120.1, 125.5, 125.7, 125.9, 127.4, 129.1, 132.8, 138.4, 143.3, 144.0, 149.0 ppm.

### • Step (3d)

Under an argon atmosphere, the compound M3c (8.67 g, 20 mmol), the compound M3b (11.91 g, 20 mmol), 1,10-phenanthroline (1.8 g, 10 mmol), copper(I) chloride (0.99 g, 10 mmol), potassium hydroxide (7.99 g, 160 mmol) ground in a mortar, and toluene (40 ml) were mixed in a 300-ml four-necked flask, and while stirring with a mechanical stirrer, the obtained mixture was heated to 135°C for 7 hours. After completion of the reaction, toluene (300 ml) was added to the reaction solution, and the obtained mixture was then filtrated with Celite. The obtained toluene solution was washed with ion exchanged water (100 m × 5), and an organic layer was dried over sodium sulfate. The resultant was then consolidated by concentration. The obtained solid was purified using an intermediate pressure fractionation column (silica gel, hexane), so as to obtain a compound M3d (10.1 g, 65%).
MS (APCI-MS: Positive) m/z: 770 ([M+H]⁺)
¹H-NMR (270 MHz, THF-d₈): δ=0.70-0.88 (m, 4H), 0.96 (t, 6H), 1.11-1.42 (m, 20H), 1.46 (s, 9H), 2.04 (t, 4H), 7.10-7.17 (m, 2H), 7.25 (d, 4H), 7.31 (d, 1H), 7.37 (t, 2H), 7.52-7.59 (m, 3H), 7.60-7.66 (m, 5H), 7.69 (d, 1H), 7.74 (d, 1H) ppm.

### • Step (3e)

Under an argon atmosphere, diphenylamine (30.00 g, 177 mmol) was mixed with chloroform (380 ml) in a 300-ml four-necked flask, and the obtained mixture was then cooled to 0°C. A solution prepared by dissolving tetrabutylammonium tribromide (171 g, 354 mmol) in chloroform (750 ml) was added dropwise thereto over 4 hours, while maintaining the temperature at 0°C. After completion of the reaction, ion exchanged water (70 ml) was added to the reaction solution, and the obtained mixture was heated to a room temperature. The obtained solution was washed with a saturated sodium bicarbonate aqueous solution (450 ml × 4), and an organic layer was then dried over sodium sulfate, followed by vacuum concentration. Toluene (500 ml) was added to the obtained oil. The obtained mixture was washed with ion exchanged water (500 ml × 10), and an organic layer was then dried over sodium sulfate, followed by vacuum concentration. The obtained solid was recrystallized from toluene (45 ml) and hexane (20 ml), so as to obtain a compound M3e (41.2 g, 71%).
¹H-NMR (270 MHz, THF-d₈): δ=7.08 (d, 2H), 7.43 (d, 2H), 7.66 (s, 1H) ppm.
¹³C-NMR (270 MHz, THF-d₈): δ=114.0, 121.0, 134.0, 144.8 ppm.

### • Step (3f)

Under an argon atmosphere, the compound M3e (18.0 g, 55 mmol), n-buthylphenylboric acid (21.5 g, 121 mmol), Aliquat336 (7.11 g, 18 mmol), and toluene (300 ml) were mixed in a 1-L four-necked flask, and argon was then bubbled into the obtained mixture for 1 hour. Thereafter, palladium acetate (123 mg, 0.55 mmol) and tris(o-methoxyphenyl)phosphine (1.36 g, 3.9 mmol) were further added to the reaction solution, and the obtained mixture was heated to 105°C. To this reaction solution, a 2M sodium carbonate aqueous solution (90 ml) was added dropwise over 1 hour, followed by reflux for 10 hours. After completion of the reaction, the reaction solution was cooled to a room temperature, and it was then washed with ion exchanged water (100 ml × 10). Chloroform (500 ml) was added to an organic layer, and the mixture was then washed with ion exchanged water (100 ml × 4). The resultant was filtrated with Radiolite, and it was dried over sodium sulfate and was concentrated. Toluene (100 ml) was added to the obtained oil, and it was then dissolved therein by reflux.
The obtained toluene solution was passed through Celite and silica gel, and it was then intensively washed with toluene (150 ml) that had been heated to 80°C. The resultant was consolidated by concentration. The obtained solid was recrystallized from toluene, so as to obtain a compound M3f (14.2 g, 60%).
¹H-NMR (270 MHz, THF-d₈): δ=1.11 (t, 6H), 1.53 (m, 4H), 1.78 (m, 4H), 2.77 (t, 4H), 7.34 (m, 8H), 7.65 (m, 9H) ppm.
¹³C-NMR (270 MHz, THF-d₈): δ=15.5, 24.4, 35.9, 37.2, 119.5, 128.0, 129.3, 130.6, 134.9, 140.5, 142.8, 145.0 ppm.

### • Step (3g)

Under an argon atmosphere, the compound M3d (10.0 g, 13 mmol), the compound M3f(6.2 g, 14 mmol), sodium-t-butoxide (1.87 g, 20 mmol), tris(dibenzylideneacetone)dipalladium(0) (238 mg, 0.26 mmol), and toluene (135 ml) were mixed in a 500-ml four-necked flask, and the obtained mixture was then heated to 40°C. Further, tri-t-butylphosphine (210 mg, 1.1 mmol) was added to the reaction solution, and the obtained mixture was heated to 80°C for 3 hours. After completion of the reaction, the reaction solution was cooled to a room temperature, and it was then washed with a saturated sodium chloride aqueous solution (250 ml × 4). An organic layer was washed with sodium sulfate, and it was passed through silica gel and was concentrated. The obtained oil was purified using an intermediate pressure fractionation column (silica gel, hexane : toluene = 5 : 1), so as to obtain a compound M3g (13.5 g, 93%).
MS (APCI-MS: Positive) m/z: 1122 ([M+H]⁺)
¹H-NMR (270 MHz, THF-d₈): δ=0.83-1.05 (m, 10H), 1.12 (t, 6H), 1.20-1.47 (m, 20H), 1.51 (s, 9H), 1.53-1.64 (m, 4H), 1.75-1.85 (m, 4H), 2.03 (t, 4H), 2.81 (t, 4H), 7.13-7.26 (m, 3H), 7.29-7.44 (m, 16H), 7.60 (d, 2H), 7.65-7.71 (m, 12H), 7.73 (d, 1H), 7.76 (d, 1H) ppm.

### • Step (3h)

Under an argon atmosphere, the compound M3g (12.3 g, 11 mmol), the compound M2a (5.6 g, 11 mmol) synthesized in the same manner as described above, and dichloromethane (200 ml) were mixed in a 500-ml four-necked flask. Under a room temperature, a mixed solution of a trifluoroborane ether complex (1.6 ml, 13 mmol) and dichloromethane (35 ml) was added dropwise to the above mixed solution over 1 hour, and the obtained mixture was then stirred for 5 hours. After completion of the reaction, ion exchanged water (100 ml) was added to the reaction solution, and the obtained mixture was then washed with ion exchanged water (100 ml × 3) using a separatory funnel. The obtained organic layer was dried over sodium sulfate, and it was then subjected to vacuum concentration. The obtained solid was dissolved in 100 ml of toluene, and it was then passed through silica gel. The resultant was intensively washed with toluene (300 ml), and it was then consolidated by concentration. The obtained solid was subjected to repulp washing with hexane (300 ml × 2), so as to obtain a compound M3 (16.33 g, 93%).
MS (APCI-MS: Positive) m/z: 1603.7 ([M]⁺)

### <Example 11> (Synthesis of compound M4)

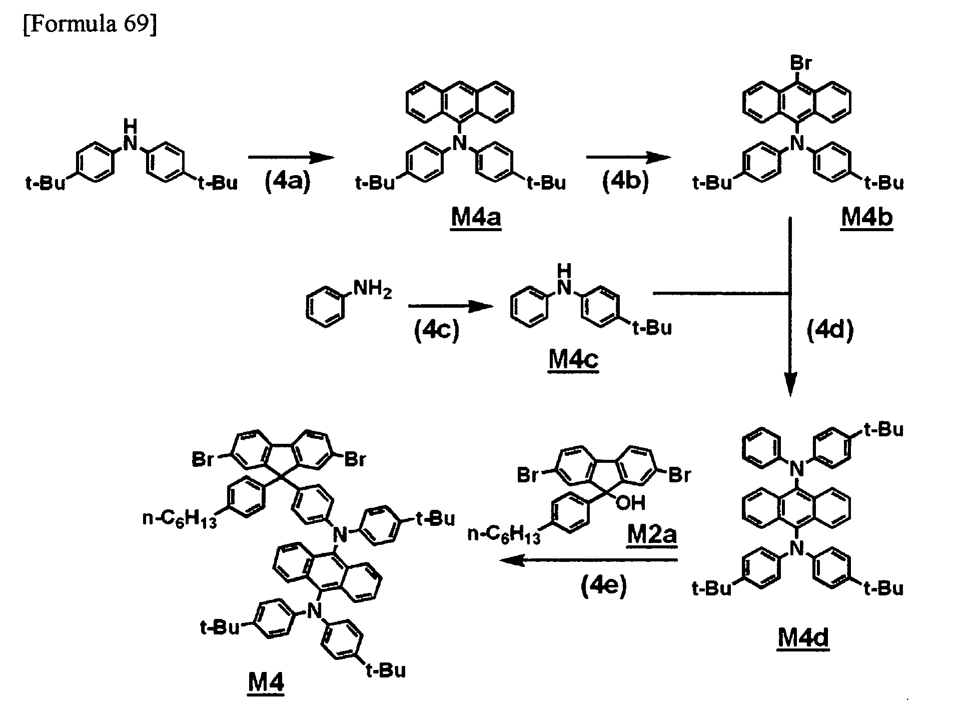

### • Step (4a)

Under an argon atmosphere, 4-tert-bromobenzene (50.0 g, 235 mmol), aniline (32.8 g, 352 mmol), tris(dibenzylideneacetone)dipalladium(0) (2.15 g, 2.3 mmol), sodium-tert-butoxide (23.7 g, 246 mmol), tri-tert-butylphosphine (1.90 g, 9.4 mmol), and toluene (255 g) were mixed in a 500-ml flask. The obtained mixture was stirred at 100°C for 10 hours. After completion of the reaction, the reaction solution was cooled to a room temperature, and it was then diluted with toluene (290 ml). Insoluble matter was removed by filtration. The filtrate was subjected to vacuum concentration, it was then supplied to a silica gel column, and it was then washed out with toluene. The obtained solution was consolidated by concentration, followed by vacuum drying, so as to obtain a solid. The obtained solid was recrystallized from hexane, so as to obtain a compound M4a (22 g). The obtained M4a was subjected to the subsequent step without performing further purification.

### • Step (4b)

Under an argon atmosphere, the compound M4a (9.79 g) was mixed with chloroform (120 ml) in a 500-ml flask, and the obtained mixture was then cooled in an ice water bath. Thereafter, trifluoroacetic acid (4 µl, 0.05 mmol) was added thereto, and N-bromosuccinimide (3.74 g, 21 mmol) dissolved in N,N-dimethylformamide (42 ml) was then added dropwise to the above solution over 1 hour. The ice water bath was removed, the obtained mixture was stirred at a room temperature for 3 hours. After completion of the reaction, the reaction solution was diluted with chloroform (100 ml), and the thus diluted solution was successively washed with a 2-weight-% sodium sulfite aqueous solution (120 ml), a 5-weight-% sodium bicarbonate aqueous solution (100 ml), and distilled water (100 ml). The obtained organic layer was dried over sodium sulfate, followed by vacuum concentration. The obtained solid was dissolved in chloroform (60 ml), and methanol (120 ml) was then slowly added to the solution. The precipitated solid was collected by filtration, and it was then washed with methanol (50 ml), followed by vacuum drying, so as to obtain a compound M4b (10.4 g). The obtained M4b was subjected to the subsequent step without further performing purification.
MS (APCI-MS: Positive) m/z: 535 ([M+H]⁺)
¹H-NMR (300 MHz, THF-d₈): δ (ppm)1.25 (s, 18H), 6.95 (d, 4H), 7.17 (d, 4H), 7.42-7.48 (m, 2H), 7.58-7.63 (m, 2H), 8.20 (d, 2H), 8.61 (d, 2H).

### • Step (4c)

Under an argon atmosphere, bis(4-tert-butylphenyl)amine (50.5 g, 179 mmol), sodium-t-butoxide (21.6 g, 224 mmol), tris(dibenzylideneacetone)dipalladium(0) (1.58 g, 1.7 mmol), a tri-t-butylphosphonium tetrafluoroborate complex (2.00 g, 6.9 mmol), and toluene (323 ml) were mixed in a 1-L flask, and the obtained mixture was then heated to 80°C. Thereafter, 9-bromoanthracene (44.4 g, 173 mmol) was slowly added to the reaction solution, while being washed out with toluene (approximately 30 ml). Thereafter, the obtained mixture was stirred at 100°C for 6 hours. After completion of the reaction, the reaction solution was cooled to a room temperature, and toluene (444 ml) and silica gel (79 g) were then added thereto. The obtained mixture was stirred, and it was then filtrated using a filter on which a thin silica gel layer was placed, so that insoluble matter could be removed. The obtained filtrate was consolidated by concentration, and it was then subjected to vacuum drying, so as to obtain a solid (81 g). Ethyl acetate (250 ml) was added to the obtained solid, and it was then dispersed in an ultrasonic bath for 30 minutes. The resultant was collected by filtration, followed by vacuum drying, so as to obtain a compound M4c (55.9 g; yield: 71%) in the form of yellow powders.

### • Step (4d)

Under an argon atmosphere, the compound M4b (8.44 g), the compound M4c (3.72 g, 16.5 mmol), sodium-t-butoxide (1.586 g, 16.5 mmol), tris(dibenzylideneacetone)dipalladium(0) (0.137 g, 0.15 mmol), a tri-t-butylphosphonium tetrafluoroborate complex (0.174 g, 0.6 mmol), and toluene (45 ml) were mixed in a 200-ml flask, and the obtained mixture was then stirred under reflux for 7 hours. After completion of the reaction, the reaction solution was cooled in an ice water bath, and a 1 N hydrochloric acid aqueous solution (30 ml) was added thereto. The obtained mixture was stirred for 30 minutes. Thereafter, toluene (100 ml) was added to the reaction solution, and an organic layer was successively washed with a saturated saline (100 ml), a 10-weight-% sodium bicarbonate aqueous solution (50 ml), and a saturated saline (50 ml × 2). The obtained organic layer was dried over magnesium sulfate, followed by vacuum concentration. The obtained solid was dissolved in toluene (60 ml), and the solution was then added dropwise to methanol (200 ml).
The precipitated solid was collected by filtration, and it was then washed with methanol (100 ml), followed by vacuum drying, so as to obtain a compound M4d (5.2 g). The obtained compound M4d was subjected to the subsequent step without further performing purification.
MS (APCI-MS: Positive) m/z: 681 ([M+H]⁺)

### • Step (4e)

Under an argon atmosphere, the compound M4d (4,48 g), the compound M2a (3.6 g, 7.2 mmol) synthesized in the same manner as described above, and dichloromethane (200 ml) were mixed in a 500-ml four-necked flask. Under a room temperature, a mixed solution of a trifluoroborane ether complex (0.95 ml, 7.7 mmol) and dichloromethane (40 ml) was added dropwise to the above mixed solution over 1 hour, and the obtained mixture was then stirred for 6 hours. After completion of the reaction, ethanol (50 ml) and ion exchanged water (50 ml) were added to the reaction solution. Using a separatory funnel, the obtained mixture was successively washed with distilled water (100 ml), a 5-weight-% sodium bicarbonate aqueous solution (100 ml), and a saturated saline (100 ml). The obtained organic layer was dried over magnesium sulfate, followed by vacuum concentration. The obtained solid was dissolved in toluene (160 ml) by heating, and when the solution was hot, it was supplied to a silica gel short column. The resultant was intensively washed with toluene (300 ml), and it was then concentrated under a reduced pressure, so that it became approximately 60 g. Thereafter, ethanol (60 ml) was slowly added to the resultant, and the precipitated solid was then collected by filtration. The solid was washed with ethanol (20 ml) and methanol (20 ml), followed by vacuum drying, so as to obtain a compound M4 (4.75g; HPLC area percent > 99% (UV 254 nm)).
MS (APCI-MS: Positive) m/z: 737 ([M+H]⁺)

### <Synthesis Example 1> (Synthesis of compound CM3)

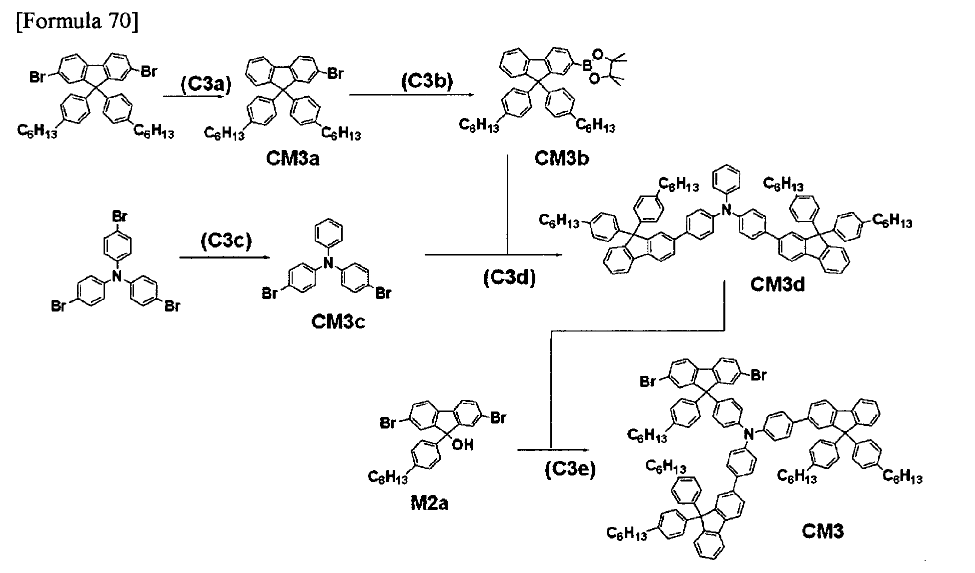

### • Step (C3a)

Under an argon atmosphere, 2,7-dibromo-9,9-bis(4-hexylphenyl)fluorene (50.27 g, 78 mmol) was mixed with THF (500 ml) in a 300-ml four-necked flask, and the obtained mixture was then cooled to -78°C. Subsequently, a 1.6 M n-butyllithium hexane solution (51 ml, 82 mmol) was added dropwise to the reaction solution over 2 hours, and while maintaining the mixture at -78°C, it was further stirred for 2 hours. Thereafter, the reaction solution was quenched by adding 100 ml of water at once. After the temperature of the reaction solution had been increased to a room temperature, an organic layer was dried over sodium sulfate, followed by vacuum concentration. Toluene (500 ml) was added to the obtained oil, and it was then washed with water (100 ml × 3). An organic layer was dried over sodium sulfate, followed by vacuum concentration. The obtained oil was purified using an intermediate pressure fractionation column (silica gel, hexane : toluene = 10 : 1), so as to obtain a compound CM3a (35.1 g, 79%).

It is to be noted that the 2,7-dibromo-9,9-bis(4-hexylphenyl)fluorene was synthesized by the method described in W02002/092723.
¹H-NMR (270 MHz, CDCl₃): δ=0.87 (t, 6H), 1.25-1.40 (m, 24H), 1.55 (m, 4H), 2.54 (t, 4H), 7.02 (d, 4H), 7.06 (d, 4H), 7.24-7.39 (m, 3H), 7.45 (dd, 1H), 7.52 (s, 1H), 7.59 (d, 1H), 7.69 (d, 1H) ppm.
¹³C-NMR (270 MHz, CDCl₃): δ=14.4, 22.9, 29.4, 31.6, 32.0, 35.8, 65.3, 120.4, 121.6, 121.7, 126.5, 127.7, 128.2, 128.3, 128.6, 129.7, 130.8, 139.2, 139.4, 141.8, 142.7, 151.7, 154.0 ppm.

### • Step (C3b)

Under an argon atmosphere, the compound CM3a (12.16 g, 22 mmol), pinacolate diborane (5.35 g, 24 mmol), potassium acetate (6.33 g, 66 mmol), dioxane (92 ml), diphenylphosphinoferrocene palladium dichloride (0.53 g, 0.66 mmol), and diphenylphosphino ferrocene (0.36 g, 0.66 mmol) were mixed in a 300-ml four-necked flask, and the obtained mixture was then heated to 110°C for 15 hours. After completion of the reaction, ion exchanged water (100 ml) was added to the reaction solution to quench it. A water layer was removed using a separatory funnel, and the residue was then washed with water (100 ml × 3). An organic layer was dried over sodium sulfate, followed by vacuum concentration. The obtained oil was purified using an intermediate pressure fractionation column (silica gel, hexane : toluene = 1 : 1), so as to obtain a compound CM3b (10.5 g, 80%).
¹H-NMR (270 MHz, CDCl₃): δ=0.87 (t, 6H), 1.25-1.40 (m, 24H), 1.56 (m, 4H), 2.53 (t, 4H), 7.00 (d, 4H), 7.10 (d, 4H)7.22-7.39 (m, 4H), 7.72-7.85 (m, 3H) ppm.
¹³C-NMR (270 MHz, CDCl₃): δ=14.4, 22.9, 25.2, 29.4, 31.6, 32.0, 35.8, 65.2, 83.9, 119.6, 120.8, 126.6, 127.5, 128.3, 128.4, 132.6, 134.5, 140.1, 141.3, 143.3, 143.4, 151.2, 152.6 ppm.

### • Step (C3c)

Under an argon atmosphere, tris-(4-bromophenyl)amine (73.75 g, 153 mmol) was mixed with anhydrous THF (980 ml) in a 2-L four-necked flask, and the obtained mixture was then cooled to -78°C. To the reaction solution, 1.6 M butyllithium (100 ml) was added dropwise over 1 hour. After completion of the dropping operation, the reaction solution was quenched by adding 100 ml of ion exchanged water at once. Toluene (400 ml) was added to the reaction solution, and a water layer was then removed using a separatory funnel. The residue was concentrated under a reduced pressure until an organic layer became 400 ml. The resultant was washed with ion exchanged water (300 ml × 3), and it was then dried over sodium sulfate, followed by vacuum drying. The obtained oil was purified using an intermediate pressure fractionation column (silica gel, hexane : toluene = 1 : 1) 3 times, so as to obtain a compound CM3c (34 g, 55%). ¹H-NMR (270 MHz, CDCl₃): δ=6.93 (d, 4H), 7.05 (m, 3H), 7.25 (m, 2H), 7.33 (d, 4H) ppm. ¹³C-NMR (270 MHz, CDCl₃): δ=115.7, 124.0, 124.9, 125.9, 129.8, 132.6, 146.8, 147.2 ppm.

### • Step (C3d)

Under an argon atmosphere, the compound CM3b (13.82 g, 23 mmol), the compound CM3c (4.43 g, 11 mmol), potassium hydroxide (9.26 g, 170 mmol), tetrabutylammonium bromide (1.77 g, 6 mmol), toluene (150 ml), ion exchanged water (70 ml), and tetrakis(triphenylphosphine)palladium (380 mg, 0.33 mmol) were mixed in a 300-ml four-necked flask, and the obtained mixture was then heated to 80°C for 2 hours. After completion of the reaction, a water layer was removed using a separatory funnel, and the residue was then washed with ion exchanged water (100 ml × 3). An organic layer was dried over sodium sulfate, followed by vacuum concentration. The obtained oil was purified using an intermediate pressure fractionation column (silica gel, hexane : toluene = 8 : 1) 3 times, so as to obtain a compound CM3d (10.7 g, 80%).
¹H-NMR (270 MHz, CDCl₃): δ=0.85 (t, 12H), 1.28 (m, 24H), 1.54 (m, 8H), 2.53 (t, 8H), 7.01 (d, 8H), 7.07-7.19 (m, 14H), 7.20-7.50 (m, 13H), 7.51-7.63 (m, 4H), 7.69-7.62 (m, 4H) ppm.
¹³C-NMR (270 MHz, CDCl₃): δ=14.4, 22.9, 29.5, 31.8, 32.0, 35.9, 65.3, 120.4, 120.5, 123.5, 124.4, 124.7, 124.9, 126.3, 126.6, 127.7, 127.8, 128.2, 128.3, 128.5, 129.6, 135.8, 139.3, 140.1, 140.3, 141.5, 143.5, 147.2, 147.8, 152.2, 152.6 ppm.

### • Step (C3e)

Under an argon atmosphere, the compound CM3d (9.7 g, 8.0 mmol), the compound CM2a (4.8 g, 10 mmol) synthesized in the same manner as described above, and dichloromethane (150 ml) were mixed in a 500-ml four-necked flask. Under a room temperature, a mixed solution of a trifluoroborane ether complex (1.2 ml, 10 mmol) and dichloromethane (50 ml) was added dropwise to the above mixed solution over 1 hour, and the obtained mixture was then stirred for 2 hours. After completion of the reaction, ion exchanged water (100 ml) was added to the reaction solution, and using a separatory funnel, the obtained mixture was then washed with ion exchanged water (100 ml × 3). The obtained organic layer was dried over sodium sulfate, followed by vacuum concentration. The obtained oil was purified using an intermediate pressure fractionation column (silica gel, hexane : toluene = 4 : 1) 3 times, so as to obtain a compound CM3 (9.11 g, 67%).
MS (APCI-MS: Positive) m/z: 1697.3 ([M+H]⁺)
¹H-NMR (270 MHz, CDCl₃): δ=0.86 (t, 15H), 1.12-1.43 (m, 30H), 1.44-1.67 (m, 10H), 2.52 (t, 10H), 6.84-7.20 (m, 30H), 7.22-7.65 (m, 26H), 7.76 (dd, 4H) ppm.
¹³C-NMR (270 MHz, CDCl₃): δ=14.4, 22.9, 29.4, 31.6, 32.0, 35.8, 65.3, 120.4, 120.6, 121.8, 122.0, 123.8, 124.7, 126.5, 127.6, 127.8, 128.1, 128.2, 128.4, 128.5, 128.8, 129.1, 129.7, 131.1, 136.1, 138.3, 139.3, 140.1, 140.3, 141.5, 141.8, 142.2, 143.5, 146.9, 152.2, 152.6, 153.7 ppm

### <Synthesis Example 2> (Synthesis of compound CM4)

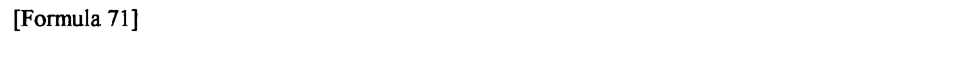

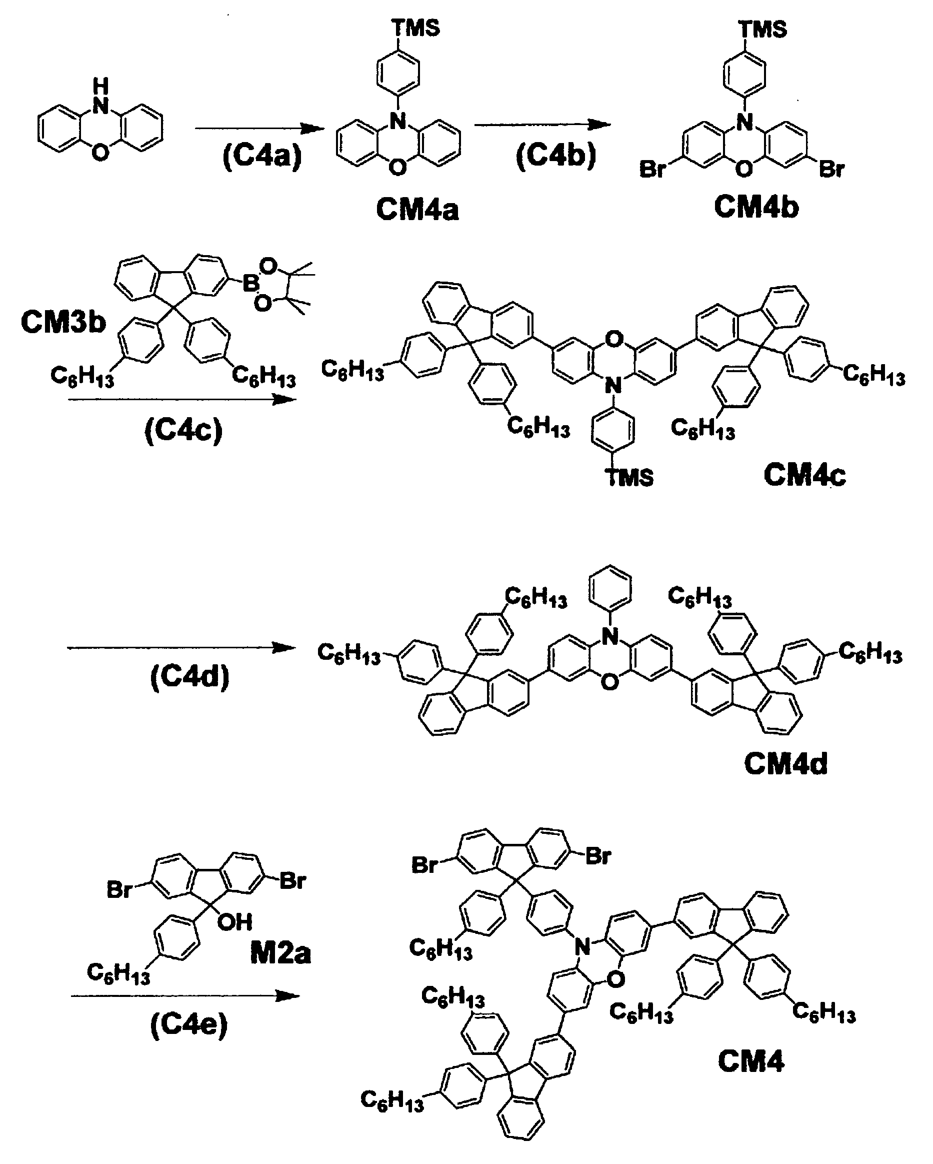

### • Step (C4a)

Under an argon atmosphere, phenoxazine (19.79 g, 108 mmol), 4-trimethylsilylbromobenzene (24.75 g, 108 mmol), sodium-t-butoxide (15.57 g, 162 mmol), palladium acetate (121 mg, 0.54 mmol), tris(o-methylphenyl)phosphine (329 mg, 1.1 mmol), and toluene (170 ml) were mixed in a 500-ml four-necked flask, and the obtained mixture was then refluxed for 6 hours. After completion of the reaction, the reaction solution was cooled, and it was then passed through Celite and alumina. The obtained toluene solution was concentrated to a volume of 100 ml, and methanol (200 ml) was then added thereto. The obtained mixture was left at rest for recrystallization. The obtained solid was collected by filtration, and it was then dried, so as to obtain a compound CM4a (26.3 g, 73%).
¹H-NMR (270 MHz, THF-d₈): δ=-0.46 (s, 9H), 6.01 (d, 2H), 6.63-6.77 (m, 6H), 7.46 (d, 2H), 7.72 (d, 2H) ppm.
¹³C-NMR (270 MHz, THF-d₈): δ=-1.2, 114.0, 115.9, 121.9, 123.8, 130.6, 135.2, 136.7, 140.5, 141.7, 144.8 ppm.

### • Step (C4b)

Under an argon atmosphere, the compound CM4a (23.2 g, 70 mmol) was mixed with chloroform (300 ml) in a 500-ml four-necked flask, and the obtained mixture was then cooled to 0°C. A mixed solution ofNBS (24.9 g, 140 mmol) and DMF (100 ml) was added dropwise to the reaction solution over 1 hour. After completion of the dropping operation, the temperature of the reaction solution was increased to a room temperature. Further, NBS was added to the reaction solution by 1 gram, until the reaction completely progressed. After completion of the reaction, the reaction solution was poured into methanol (1.5 L). The obtained solid was collected by filtration, and it was then dried. Toluene (500 ml) was added to the obtained solid, and the solution was then refluxed. The resultant was hot filtered, so as to obtain a solid A. In addition, the toluene solution was concentrated, and it was then recrystallized from toluene-isopropanol. Thus, a solid B was collected by filtration. The solid A was mixed with the solid B, and the mixture was then recrystallized from toluene-isopropanol 3 times, so as to obtain a product of interest CM4b (25.1 g, 64%).
¹H-NMR (270 MHz, THF-d₈): δ=-0.50 (s, 9H), 5.96 (d, 2H), 6.89 (dd, 2H), 7.02 (s, 2H), 7.49 (d, 2H), 7.99 (d, 2H) ppm.

### • Step (C4c)

Under an argon atmosphere, the compound CM3b (12.1 g, 20 mmol) synthesized in the same manner as described above, the compound CM4b (4.6 g, 9.4 mmol), Aliquat 336 (1.2 g, 3 mmol), and toluene (150 ml) were mixed in a 500-ml four-necked flask, and argon was then bubbled into the obtained mixture for 1 hour. Thereafter, palladium acetate (21 mg, 0.1 mmol) and tris(o-methoxyphenyl)phosphine (33 mg, 0.1 mmol) were mixed into the reaction solution, and the obtained mixture was then heated to 105°C. A 2 M sodium carbonate aqueous solution (15 ml) was added dropwise to the reaction solution over 1 hour, and the reaction was then carried out for 3 hours. After completion of the reaction, liquid separation was performed. An organic layer was washed with ion exchanged water (100 ml × 3), and it was then dried over sodium sulfate. The resultant was purified by passing through alumina, followed by concentration drying, so as to obtain a compound CM4c (11.9 g, 99%). The obtained compound was not subjected to purification, and it was used as a crude product in the subsequent reaction.

### • Step (C4d)

Under an argon atmosphere, the compound CM4c (11.9 g, 9 mmol), trifluoroacetic acid (22 g, 180 mmol), and toluene (100 ml) were mixed in a 300-ml four-necked flask, and the obtained mixture was then heated to 80°C for 3 hours. After completion of the reaction, liquid separation was performed. An organic layer was washed with ion exchanged water (100 ml × 3) and a sodium bicarbonate saturated aqueous solution (100 ml × 4), and it was then dried over sodium sulfate. The resultant was passed through alumina, and the toluene solution was then consolidated by concentration. The obtained solid was purified by silica gel column chromatography (toluene : hexane = 1 : 1), and the obtained solution was then concentrated. The resultant was reprecipitated from toluene-hexane, so as to obtain a compound CM4d (9.41 g, 83%).
MS (APCI-MS: Positive) m/z: 1228.6 ([M+H]⁺).
¹H-NMR (270 MHz, THF-d₈): δ=0.99 (t, 12H), 1.32-1.56 (m, 24H), 1.63-1.73 (m, 8H), 2.65 (t, 8H), 6.06 (d, 2H), 6.94 (dd, 2H), 7.09 (d, 2H), 7.13 (d, 8H), 7.23 (d, 8H), 7.33 (dd, 2H), 7.43 (dd, 2H), 7.49-7.52 (m, 4H), 7.60-7.67 (m, 3H), 7.68 (s, 2H), 7.76 (dd, 2H), 7.89-7.95 (m, 4H) ppm.

### • Step (C4e)

Under an argon atmosphere, the compound CM4d (8.6 g, 7.0 mmol), the compound M2a (3.6 g, 7.1 mmol) synthesized in the same manner as described above, and dichloromethane (130 ml) were mixed in a 500-ml four-necked flask. Under a room temperature, a mixed solution of a trifluoroborane ether complex (1 ml, 8 mmol) and dichloromethane (10 ml) was added dropwise to the above mixed solution over 1 hour. Thereafter, the reaction solution was stirred at 30°C for 100 hours. After completion of the reaction, ion exchanged water (100 ml) was added to the reaction solution, and the obtained mixture was then washed with ion exchanged water (100 ml × 3) using a separatory funnel.
The obtained organic layer was dried over sodium sulfate, followed by vacuum concentration.
The obtained oil was purified using an intermediate pressure fractionation column (silica gel, hexane : toluene = 4 : 1), so as to obtain a compound CM4 (2.2 g, 18%).
MS (APCI-MS: Positive) m/z: 1711.6 ([M+H]⁺).

### <Example 12> (Synthesis of polymer compound P5)

Under an argon gas atmosphere, 2,7-bis(1,3,2-dioxaborolane-2-yl)-9,9-dioctylfluorene (1.0589 g, 1.996 mmol), 2,7-dibromo-9,9-dioctylfluorene (0.8775 g, 1.600 mmol), the compound M3 (0.6416 g, 0.400 mmol), and trioctylmethylammonium chloride (manufactured by Aldrich; product name: Aliquat336) (0.26 g, 0.64 mmol) were dissolved in toluene (32 ml). Argon gas was bubbled into the solution, and the temperature was then increased to 80°C. Thereafter, dichlorobistriphenylphosphine palladium (4.2 mg, 6.0 µmol) and a 17.5-weight% sodium carbonate aqueous solution (8.2 ml, 9.0 mmol) were added to the reaction solution, and the obtained mixture was then reacted under reflux for 20 hours. Thereafter, the reaction solution was once cooled, and phenylboric acid (0.02 g, 0.2 mmol) was then added thereto. The mixture was further reacted under reflux for 2 hours. Thereafter, toluene (20 ml) was added to the reaction solution for dilution, and a water layer was then removed. A 9-weight-% sodium N,N-diethyldithiocarbamate aqueous solution (12 ml) was added to the residue, and the obtained mixture was then stirred at 90°C for 2 hours. Thereafter, an organic layer was successively washed with ion exchanged water (26 ml) twice, then with a 3-weight-% acetic acid aqueous solution (26 ml) twice, and then with ion exchanged water (26 ml) twice. Subsequently, the resultant was added dropwise to methanol (300 ml), and the obtained mixture was then stirred for 30 minutes. Thereafter, the precipitated polymer was collected by filtration, and it was then washed with methanol (60 ml), followed by vacuum drying, so as to obtain a crude polymer.

This crude polymer was dissolved in toluene (60 ml), and it was then supplied to a column filled with alumina (13 g) and silica gel (31 g). Thereafter, toluene (50 ml) was also supplied. The obtained solution was added dropwise to methanol (300 ml), and the obtained mixture was stirred for 30 minutes. Thereafter, the precipitated polymer was collected by filtration, and it was then washed with methanol (50 ml), followed by vacuum drying, so as to obtain a polymer compound P5 (1.8 g; yield: 92%) as a polymer. The polystyrene equivalent number average molecular weight (Mn) of the polymer compound P5 was 1.9 × 10⁵, and the polystyrene equivalent weight average molecular weight (Mw) thereof was 6.8 × 10⁵. The glass transition temperature was 99°C, and the fluorescence peak wavelengths of a thin film were 422 nm and 446 nm.

From the raw materials used, the polymer compound P5 was assumed to comprise the following repeating units at the following ratio (molar ratio).

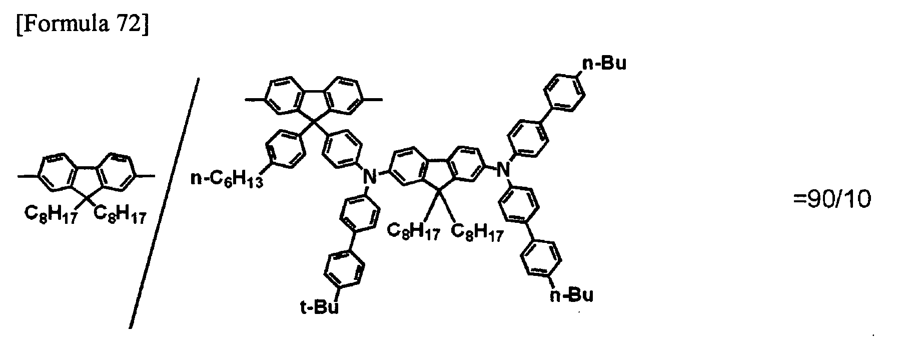

### <Example 13> (Synthesis of polymer compound P6)

Under an argon gas atmosphere, 2,7-bis(1,3,2-dioxaborolane-2-yl)-9,9-dioctylfluorene (0.7836 g, 1.478 mmol), 2,7-dibromo-9,9-dioctylfluorene (0.7733 g, 1.410 mmol), the compound M4 (0.1047 g, 0.090 mmol), and trioctylmethylammonium chloride (manufactured by Aldrich; product name: Aliquat336) (0.19 g, 0.48 mmol) were dissolved in toluene (15 ml). Argon gas was bubbled into the solution, and the temperature was then increased to 80°C. Thereafter, dichlorobistriphenylphosphine palladium (1.1 mg, 1.5 µmol) and a 17.5-weight-% sodium carbonate aqueous solution (4.1 ml, 6.8 mmol) were added to the reaction solution, and the obtained mixture was then reacted under reflux for 20 hours. Thereafter, the reaction solution was once cooled, and phenylboric acid (0.02 g, 0.2 mmol) was then added thereto. The mixture was further reacted under reflux for 2 hours. Thereafter, toluene (15 ml) was added to the reaction solution for dilution, and a water layer was then removed. A 9-weight-% sodium N,N-diethyldithiocarbamate aqueous solution (9 ml) was added to the residue, and the obtained mixture was then stirred at 90°C for 2 hours. Thereafter, an organic layer was successively washed with ion exchanged water (20 ml) twice, then with a 3-weight-% acetic acid aqueous solution (20 ml) twice, and then with ion exchanged water (20 ml) twice. Subsequently, the resultant was added dropwise to methanol (250 ml), and the obtained mixture was then stirred for 30 minutes. Thereafter, the precipitated polymer was collected by filtration, and it was then washed with methanol (50 ml), followed by vacuum drying, so as to obtain a crude polymer.

This crude polymer was dissolved in toluene (50 ml), and it was then supplied to a column filled with alumina (10 g) and silica gel (23 g). Thereafter, toluene (70 ml) was also supplied. The obtained solution was added dropwise to methanol (250 ml), and the obtained mixture was then stirred for 30 minutes. Thereafter, the precipitated polymer was collected by filtration, and it was then washed with methanol (50 ml), followed by vacuum drying, so as to obtain a polymer compound P6 (1.07 g; yield: 89%) as a polymer. The polystyrene equivalent number average molecular weight (Mn) of the polymer compound P6 was 1.5 × 10⁵, and the polystyrene equivalent weight average molecular weight (Mw) thereof was 3.6 × 10⁵. The glass transition temperature was 128°C, and the fluorescence peak wavelength of a thin film was 515 nm.

From the raw materials used, the polymer compound P6 was assumed to comprise the following repeating units at the following ratio (molar ratio).

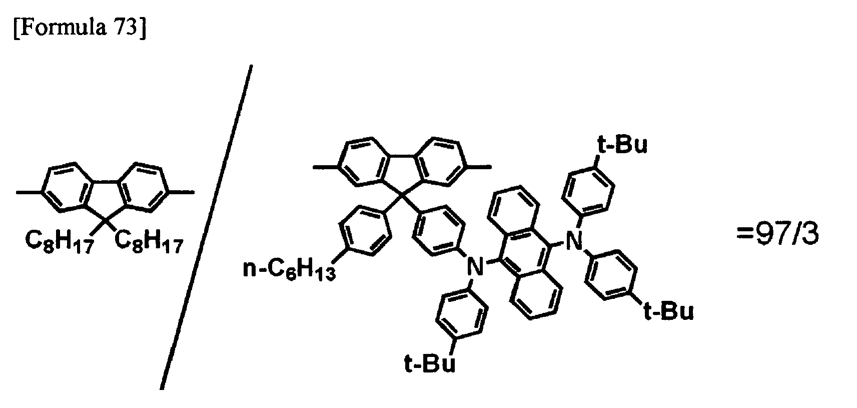

### <Example 14> (Synthesis of polymer compound P7)

Under an argon gas atmosphere, 2,7-bis(1,3,2-dioxaborolane-2-yl)-9,9-dioctylfluorene (1.0675 g, 2.012 mmol), 2,7-dibromo-9,9-dioctylfluorene (0.8873 g, 1.618 mmol), the compound M1 (0.4655 g, 0.324 mmol), 4,4'-dibromo-4"-sec-butyltriphenylamine (0.0371 g, 0.081 mmol), and trioctylmethylammonium chloride (manufactured by Aldrich; product name: Aliquat336) (0.26 g, 0.65 mmol) were dissolved in toluene (20 ml). Argon gas was bubbled into the solution, and the temperature was then increased to 80°C. Thereafter, dichlorobistriphenylphosphine palladium (2.8 mg, 4.0 µmol) and a 17.5-weight% sodium carbonate aqueous solution (5.5 ml, 9.1 mmol) were added to the reaction solution, and the obtained mixture was then reacted under reflux for 7 hours. Thereafter, the reaction solution was once cooled, and phenylboric acid (0.02 g, 0.2 mmol) and dichlorobistriphenylphosphine palladium (1.4 mg, 2.0 µmol) were then added thereto. The mixture was further reacted under reflux for 2 hours. Thereafter, toluene (20 ml) was added to the reaction solution for dilution, and a water layer was then removed. A 9-weight-% sodium N,N-diethyldithiocarbamate aqueous solution (12 ml) was added to the residue, and the obtained mixture was then stirred at 90°C for 2 hours. Thereafter, an organic layer was successively washed with ion exchanged water (26 ml) twice, then with a 3-weight-% acetic acid aqueous solution (26 ml) twice, and then with ion exchanged water (26 ml) twice. Subsequently, the resultant was added dropwise to methanol (300 ml), and the obtained mixture was then stirred for 30 minutes. Thereafter, the precipitated polymer was collected by filtration, and it was then washed with methanol (60 ml), followed by vacuum drying, so as to obtain a crude polymer.

This crude polymer was dissolved in toluene (80 ml), and it was then supplied to a column filled with alumina (14 g) and silica gel (31 g). Thereafter, toluene (50 ml) was also supplied. The obtained solution was added dropwise to methanol (300 ml), and the obtained mixture was then stirred for 30 minutes. Thereafter, the precipitated polymer was collected by filtration, and it was then washed with methanol (60 ml), followed by vacuum drying, so as to obtain a polymer compound P7 (1.14 g; yield: 62%) as a polymer. The polystyrene equivalent number average molecular weight (Mn) of the polymer compound P7 was 1.1 × 10⁵, and the polystyrene equivalent weight average molecular weight (Mw) thereof was 3.1 × 10⁵. The glass transition temperature was 107°C, and the fluorescence peak wavelength of a thin film was 425 nm.

From the raw materials used, the polymer compound P7 was assumed to comprise the following repeating units at the following ratio (molar ratio).

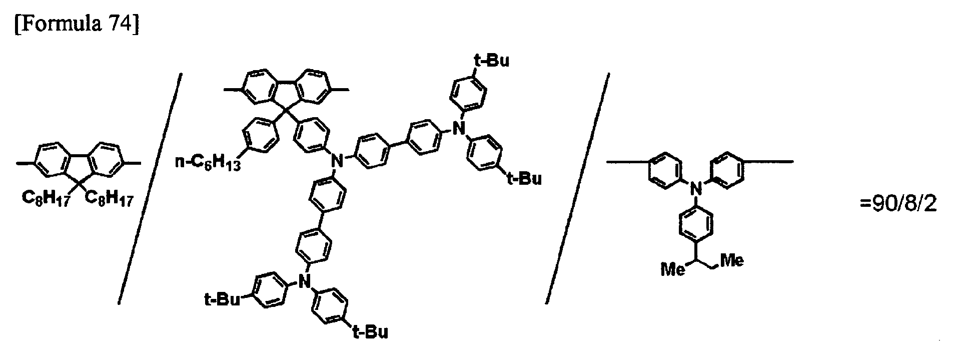

### <Example 15> (Synthesis of polymer compound P8)

Under an argon gas atmosphere, 2,7-bis(1,3,2-dioxaborolane-2-yl)-9,9-dioctylfluorene (1.0675 g, 2.012 mmol), 2,7-dibromo-9,9-dioctylfluorene (0.8873 g, 1.618 mmol), the compound M1 (0.2909 g, 0.202 mmol), the compound M3 (0.3244 g, 0.202 mmol), and trioctylmethylammonium chloride (manufactured by Aldrich; product name: Aliquat336) (0.26 g, 0.65 mmol) were dissolved in toluene (20 ml). Argon gas was bubbled into the solution, and the temperature was then increased to 80°C. Thereafter, dichlorobistriphenylphosphine palladium (2.8 mg, 4.0 µmol) and a 17.5-weight-% sodium carbonate aqueous solution (5.5 ml, 9.1 mmol) were added to the reaction solution, and the obtained mixture was then reacted under reflux for 7 hours. Thereafter, the reaction solution was once cooled, and phenylboric acid (0.02 g, 0.2 mmol) and dichlorobistriphenylphosphine palladium (1.4 mg, 2.0 µmol) were then added thereto. The mixture was further reacted under reflux for 2 hours. Thereafter, toluene (20 ml) was added to the reaction solution for dilution, and a water layer was then removed. A 9-weight-% sodium N,N-diethyldithiocarbamate aqueous solution (12 ml) was added to the residue, and the obtained mixture was then stirred at 90°C for 2 hours. Thereafter, an organic layer was successively washed with ion exchanged water (26 ml) twice, then with a 3-weight-% acetic acid aqueous solution (26 ml) twice, and then with ion exchanged water (26 ml) twice. Subsequently, the resultant was added dropwise to methanol (300 ml), and the obtained mixture was then stirred for 30 minutes. Thereafter, the precipitated polymer was collected by filtration, and it was then washed with methanol (60 ml), followed by vacuum drying, so as to obtain a crude polymer.

This crude polymer was dissolved in toluene (80 ml), and it was then supplied to a column filled with alumina (14 g) and silica gel (31 g). Thereafter, toluene (50 ml) was also supplied. The obtained solution was added dropwise to methanol (300 ml), and the obtained mixture was then stirred for 30 minutes. Thereafter, the precipitated polymer was collected by filtration, and it was then washed with methanol (60 ml), followed by vacuum drying, so as to obtain a polymer compound P8 (1.27 g; yield: 65%) as a polymer. The polystyrene equivalent number average molecular weight (Mn) of the polymer compound P8 was 1.3 × 10⁵, and the polystyrene equivalent weight average molecular weight (Mw) thereof was 4.3 × 10⁵. The glass transition temperature was 105°C, and the fluorescence peak wavelengths of a thin film were 422 nm and 446 nm.

From the raw materials used, the polymer compound P8 was assumed to comprise the following repeating units at the following ratio (molar ratio).

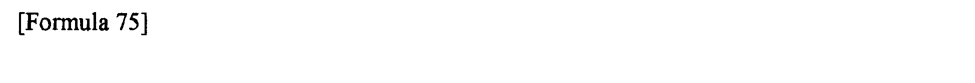

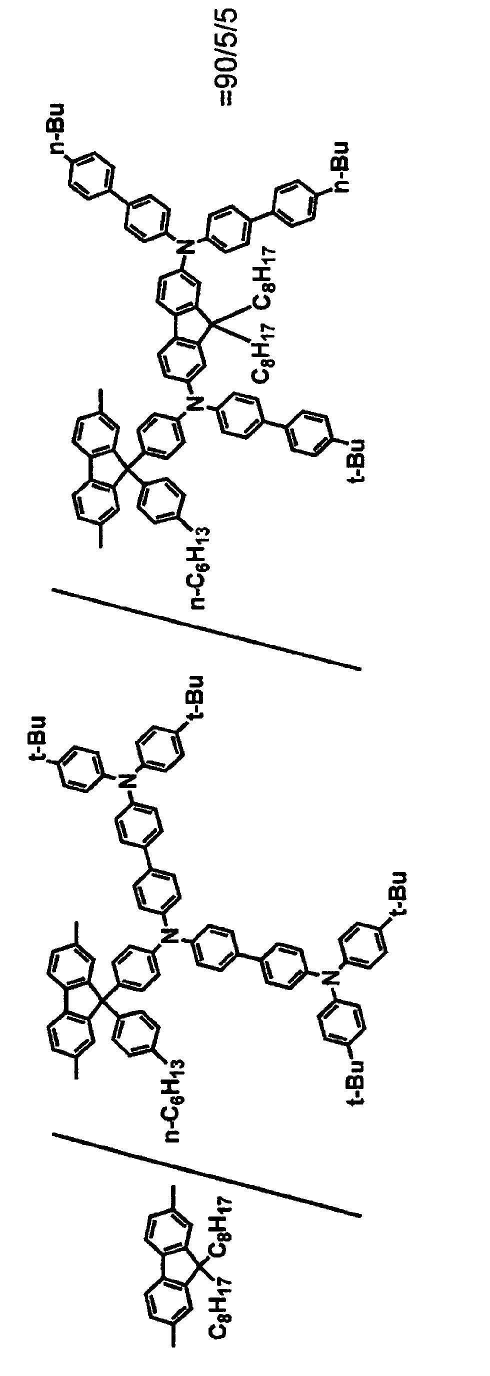

### <Example 16> (Synthesis of polymer compound P9)

Under an argon gas atmosphere, 2,7-bis(1,3,2-dioxaborolane-2-yl)-9,9-dioctylfluorene (1.0675 g, 2.012 mmol), 2,7-dibromo-9,9-dioctylfluorene (0.8873 g, 1.618 mmol), the compound M1 (0.3782 g, 0.263 mmol), the compound M3 (0.1622 g, 0.101 mmol), N-(4-n-butylphenyl)-3,7-dibromophenoxazine (0.0191 g, 0.040 mmol), and trioctylmethylammonium chloride (manufactured by Aldrich; product name: Aliquat336) (0.26 g, 0.65 mmol) were dissolved in toluene (20 ml). Argon gas was bubbled into the solution, and the temperature was then increased to 80°C. Thereafter, dichlorobistriphenylphosphine palladium (2.8 mg, 4.0 µmol) and a 17.5-weight% sodium carbonate aqueous solution (5.5 ml, 9.1 mmol) were added to the reaction solution, and the obtained mixture was then reacted under reflux for 7 hours. Thereafter, the reaction solution was once cooled, and phenylboric acid (0.02 g, 0.2 mmol) and dichlorobistriphenylphosphine palladium (1.4 mg, 2.0 µmol) were then added thereto. The mixture was further reacted under reflux for 2 hours. Thereafter, toluene (20 ml) was added to the reaction solution for dilution, and a water layer was then removed. A 9-weight-% sodium N,N-diethyldithiocarbamate aqueous solution (12 ml) was added to the residue, and the obtained mixture was then stirred at 90°C for 2 hours. Thereafter, an organic layer was successively washed with ion exchanged water (26 ml) twice, then with a 3-weight-% acetic acid aqueous solution (26 ml) twice, and then with ion exchanged water (26 ml) twice. Subsequently, the resultant was added dropwise to methanol (300 ml), and the obtained mixture was then stirred for 30 minutes. Thereafter, the precipitated polymer was collected by filtration, and it was then washed with methanol (60 ml), followed by vacuum drying, so as to obtain a crude polymer.

This crude polymer was dissolved in toluene (80 ml), and it was then supplied to a column filled with alumina (14 g) and silica gel (31 g). Thereafter, toluene (50 ml) was also supplied. The obtained solution was added dropwise to methanol (300 ml), and the obtained mixture was then stirred for 30 minutes. Thereafter, the precipitated polymer was collected by filtration, and it was then washed with methanol (60 ml), followed by vacuum drying, so as to obtain a polymer compound P9 (1.38 g; yield: 72%) as a polymer. The polystyrene equivalent number average molecular weight (Mn) of the polymer compound P9 was 1.2 × 10⁵, and the polystyrene equivalent weight average molecular weight (Mw) thereof was 3.3 × 10⁵. The glass transition temperature was 109°C, and the fluorescence peak wavelengths of a thin film were 446 nm and 462 nm.

From the raw materials used, the polymer compound P9 was assumed to comprise the following repeating units at the following ratio (molar ratio).

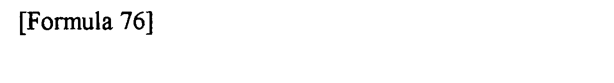

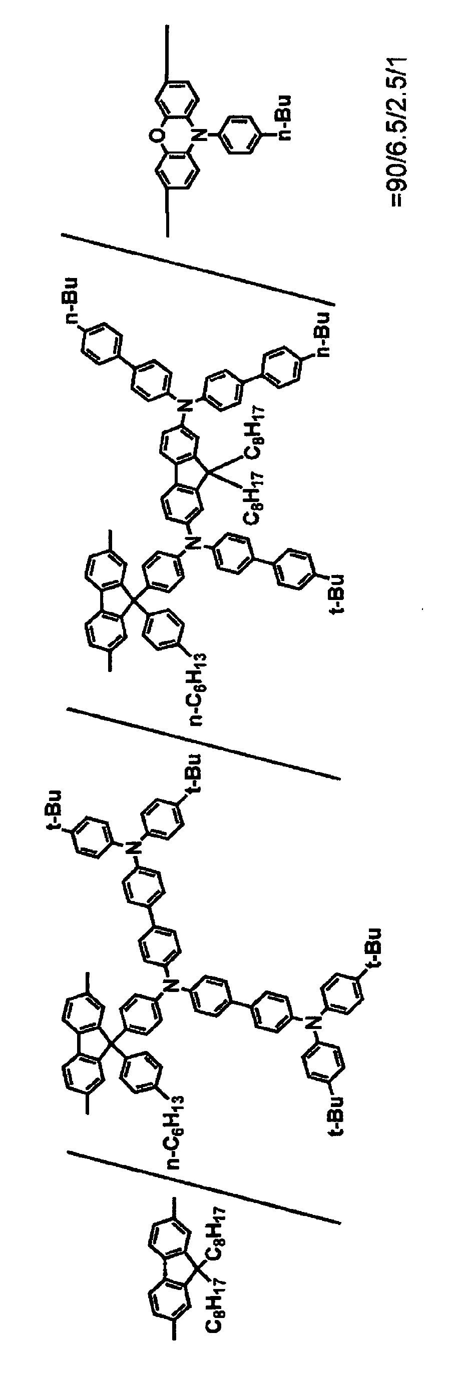

### <Example 17> (Synthesis of polymer compound P10)

Under an argon gas atmosphere, 5,9-bis(4,4,5,5-tetramethyl-1,3,2-dioxaborolane-2-yl)-7,7-dioctylbenzo[c]fluorene (0.5541 g, 0.800 mmol), 5,9-dibromo-7,7-dioctylbenzo[c]fluorene (0.3830 g, 0.640 mmol), the compound CM3 (0.2308 g, 0.136 mmol), and the compound M3 (0.0385 g, 0.024 mmol) were dissolved in toluene (18 ml). Argon gas was bubbled into the solution, and the temperature was then increased to 80°C. Thereafter, dichlorobistriphenylphosphine palladium (0.6 mg, 0.8 µmol) and a 20-weight-% tetraethylammonium hydroxide aqueous solution (2.7 ml, 3.8 mmol) were added to the reaction solution, and the obtained mixture was then reacted under reflux for 20 hours. Thereafter, the reaction solution was once cooled, and phenylboric acid (0.10 g, 0.8 mmol) and dichlorobistriphenylphosphine palladium (0.6 mg, 0.8 µmol) were then added thereto. The mixture was further reacted under reflux for 2 hours. Thereafter, the reaction solution was once cooled again, and bromobenzene (0.18 g, 1.2 mmol) and dichlorobistriphenylphosphine palladium (0.6 mg, 0.8 µmol) were then added thereto. The mixture was further reacted under reflux for 2 hours. Thereafter, toluene (20 ml) was added to the reaction solution for dilution, and a water layer was then removed. A 9-weight-% sodium N,N-diethyldithiocarbamate aqueous solution (5 ml) was added to the residue, and the obtained mixture was then stirred at 90°C for 2 hours. Thereafter, an organic layer was successively washed with ion exchanged water (10 ml) twice, then with a 3-weight-% acetic acid aqueous solution (10 ml) twice, and then with ion exchanged water (10 ml) twice. Subsequently, the resultant was added dropwise to methanol (125 ml), and the obtained mixture was then stirred for 30 minutes. Thereafter, the precipitated polymer was collected by filtration, and it was then washed with methanol (25 ml), followed by vacuum drying, so as to obtain a crude polymer.

This crude polymer was dissolved in toluene (25 ml), and it was then supplied to a column filled with alumina (5 g) and silica gel (12 g). Thereafter, toluene (20 ml) was also supplied. The obtained solution was added dropwise to methanol (125 ml), and the obtained mixture was then stirred for 30 minutes. Thereafter, the precipitated polymer was collected by filtration, and it was then washed with methanol (25 ml), followed by vacuum drying, so as to obtain a polymer compound P10 (0.61 g; yield: 70%) as a polymer. The polystyrene equivalent number average molecular weight (Mn) of the polymer compound P10 was 7.7 × 10⁴, and the polystyrene equivalent weight average molecular weight (Mw) thereof was 1.5 × 10⁵. The glass transition temperature was 133°C, and the fluorescence peak wavelength of a thin film was 450 nm.

From the raw materials used, the polymer compound P10 was assumed to comprise the following repeating units at the following ratio (molar ratio).

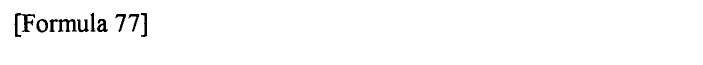

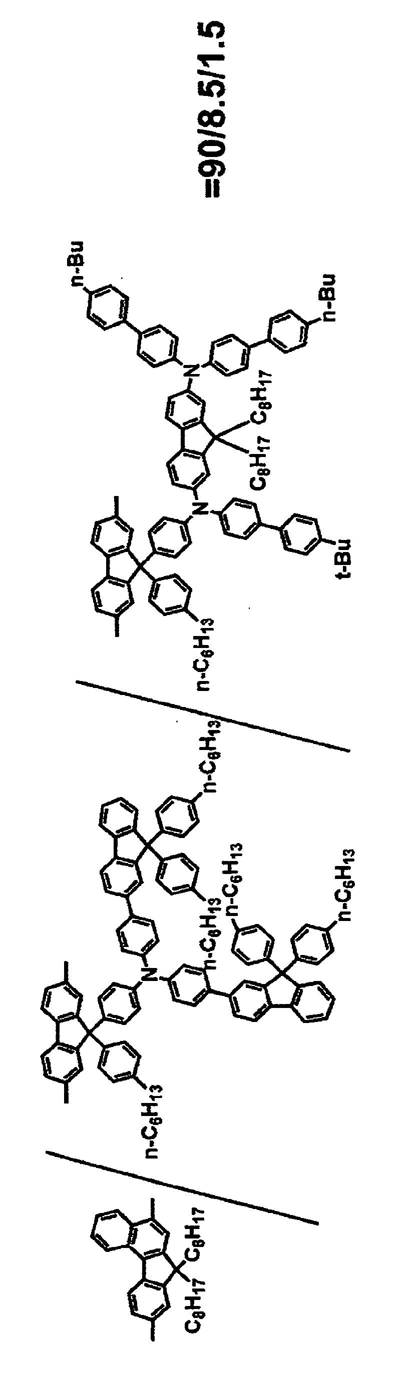

### <Example 18> (Synthesis of polymer compound P11)

Under an argon gas atmosphere, 2,7-bis(1,3,2-dioxaborolane-2-yl)-9,9-dioctylfluorene (0.4223 g, 0.796 mmol), 2,7-dibromo-9,9-dioctylfluorene (0.3247 g, 0.592 mmol), the compound CM3 (0.2715 g, 0.160 mmol), the compound M3 (0.0257 g, 0.016 mmol), the compound CM4 (0.0548 g, 0.032 mmol), and trioctylmethylammonium chloride (manufactured by Aldrich; product name: Aliquat336) (0.10 g, 0.26 mmol) were dissolved in toluene (16 ml). Argon gas was bubbled into the solution, and the temperature was then increased to 80°C. Thereafter, dichlorobistriphenylphosphine palladium (0.6 mg, 0.8 µmol) and a 17.5-weight-% sodium carbonate aqueous solution (2.2 ml, 3.6 mmol) were added to the reaction solution, and the obtained mixture was then reacted under reflux for 20 hours. Thereafter, the reaction solution was once cooled, and phenylboric acid (0.10 g, 0.8 mmol) was then added thereto. The mixture was further reacted under reflux for 2 hours. Thereafter, toluene (10 ml) was added to the reaction solution for dilution, and a water layer was then removed. A 9-weight-% sodium N,N-diethyldithiocarbamate aqueous solution (5 ml) was added to the residue, and the obtained mixture was then stirred at 90°C for 2 hours. Thereafter, an organic layer was successively washed with ion exchanged water (10 ml) twice, then with a 3-weight-% acetic acid aqueous solution (10 ml) twice, and then with ion exchanged water (10 ml) twice. Subsequently, the resultant was added dropwise to methanol (125 ml), and the obtained mixture was then stirred for 30 minutes. Thereafter, the precipitated polymer was collected by filtration, and it was then washed with methanol (25 ml), followed by vacuum drying, so as to obtain a crude polymer.

This crude polymer was dissolved in toluene (25 ml), and it was then supplied to a column filled with alumina (5 g) and silica gel (12 g). Thereafter, toluene (20 ml) was also supplied. The obtained solution was added dropwise to methanol (125 ml), and the obtained mixture was then stirred for 30 minutes. Thereafter, the precipitated polymer was collected by filtration, and it was then washed with methanol (25 ml), followed by vacuum drying, so as to obtain a polymer compound P11 (0.50 g; yield: 70%) as a polymer. The polystyrene equivalent number average molecular weight (Mn) of the polymer compound P11 was 1.4 × 10⁵, and the polystyrene equivalent weight average molecular weight (Mw) thereof was 3.2 × 10⁵. The glass transition temperature was 124°C, and the fluorescence peak wavelength of a thin film was 446 nm.

From the raw materials used, the polymer compound P11 was assumed to comprise the following repeating units at the following ratio (molar ratio).

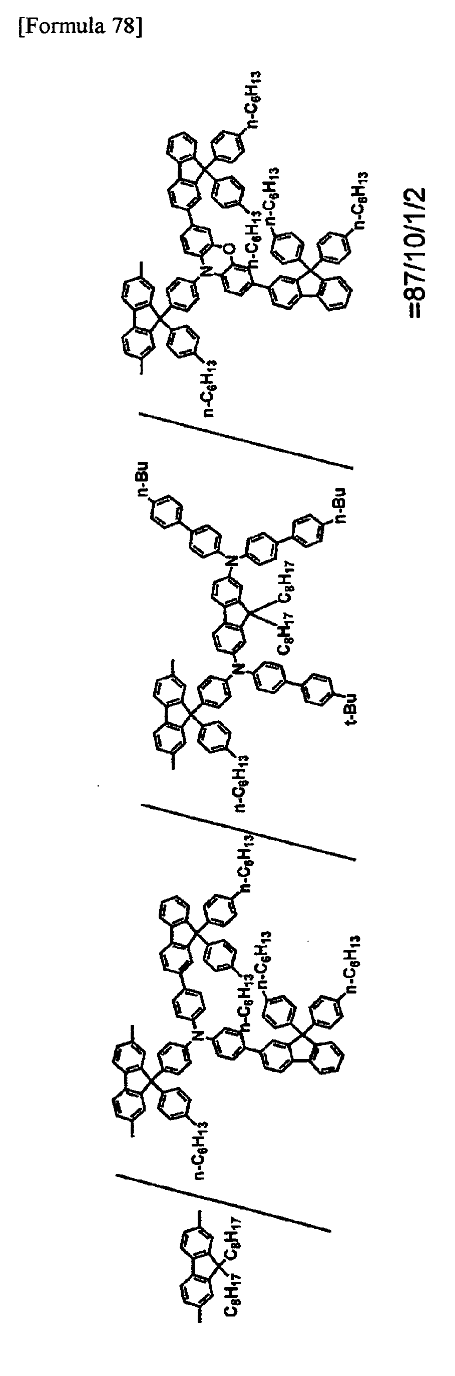

### <Example 19> (Synthesis of polymer compound P12)

Under an argon gas atmosphere, 2,7-bis(1,3,2-dioxaborolane-2-yl)-9,9-dioctylfluorene (0.4223 g, 0.796 mmol), 2,7-dibromo-9,9-dioctylfluorene (0.3071 g, 0.560 mmol), the compound CM3 (0.2308 g, 0.136 mmol), the compound M3 (0.0385 g, 0.024 mmol), 4,7-dibromo-2,1,3-benzothiadiazole (0.0235 g, 0.080 mmol), and trioctylmethylammonium chloride (manufactured by Aldrich; product name: Aliquat336) (0.10 g, 0.26 mmol) were dissolved in toluene (16 ml). Argon gas was bubbled into the solution, and the temperature was then increased to 80°C. Thereafter, dichlorobistriphenylphosphine palladium (0.6 mg, 0.8 µmol) and a 17.5-weight-% sodium carbonate aqueous solution (2.2 ml, 3.6 mmol) were added to the reaction solution, and the obtained mixture was then reacted under reflux for 20 hours. Thereafter, the reaction solution was once cooled, and phenylboric acid (0.10 g, 0.8 mmol) was then added thereto. The mixture was further reacted under reflux for 2 hours. Thereafter, toluene (10 ml) was added to the reaction solution for dilution, and a water layer was then removed. A 9-weight-% sodium N,N-diethyldithiocarbamate aqueous solution (5 ml) was added to the residue, and the obtained mixture was then stirred at 90°C for 2 hours. Thereafter, an organic layer was successively washed with ion exchanged water (10 ml) twice, then with a 3-weight-% acetic acid aqueous solution (10 ml) twice, and then with ion exchanged water (10 ml) twice. Subsequently, the resultant was added dropwise to methanol (125 ml), and the obtained mixture was then stirred for 30 minutes. Thereafter, the precipitated polymer was collected by filtration, and it was then washed with methanol (25 ml), followed by vacuum drying, so as to obtain a crude polymer.

This crude polymer was dissolved in toluene (25 ml), and it was then supplied to a column filled with alumina (5 g) and silica gel (12 g). Thereafter, toluene (20 ml) was also supplied. The obtained solution was added dropwise to methanol (125 ml), and the obtained mixture was then stirred for 30 minutes. Thereafter, the precipitated polymer was collected by filtration, and it was then washed with methanol (25 ml), followed by vacuum drying, so as to obtain a polymer compound P12 (0.49 g; yield: 72%) as a polymer. The polystyrene equivalent number average molecular weight (Mn) of the polymer compound P12 was 1.6 × 10⁵, and the polystyrene equivalent weight average molecular weight (Mw) thereof was 3.4 × 10⁵. The glass transition temperature was 105°C, and the fluorescence peak wavelength of a thin film was 531 nm.

From the raw materials used, the polymer compound P12 was assumed to comprise the following repeating units at the following ratio (molar ratio).

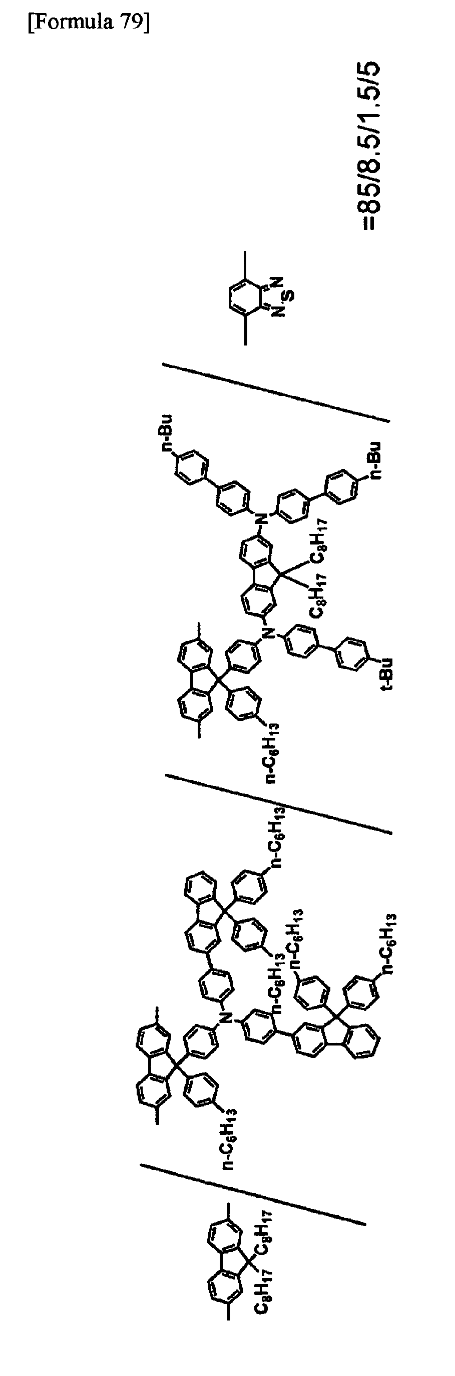

### <Example 20> (Synthesis of polymer compound P13)

Under an argon gas atmosphere, 2,7-bis(1,3,2-dioxaborolane-2-yl)-9,9-dioctylfluorene (0.4223 g, 0.796 mmol), 2,7-dibromo-9,9-dioctylfluorene (0.3247 g, 0.592 mmol), the compound CM3 (0.2308 g, 0.136 mmol), the compound M3 (0.0385 g, 0.024 mmol), 4,7-bis(5-bromothiophene-2-yl)-2,1,3-benzothiadiazole (0.0220 g, 0.048 mmol), and trioctylmethylammonium chloride (manufactured by Aldrich; product name: Aliquat336) (0.10 g, 0.26 mmol) were dissolved in toluene (16 ml). Argon gas was bubbled into the solution, and the temperature was then increased to 80°C. Thereafter, dichlorobistriphenylphosphine palladium (0.6 mg, 0.8 µmol) and a 17.5-weight-% sodium carbonate aqueous solution (2.2 ml, 3.6 mmol) were added to the reaction solution, and the obtained mixture was then reacted under reflux for 20 hours. Thereafter, the reaction solution was once cooled, and phenylboric acid (0.10 g, 0.8 mmol) was then added thereto. The mixture was further reacted under reflux for 2 hours. Thereafter, toluene (10 ml) was added to the reaction solution for dilution, and a water layer was then removed. A 9-weight-% sodium N,N-diethyldithiocarbamate aqueous solution (5 ml) was added to the residue, and the obtained mixture was then stirred at 90°C for 2 hours. Thereafter, an organic layer was successively washed with ion exchanged water (10 ml) twice, then with a 3-weight-% acetic acid aqueous solution (10 ml) twice, and then with ion exchanged water (10 ml) twice. Subsequently, the resultant was added dropwise to methanol (125 ml), and the obtained mixture was then stirred for 30 minutes. Thereafter, the precipitated polymer was collected by filtration, and it was then washed with methanol (25 ml), followed by vacuum drying, so as to obtain a crude polymer.

This crude polymer was dissolved in toluene (25 ml), and it was then supplied to a column filled with alumina (5 g) and silica gel (12 g). Thereafter, toluene (20 ml) was also supplied. The obtained solution was added dropwise to methanol (125 ml), and the obtained mixture was then stirred for 30 minutes. Thereafter, the precipitated polymer was collected by filtration, and it was then washed with methanol (25 ml), followed by vacuum drying, so as to obtain a polymer compound P13 (0.50 g; yield: 72%) as a polymer. The polystyrene equivalent number average molecular weight (Mn) of the polymer compound P13 was 1.4 × 10⁵, and the polystyrene equivalent weight average molecular weight (Mw) thereof was 3.0 × 10⁵. The glass transition temperature was 100°C, and the fluorescence peak wavelengths of a thin film were 627 nm and 653 nm.

From the raw materials used, the polymer compound P13 was assumed to comprise the following repeating units at the following ratio (molar ratio).

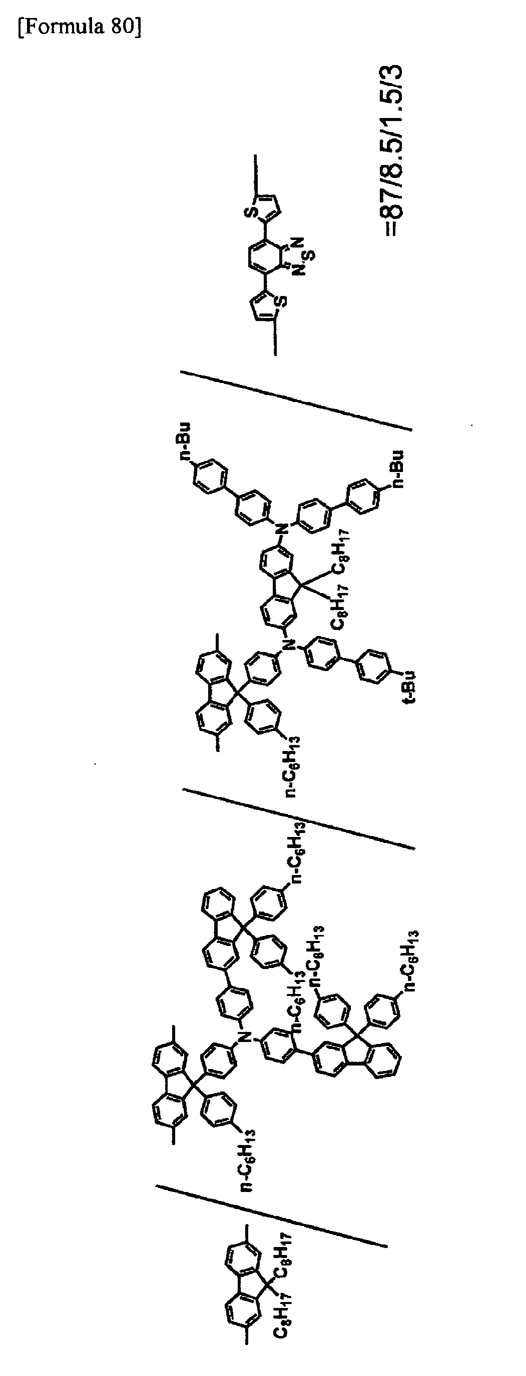

### <Example 21> (Synthesis of polymer compound P14)

Under an argon gas atmosphere, 2,7-bis(1,3,2-dioxaborolane-2-yl)-9,9-dioctylfluorene (0.3776 g, 0.796 mmol), 2,7-dibromo-9,9-dihexylfluorene (0.1339 g, 0.272 mmol), the compound CM3 (0.2308 g, 0.136 mmol), the compound M3 (0.0385 g, 0.024 mmol), 4,7-bis(5-bromothiophene-2-yl)-2,1,3-benzothiadiazole (0.0220 g, 0.048 mmol), 2,7-dibromo-9,9-bis(4-hexyloxyphenyl)fluorene (0.2165 g, 0.320 mmol), and trioctylmethylammonium chloride (manufactured by Aldrich; product name: Aliquat336) (0.10 g, 0.26 mmol) were dissolved in toluene (16 ml). Argon gas was bubbled into the solution, and the temperature was then increased to 80°C. Thereafter, dichlorobistriphenylphosphine palladium (0.6 mg, 0.8 µmol) and a 17.5-weight% sodium carbonate aqueous solution (2.2 ml, 3.6 mmol) were added to the reaction solution, and the obtained mixture was then reacted under reflux for 20 hours. Thereafter, the reaction solution was once cooled, and phenylboric acid (0.10 g, 0.8 mmol) was then added thereto. The mixture was further reacted under reflux for 2 hours. Thereafter, toluene (10 ml) was added to the reaction solution for dilution, and a water layer was then removed. A 9-weight-% sodium N,N-diethyldithiocarbamate aqueous solution (5 ml) was added to the residue, and the obtained mixture was then stirred at 90°C for 2 hours. Thereafter, an organic layer was successively washed with ion exchanged water (10 ml) twice, then with a 3-weight-% acetic acid aqueous solution (10 ml) twice, and then with ion exchanged water (10 ml) twice. Subsequently, the resultant was added dropwise to methanol (125 ml), and the obtained mixture was then stirred for 30 minutes. Thereafter, the precipitated polymer was collected by filtration, and it was then washed with methanol (25 ml), followed by vacuum drying, so as to obtain a crude polymer.

This crude polymer was dissolved in toluene (25 ml), and it was then supplied to a column filled with alumina (5 g) and silica gel (12 g). Thereafter, toluene (20 ml) was also supplied. The obtained solution was added dropwise to methanol (125 ml), and the obtained mixture was then stirred for 30 minutes. Thereafter, the precipitated polymer was collected by filtration, and it was then washed with methanol (25 ml), followed by vacuum drying, so as to obtain a polymer compound P14 (0.50 g; yield: 74%) as a polymer. The polystyrene equivalent number average molecular weight (Mn) of the polymer compound P14 was 1.5 × 10⁵, and the polystyrene equivalent weight average molecular weight (Mw) thereof was 3.2 × 10⁵. The glass transition temperature was 149°C, and the fluorescence peak wavelengths of a thin film were 628 nm and 652 nm.

From the raw materials used, the polymer compound P14 was assumed to comprise the following repeating units at the following ratio (molar ratio).

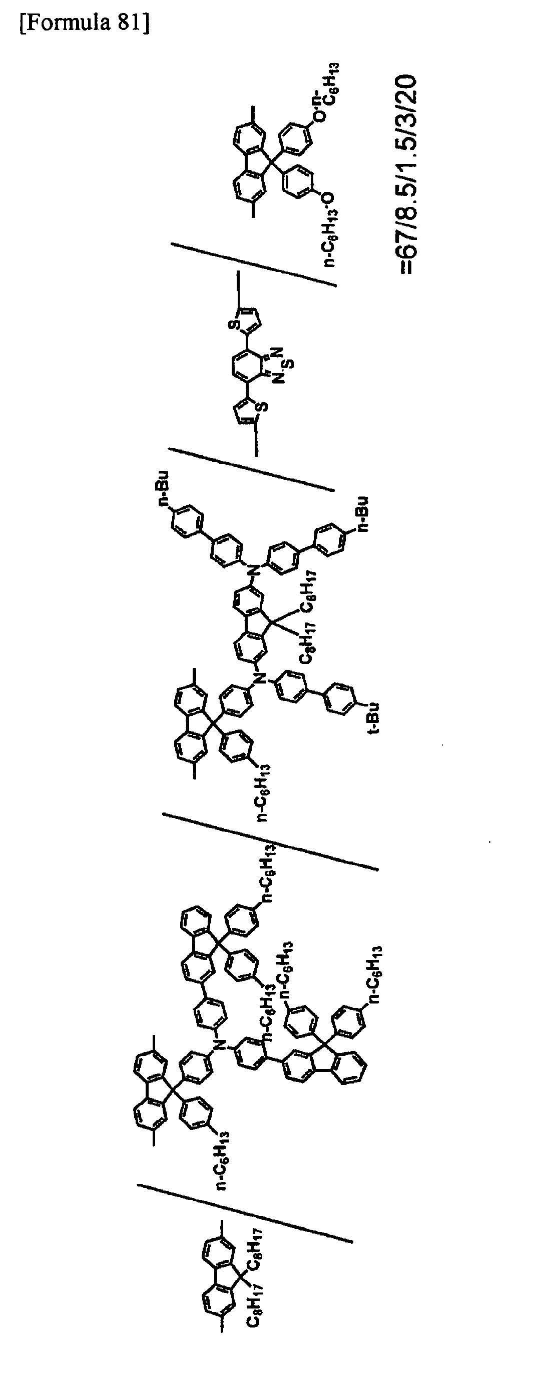

### <Example 22> (Production of light-emitting device DP4)

A light-emitting device DP4 was produced in the same manner as that of Example 7, with the exceptions that a 1.0-weight% xylene solution of the polymer compound P5 was used instead of a 1.3-weight-% xylene solution of the polymer compound P1, and that the rotation rate in spin-coating was set at 900 rpm instead of 1700 rpm. The thickness of the film was approximately 100 nm.

Voltage was applied to the light-emitting device DP4. As a result, the device emitted deep blue light having a peak wavelength (EL) derived from the polymer compound P5 at 450 nm. The maximum luminous efficiency was 1.5 cd/A. At that time, the voltage was 4.8 V, and the external quantum yield was 1.4%. The voltage at a luminance of 100 cd/m² was 6.0 V, and the chromaticity coordinate C.I.E. 1931 was (x, y) = (0.16, 0.13). The luminous efficiency was 1.4 cd/A, and the external quantum yield was 1.3%. These results are shown in Table 2.

### <Example 23> (Production of light-emitting device DP5)

A light-emitting device DP5 was produced in the same manner as that of Example 7, with the exceptions that a 1.2-weight-% xylene solution of the polymer compound P6 was used instead of a 1.3-weight% xylene solution of the polymer compound P1, and that the rotation rate in spin-coating was set at 1500 rpm instead of 1700 rpm. The thickness of the film was approximately 100 nm.

Voltage was applied to the light-emitting device DP5. As a result, the device emitted green light having a peak wavelength (EL) derived from the polymer compound P6 at 520 nm. The maximum luminous efficiency was 7.0 cd/A. At that time, the voltage was 4.6 V, and the external quantum yield was 2.0%. The voltage at a luminance of 100 cd/m² was 4.0 V, and the chromaticity coordinate C.I.E. 1931 was (x, y) = (0.29, 0.63). The luminous efficiency was 6.3 cd/A, and the external quantum yield was 1.8%. These results are shown in Table 2.

### <Example 24> (Production of light-emitting device DP6)

A light-emitting device DP6 was produced in the same manner as that of Example 7, with the exceptions that a 1.5-weight-% xylene solution of the polymer compound P7 was used instead of a 1.3-weight-% xylene solution of the polymer compound P1, and that the rotation rate in spin-coating was set at 1800 rpm instead of 1700 rpm. The thickness of the film was approximately 100 nm.

Voltage was applied to the light-emitting device DP6. As a result, the device emitted deep blue light having a peak wavelength (EL) derived from the polymer compound P7 at 450 nm. The maximum luminous efficiency was 1.5 cd/A. At that time, the voltage was 5.4 V, and the external quantum yield was 1.6%. The voltage at a luminance of 100 cd/m² was 5.6 V, and the chromaticity coordinate C.I.E. 1931 was (x, y) = (0.16, 0.11). The luminous efficiency was 1.4 cd/A, and the external quantum yield was 1.6%. These results are shown in Table 2.

### <Example 25> (Production of light-emitting device DP7)

A light-emitting device DP7 was produced in the same manner as that of Example 7, with the exceptions that a 1.4-weight-% xylene solution of the polymer compound P8 was used instead of a 1.3-weight-% xylene solution of the polymer compound P1, and that the rotation rate in spin-coating was set at 2000 rpm instead of 1700 rpm. The thickness of the film was approximately 100 nm.

Voltage was applied to the light-emitting device DP7. As a result, the device emitted deep blue light having a peak wavelength (EL) derived from the polymer compound P8 at 450 nm. The maximum luminous efficiency was 1.4 cd/A. At that time, the voltage was 5.2 V, and the external quantum yield was 1.5%. The voltage at a luminance of 100 cd/m² was 5.8 V, and the chromaticity coordinate C.I.E. 1931 was (x, y) = (0.16, 0.12). The luminous efficiency was 1.4 cd/A, and the external quantum yield was 1.4%. These results are shown in Table 2.

### <Example 26> (Production of light-emitting device DP8)

A light-emitting device DP8 was produced in the same manner as that of Example 7, with the exceptions that a 1.5-weight-% xylene solution of the polymer compound P9 was used instead of a 1.3-weight-% xylene solution of the polymer compound P1, and that the rotation rate in spin-coating was set at 2000 rpm instead of 1700 rpm. The thickness of the film was approximately 100 nm.

Voltage was applied to the light-emitting device DP8. As a result, the device emitted blue light having a peak wavelength (EL) derived from the polymer compound P9 at 460 nm. The maximum luminous efficiency was 3.2 cd/A. At that time, the voltage was 6.0 V, and the external quantum yield was 2.2%. The voltage at a luminance of 100 cd/m² was 5.0 V, and the chromaticity coordinate C.I.E. 1931 was (x, y) = (0.15, 0.19). The luminous efficiency was 3.0 cd/A, and the external quantum yield was 2.2%. These results are shown in Table 2.

### <Example 27> (Production of light-emitting device DP9)

A light-emitting device DP9 was produced in the same manner as that of Example 7, with the exceptions that a 1.6-weight% xylene solution of the polymer compound P10 was used instead of a 1.3-weight-% xylene solution of the polymer compound P1, and that the rotation rate in spin-coating was set at 1000 rpm instead of 1700 rpm. The thickness of the film was approximately 100 nm.

Voltage was applied to the light-emitting device DP9. As a result, the device emitted blue light having a peak wavelength (EL) derived from the polymer compound P10 at 475 nm. The maximum luminous efficiency was 2.9 cd/A. At that time, the voltage was 4.8 V, and the external quantum yield was 1.9%. The voltage at a luminance of 100 cd/m² was 6.2 V, and the chromaticity coordinate C.I.E. 1931 was (x, y) = (0.15, 0.22). The luminous efficiency was 2.6 cd/A, and the external quantum yield was 1.7%. These results are shown in Table 2.

### <Example 28> (Production of light-emitting device DP10)

A light-emitting device DP10 was produced in the same manner as that of Example 7, with the exceptions that a 1.5-weight-% xylene solution of the polymer compound P11 was used instead of a 1.3-weight-% xylene solution of the polymer compound P1, and that the rotation rate in spin-coating was set at 1800 rpm instead of 1700 rpm. The thickness of the film was approximately 100 nm.

Voltage was applied to the light-emitting device DP10. As a result, the device emitted blue light having a peak wavelength (EL) derived from the polymer compound P11 at 480 nm. The maximum luminous efficiency was 2.6 cd/A. At that time, the voltage was 4.6 V, and the external quantum yield was 1.9%. The voltage at a luminance of 100 cd/m² was 6.6 V, and the chromaticity coordinate C.I.E. 1931 was (x, y) = (0.15, 0.19). The luminous efficiency was 2.2 cd/A, and the external quantum yield was 1.6%. These results are shown in Table 2.

### <Example 29> (Production of light-emitting device DP11)

A light-emitting device DP11 was produced in the same manner as that of Example 7, with the exceptions that a 1.2-weight-% xylene solution of the polymer compound P12 was used instead of a 1.3-weight-% xylene solution of the polymer compound P1, and that the rotation rate in spin-coating was set at 900 rpm instead of 1700 rpm. The thickness of the film was approximately 100 nm.

Voltage was applied to the light-emitting device DP11. As a result, the device emitted green light having a peak wavelength (EL) derived from the polymer compound P12 at 535 nm. The maximum luminous efficiency was 9.3 cd/A. At that time, the voltage was 6.8 V, and the external quantum yield was 2.8%. The voltage at a luminance of 100 cd/m² was 7.4 V, and the chromaticity coordinate C.I.E. 1931 was (x, y) = (0.39, 0.58). The luminous efficiency was 9.0 cd/A, and the external quantum yield was 2.7%. These results are shown in Table 2.

### <Example 30> (Production of light-emitting device DP12)

A light-emitting device DP12 was produced in the same manner as that of Example 7, with the exceptions that a 1.4-weight-% xylene solution of the polymer compound P13 was used instead of a 1.3-weight-% xylene solution of the polymer compound P1, and that the rotation rate in spin-coating was set at 1300 rpm instead of 1700 rpm. The thickness of the film was approximately 100 nm.

Voltage was applied to the light-emitting device DP12. As a result, the device emitted red light having a peak wavelength (EL) derived from the polymer compound P13 at 645 nm. The maximum luminous efficiency was 0.83 cd/A. At that time, the voltage was 6.2 V, and the chromaticity coordinate C.I.E. 1931 was (x, y) = (0.67, 0.32). The external quantum yield was 1.4%.

### <Example 31> (Production of light-emitting device DP13)

A light-emitting device DP13 was produced in the same manner as that of Example 7, with the exceptions that a 1.5-weight% xylene solution of the polymer compound P14 was used instead of a 1.3-weight-% xylene solution of the polymer compound P1, and that the rotation rate in spin-coating was set at 1700 rpm instead of 1700 rpm. The thickness of the film was approximately 100 nm.

Voltage was applied to the light-emitting device DP13. As a result, the device emitted red light having a peak wavelength (EL) derived from the polymer compound P14 at 655 nm. The maximum luminous efficiency was 1.1 cd/A. At that time, the voltage was 7.2 V, and the chromaticity coordinate C.I.E. 1931 was (x, y) = (0.66, 0.33). The external quantum yield was 1.7%.

[Table 2]

**Table 2**

| | Polymer compound | Maximum external quantum yield (%) | Value at luminance of 100cd/m² | | Peak wavelength | |
|---|---|---|---|---|---|---|
| | | | External quantum yield (%) | Chromaticity CIE (x,y) | λ max | |
| | | | | | EL | PL |
| Example 22 | P5 | 1.4 | 1.3 | (0.16, 0.13) | 450nm | 422, 446nm |
| Example 23 | P6 | 2.0 | 1.8 | (0.29, 0.63) | 520nm | 515nm |
| Example 24 | P7 | 1.6 | 1.6 | (0.16, 0.11) | 450nm | 425nm |
| Example 25 | P8 | 1.5 | 1.4 | (0.16, 0.12) | 450nm | 422,446nm |
| Example 26 | P9 | 2.2 | 2.2 | (0.15, 0.19) | 460nm | 446, 462nm |
| Example 27 | P10 | 1.9 | 1.7 | (0.15, 0.22) | 475nm | 450nm |
| Example 28 | P11 | 1.9 | 1.6 | (0.15, 0.19) | 480nm | 446nm |
| Example 29 | P12 | 2.8 | 2.7 | (0.39, 0.58) | 535nm | 531nm |

### <Synthesis Example 3> (Synthesis of light-emitting material EM-A)

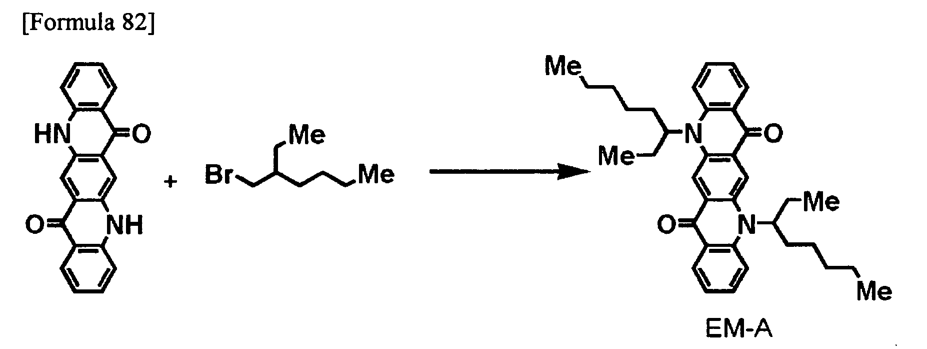

Under an argon atmosphere, N,N-dimethylacetamide (300 ml) was mixed with quinacridon (15.0 g, 48.0 mmol) in a 1000-ml flask. Thereafter, approximately 60% by weight of sodium hydride (5.76 g, 144 mmol) diluted with mineral oil was gradually added to the above mixture, and the thus obtained mixture was then stirred at 80°C for 1 hour. Thereafter, 2-ethylhexylbromide (38.6 g, 200 mmol) was added dropwise to the reaction solution over 15 minutes, and the obtained mixture was then stirred at 80°C for 6 hours. After completion of the reaction, the reaction solution was poured into distilled water (900 ml) cooled in an ice water bath, and it was then neutralized with 1 N hydrochloric acid water. Thereafter, 800 ml of ethyl acetate was added thereto for extraction, and an organic layer was then washed with a 5-weight-% saline (300 ml). The obtained organic layer was dried over magnesium sulfate, followed by vacuum concentration. The obtained solid was dissolved in chloroform (200 ml), and the solution was then supplied to a silica gel short column, followed by vacuum concentration. Thereafter, hexane (150 ml) was added to the resultant, and the precipitated solid was then collected by filtration. The resultant was washed with hexane (100 ml), followed by vacuum drying, so as to obtain a crude product (11.2 g). The obtained solid was purified by intermediate pressure silica gel column chromatography (chloroform/ethyl acetate = 5/1), so as to obtain a light-emitting material EM-A (9.4 g; yield: 46%).
¹H-NMR (300 MHz, CDCl₃): δ (ppm)=0.84 (t, 6H), 0.97 (t, 6H), 1.20-1.54 (m, 16H), 2.20 (bs, 2H), 4.50 (bs, 4H), 7.26 (t, 2H), 7.56 (d, 2H), 7.72 (t, 2H), 8.58 (d, 2H), 8.84 (s, 2H).
¹³C-NMR (75 MHz, CDCl₃): δ (ppm)=11.6, 14.3, 23.4, 24.7, 29.1, 31.3, 38.7, 50.0, 114.7, 115.8, 121.2, 121.6, 126.5, 128.4, 134.7, 136.7, 143.4, 178.5.

### <Synthesis Example 4> (Synthesis of light-emitting material EM-B)

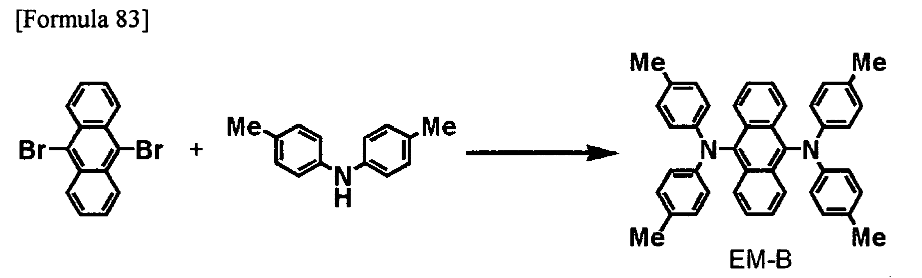

Under an argon atmosphere, argon gas was bubbled into a mixture of 9,10-dibromoanthracene (16.8 g, 50.0 mmol), sodium-tert-butoxide (10.5 g, 110 mmol), tris(dibenzylideneacetone)dipalladium(0) (0.46 g, 0.5 mmol) and toluene (100 ml) in a 500-ml flask. Thereafter, tri-tert-butylphosphine (0.4 g, 2 mmol) was then added to the reaction solution using a syringe. The obtained mixture was heated to 90°C, and a solution prepared by dissolving N,N-di-p-tolylamine (21.7 g, 110 mmol) in toluene (100 ml) was added dropwise thereto over 40 minutes. The obtained mixture was stirred at 90°C for 40 minutes, and it was then stirred under reflux for 3 hours. Thereafter, the reaction solution was cooled to a room temperature, and the precipitate was then collected by filtration. The obtained solid was dissolved in chloroform (500 ml) under reflux, and insoluble matter was then removed by filtration using a funnel and a filter that had previously been heated. The filtrate was concentrated to a volume of approximately 350 g, and it was then added dropwise to methanol (250 ml) while stirring. The precipitated solid was collected by filtration, and it was then washed with methanol, followed by vacuum drying. Toluene (200 ml) was added to the obtained solid, and the obtained mixture was intensively stirred at 80°C. The reaction solution was cooled to a room temperature. A solid was collected by filtration, and it was successively washed with toluene (100 ml) and hexane (100 ml), followed by vacuum drying, so as to obtain a light-emitting material EM-B in the form of orange powders (19.9 g; yield: 70%).
¹H-NMR (300 MHz, CDCl₃): δ (ppm)=2.26 (s, 12H), 6.98 (s, 16H), 7.30-7.34 (m, 4H), 8.15-8.18 (m, 4H).

### <Example 32>

A 0.8-weight% xylene solution of the polymer compound P1 was prepared. This solution was applied onto a quartz substrate by spin-coating at a rotation rate of 1000 rpm, and the photoluminescence quantum yield (PLQY) thereof was then measured. As a result, the quantum yield was 47%, and the chromaticity coordinate C.I.E. 1931 was found to be (x, y) = (0.16, 0.07) based on the emission spectrum, thereby emitting deep blue light. These results are shown in Table 3.

### <Example 33>

A photoluminescence quantum yield (PLQY) was measured in the same manner as that of Example 32, with the exception that a 0.8-weight-% xylene solution of a mixture of the polymer compound P1 and 5% by weight of the above-described light-emitting material EM-A was used instead of a 0.8-weight-% xylene solution of the polymer compound P1. As a result, the quantum yield was 15%, and the chromaticity coordinate C.I.E. 1931 was found to be (x, y) = (0.35, 0.53) based on the emission spectrum, thereby emitting green light. These results are shown in Table 3.

### <Example 34>

A photoluminescence quantum yield (PLQY) was measured in the same manner as that of Example 32, with the exception that a 0.8-weight-% xylene solution of a mixture of the polymer compound P1 and 5% by weight of the above-described light-emitting material EM-B was used instead of a 0.8-weight-% xylene solution of the polymer compound P1. As a result, the quantum yield was 77%, and the chromaticity coordinate C.I.E. 1931 was found to be (x, y) = (0.29, 0.63) based on the emission spectrum, thereby emitting green light. These results are shown in Table 3.

### <Example 35>

A photoluminescence quantum yield (PLQY) was measured in the same manner as that of Example 32, with the exception that a 0.8-weight-% xylene solution of a mixture of the polymer compound P1 and a 5-weight-% light-emitting material EM-C (manufactured by American Dye Source; product name: ADS077RE) as shown in a formula below was used instead of a 0.8-weight-% xylene solution of the polymer compound P1. As a result, the quantum yield was 24%, and the chromaticity coordinate C.I.E. 1931 was found to be (x, y) = (0.51, 0.22) based on the emission spectrum, thereby emitting red light. These results are shown in Table 3.

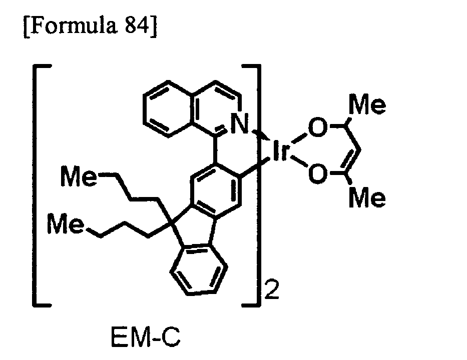

### <Example 36>

A photoluminescence quantum yield (PLQY) was measured in the same manner as that of Example 32, with the exception that a 0.8-weight-% xylene solution of a mixture of the polymer compound P1 and a 10-weight-% light-emitting material EM-D (manufactured by Tokyo Chemical Industry Co., Ltd.; 5,6,11,12-tetraphenylnaphthacene) as shown in a formula below was used instead of a 0.8-weight-% xylene solution of the polymer compound P1. As a result, the quantum yield was 53%, and the chromaticity coordinate C.I.E. 1931 was found to be (x, y) = (0.48, 0.49) based on the emission spectrum, thereby emitting yellow light. These results are shown in Table 3.

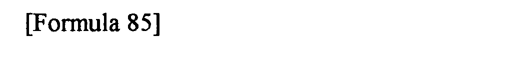

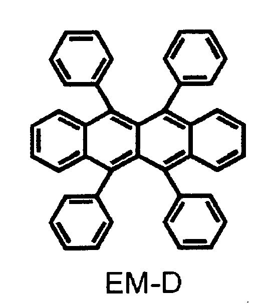

### <Example 37>

A photoluminescence quantum yield (PLQY) was measured in the same manner as that of Example 32, with the exception that a 0.8-weight-% xylene solution of the polymer compound P9 was used instead of a 0.8-weight-% xylene solution of the polymer compound P1. As a result, the quantum yield was 71%, and the chromaticity coordinate C.I.E. 1931 was found to be (x, y) = (0.14, 0.12) based on the emission spectrum, thereby emitting deep blue light. These results are shown in Table 3.

### <Example 38>

A photoluminescence quantum yield (PLQY) was measured in the same manner as that of Example 32, with the exception that a 0.8-weight-% xylene solution of a mixture of the polymer compound P9 and 5% by weight of the above-described light-emitting material EM-A was used instead of a 0.8-weight-% xylene solution of the polymer compound P1. As a result, the quantum yield was 15%, and the chromaticity coordinate C.I.E. 1931 was found to be (x, y) = (0.37, 0.52) based on the emission spectrum, thereby emitting green light. These results are shown in Table 3.

### <Example 39>

A photoluminescence quantum yield (PLQY) was measured in the same manner as that of Example 32, with the exception that a 0.8-weight-% xylene solution of a mixture of the polymer compound P9 and 5% by weight of the above-described light-emitting material EM-B was used instead of a 0.8-weight-% xylene solution of the polymer compound P1. As a result, the quantum yield was 75%, and the chromaticity coordinate C.I.E. 1931 was found to be (x, y) = (0.29, 0.63) based on the emission spectrum, thereby emitting green light. These results are shown in Table 3.

### <Example 40>

A photoluminescence quantum yield (PLQY) was measured in the same manner as that of Example 32, with the exception that a 0.8-weight-% xylene solution of a mixture of the polymer compound P9 and 5% by weight of the above-described light-emitting material EM-C was used instead of a 0.8-weight-% xylene solution of the polymer compound P1. As a result, the quantum yield was 24%, and the chromaticity coordinate C.I.E. 1931 was found to be (x, y) = (0.48, 0.22) based on the emission spectrum, thereby emitting red light. These results are shown in Table 3.

### <Example 41>

A photoluminescence quantum yield (PLQY) was measured in the same manner as that of Example 32, with the exception that a 0.8-weight-% xylene solution of a mixture of the polymer compound P9 and 10% by weight of the above-described light-emitting material EM-D was used instead of a 0.8-weight-% xylene solution of the polymer compound P1. As a result, the quantum yield was 60%, and the chromaticity coordinate C.I.E. 1931 was found to be (x, y) = (0.49, 0.49) based on the emission spectrum, thereby emitting yellow light. These results are shown in Table 3.

[Table 3]

**Table 3**

| | Polymer compound | Light-emitting material | Composition ratio (weight ratio) | Quantum yield (PLQY) | Chromaticity CIE (x, y) |
|---|---|---|---|---|---|
| Example 32 | P1 | n/a | | 47% | (0.16, 0.07) |
| Example 33 | P1 | EM-A | 95/5 | 15% | (0.35, 0.53) |
| Example 34 | P1 | EM-B | 95/5 | 77% | (0.29, 0.63) |
| Example 35 | P1 | EM-C | 95/5 | 24% | (0.51, 0.22) |
| Example 36 | P1 | EM-D | 90/10 | 53% | (0.48, 0.49) |
| Example 37 | P9 | n/a | | 71% | (0.14,0.12) |
| Example 38 | P9 | EM-A | 95/5 | 15% | (0.37, 0.52) |
| Example 39 | P9 | EM-B | 95/5 | 75% | (0.29, 0.63) |
| Example 40 | P9 | EM-C | 95/5 | 24% | (0.48, 0.22) |
| Example 41 | P9 | EM-D | 90/10 | 60% | (0.49, 0.49) |

### <Evaluation>

As is found from Table 1, a light-emitting device that uses the polymer compound of the present invention has an excellent external quantum yield, in comparison with a light-emitting device that uses the conventional polymer compound.

As is found from Table 2, a light-emitting device that uses the polymer compound of the present invention has an excellent external quantum yield, even if the above-described polymer compound is a copolymer.

Moreover, as is found from Table 2 and Table 3, a light-emitting device that uses the polymer compound of the present invention is able to adjust the other monomer(s) to be copolymerized therewith, or it can be prepared in the form of a composition with various types of light-emitting materials, so that it can easily adjust luminescence chromaticity.

### INDUSTRIAL APPLICATIBLITY

When the polymer compound of the present invention is used to produce light-emitting devices such as polymer LED, the obtained light-emitting device has an excellent external quantum yield. Moreover, in general, the polymer compound of the present invention is excellent in terms of heat resistance, and it is useful as a material for light-emitting devices. Furthermore, in a preferred embodiment of the present invention, since the polymer compound of the present invention emits pure blue light, it is useful as a material for the light-emitting layer of a light-emitting device.
A light-emitting device, in which the polymer compound of the present invention is used, is useful as a backlight for liquid crystal display, a curved or planar light source for lighting, a segment-type display device, and a display device such as a flat panel display of dot matrix.

## Claims

1. A polymer compound comprising a constitutional unit represented by the following formula (1a): 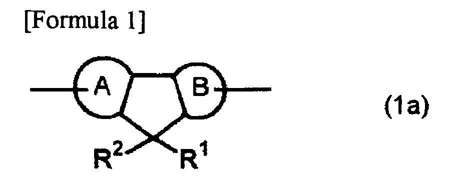 wherein each of ring A and ring B independently represents an aromatic hydrocarbon ring that may have a substituent; R1 represents a group represented by formula (2) provided below; and R2 represents an aryl group or a monovalent aromatic heterocyclic group, and these groups may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group; 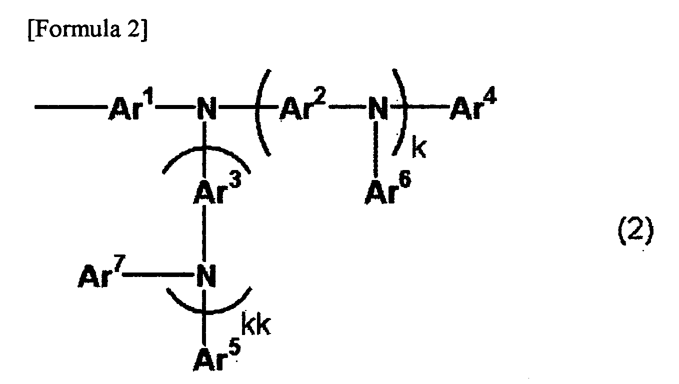 wherein each of Ar1, Ar2 and Ar3 independently represents an arylene group or a divalent group in which two or more identical or different arylene groups are bound to one another by a single bond; each of Ar4, Ar5, Ar6 and Ar7 independently represents an aryl group or a monovalent aromatic heterocyclic group; a group selected from among the groups represented by Ar3, Ar6 and Ar7 may form a 5- to 7-membered ring by being bound by a single bond or by -O-, -S-, - C(=O)-, -C(O=)-O-, -N(R6)-, -C(=O)-N(R6)- or -C(R6)(R6)- to a group that is selected from among the groups represented by Ar1, Ar2, Ar3, Ar4, Ar5, Ar6 and Ar7 and that is attached to a nitrogen atom which is the same as that to which the group selected from the groups represented by Ar3, Ar6 and Ar7 is attached. R6 represents a hydrogen atom, an alkyl group, an aryl group or a monovalent aromatic heterocyclic group; each of the groups represented by Ar1, Ar2, Ar3, Ar4, Ar5, Ar6, Ar7 and R6 may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group; each of k and kk independently represents an integer of 0 to 3, but at least one of k and kk is an integer of 1 to 3; and if a plurality of Ar2, Ar3, Ar6, Ar7 or R6 are present, they may be identical to or different from one another.

2. The polymer compound according to claim 1, wherein the constitutional unit represented by formula (1a) is represented by the following formula (1): 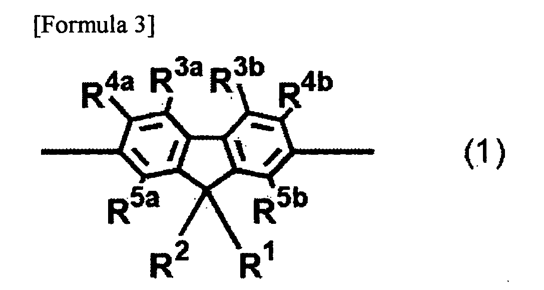 wherein R1 and R2 are the same as above in meaning; each of R3a, R4a, R5a, R3b, R4b and R5b independently represents a hydrogen atom, an alkyl group, an aryl group, a monovalent aromatic heterocyclic group, -N(R8)(R9), a fluorine atom or a cyano group; each of R8 and R9 independently represents a hydrogen atom, an alkyl group, an aryl group or a monovalent aromatic heterocyclic group; each of the aryl groups and the monovalent aromatic heterocyclic groups represented by R3a, R4a, R5a, R3b, R4b, R5b, R8 and R9 may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group; and each of R3a and R4a, R3b and R4b, R3a and R3b, and R8 and R9, may together form a ring.

3. The polymer compound according to claim 1 or 2, wherein the formula weight of the group represented by R1 is 2000 or less.

4. The polymer compound according to any one of claims 1 to 3, wherein the R2 represents an aryl group substituted with an alkyl group or an aryl group, or an unsubstituted aryl group.

5. The polymer compound according to any one of claims 1 to 4, wherein the formula weight of the group represented by R2 is 500 or less.

6. The polymer compound according to any one of claims 1 to 5, wherein the sum of the value of k and the value of kk is an integer of 1 to 4.

7. The polymer compound according to any one of claims 1 to 6, further comprising a constitutional unit selected from among constitutional units represented by the following formulae (3) to (5):
[Formula 4] **-Ar⁸-** (3) 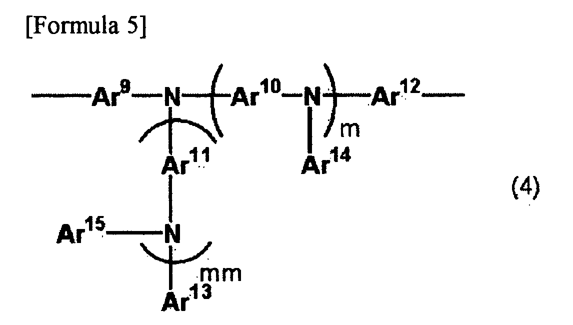
[Formula 6] **-Ar¹⁶-X¹-** (5)
wherein each of Ar8 and Ar16 independently represents an arylene group, or a divalent aromatic heterocyclic group, or a divalent group in which two or more identical or different groups selected from the group consisting of arylene groups and divalent aromatic heterocyclic groups are bound to one another by a single bond; each of Ar9, Ar10, Ar11 and Ar12 independently represents an arylene group or a divalent group in which two or more identical or different arylene groups are bound to one another by a single bond; each of Ar13, Ar14 and Ar15 independently represents an aryl group or a monovalent aromatic heterocyclic group; each of arylene groups, divalent aromatic heterocyclic groups and divalent groups represented by Ar8 and Ar16 may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, -N(R8)(R9), a fluorine atom or a cyano group; each of the groups represented by Ar9, Ar10, Ar11, Ar12, Ar13, Ar14 and Ar15 may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group; a group selected from among the groups represented by Ar11, Ar14 and Ar15 may form a 5- to 7-membered ring by being bound by a single bond or by -O-, -S-, -C(=O)-, -C(=O)-O-, -N(R6)-, -C(=O)-N(R6)- or -C(R6)(R6)- to a group that is selected from among the groups represented by Ar9, Ar10, Ar11, Ar12, Ar13, Ar14 and Ar15 and that is attached to a nitrogen atom which is the same as that to which the group selected from among the groups represented by Ar11, Ar 14 and Ar15 is attached; each of m and mm independently represents 0 or 1; X1 represents -C(R7)=C(R7)- or -C≡C-; R7 independently represents a hydrogen atom, an alkyl group, an aryl group, a monovalent aromatic heterocyclic group, a fluorine atom or a cyano group; the group represented by R7 may have a substituent; if a plurality of Ar10, Ar11, Ar14 or Ar15 are present, they may be identical to or different from one another; and R6, R8 and R9 are the same as above in meaning.

8. The polymer compound according to claim 7, wherein the total number of moles of the constitutional unit represented by formula (1a), the constitutional unit represented by formula (3), the constitutional unit represented by formula (4) and the constitutional unit represented by formula (5), relative to the total number of moles of all constitutional units, is 90% to 100%.

9. The polymer compound according to any one of claims 1 to 8 having polystyrene equivalent number average molecular weight of 1 × 103 to 1 × 108.

10. A compound represented by the following formula (A): 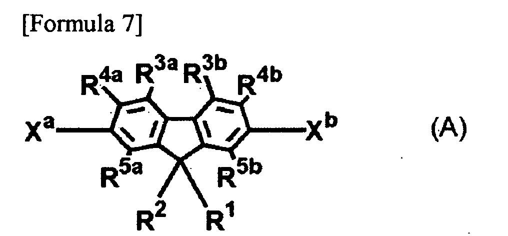 wherein R1, R2, R3a, R4a, R5a, R3b, R4b and R5b are the same as above in meaning; each of Xa and Xb independently represents a bromine atom, an iodine atom, a chlorine atom, -O-S(=O)2R20, -B(OR21)2, -BF4Q1, -Sn(R22)3, -MgY1 or -ZnY1; R20 represents an alkyl group, or an aryl group that may be substituted with an alkyl group, an alkoxy group, a nitro group, a fluorine atom or a cyano group; each of R21 and R22 independently represents a hydrogen atom or an alkyl group; two R21s may be identical to or different from each other or may together form a ring; three R22s may be identical to or different from one another, or may together form a ring; Q1 represents a monovalent cation of lithium, sodium, potassium, rubidium or cesium; and Y1 represents a bromine atom, an iodine atom or a chlorine atom.

11. A compound represented by the following formula (B): 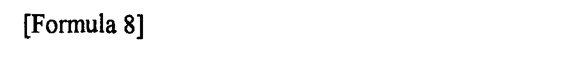 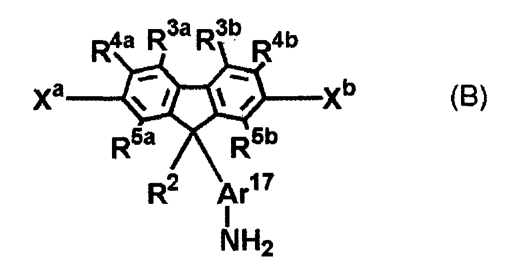 wherein R2, R3a, R4a, R5a, R3b, R4b, R5b, Xa and Xb are the same as above in meaning, and Ar17 represents an arylene group, wherein the arylene group represented by Ar17 may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group.

12. A compound represented by the following formula (C): 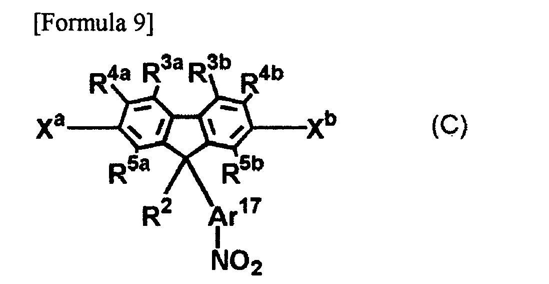 wherein R2, R3a, R4a, R5a, R3b, R4b, R5b, Xa, Xb and Ar17 are the same as above in meaning.

13. A method for producing the compound represented by formula (A), which comprises making the compound represented by formula (B) react with a compound represented by the following formula (E): 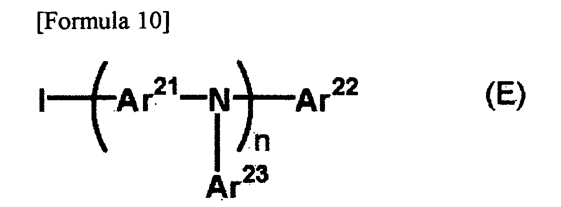 wherein Ar21 represents an arylene group, or a divalent group in which two or more identical or different arylene groups are bound to one another by a single bond; the arylene group and the divalent group represented by Ar21 may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group; each of Ar22 and Ar23 independently represents an aryl group or a monovalent aromatic heterocyclic group; the aryl group and the monovalent aromatic heterocyclic group represented by Ar22 and Ar23 may be substituted with an alkyl group, an alkoxy group, an alkylthio group, a substituted carbonyl group, a substituted carboxyl group, an aryl group, an aryloxy group, an arylthio group, an aralkyl group, a monovalent aromatic heterocyclic group, a fluorine atom or a cyano group; two groups which are selected from among the groups represented by Ar21, Ar22 and Ar23 and which are attached to the same nitrogen atom may be bound to each other to form a 5- to 7-membered ring by a single bond or by -O-, - S-, -C(=O)-, -C(=O)-O-, -N(R6)-, -C(=O)-N(R6)- or -C(R6)(R6)-; n represents an integer of 0 to 2; if a plurality of Ar21 or Ar23 are present, they may be identical to or different from one another; and R6 is the same as above in meaning.

14. A method for producing the compound represented by formula (A), which comprises making a compound represented by the following formula (D) react with a compound represented by the following formula (F): 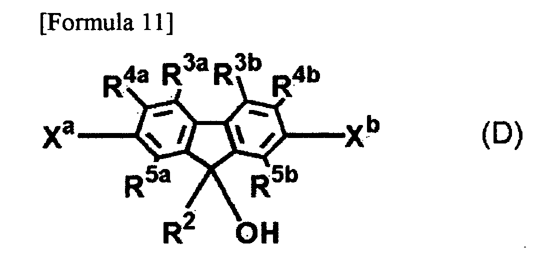 wherein R2, R3a, R4a, R5a, R3b, R4b, R5b, Xa and Xb are the same as above in meaning; 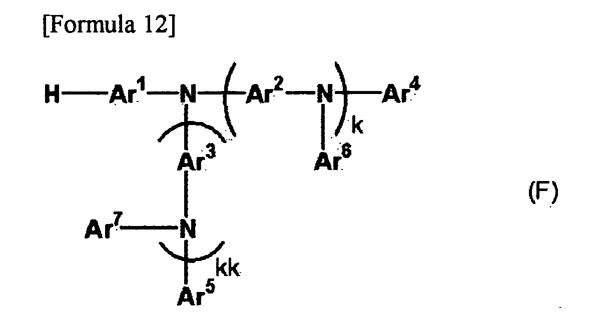 wherein, Ar1, Ar2, Ar3, Ar4, Ar5, Ar6, Ar7, k and kk are the same as above in meaning.

15. A composition comprising a polymer compound according to any one of claims 1 to 9.

16. A solution comprising a polymer compound according to any one of claims 1 to 9 and a solvent.

17. A thin film comprising a polymer compound according to any one of claims 1 to 9.

18. A light-emitting device which comprises electrodes consisting of an anode and a cathode, and an organic layer containing a polymer compound according to any one of claims 1 to 9 provided between the electrodes.

19. A planar light source comprising a light-emitting device according to claim 18.

20. A display device comprising a light-emitting device according to claim 18.
